# EUROPEAN PATENT APPLICATION

(11) **EP 2 018 873 A1**
(43) Date of publication of application: **28.01.2009**
(21) Application number: 07741561.0
(22) Date of filing: 06.04.2007
(51) Int. Cl.: A61K 45/06, A61K 31/7088, A61K 39/395, A61K 48/00, A61P 35/00, A61P 35/02, A61P 35/04, A61P 43/00

(54) **PROPHYLACTIC/THERAPEUTIC AGENT FOR CANCER**

(30) Priority: 07.04.2006 JP 2006106928
(71) Applicant: JAPANESE FOUNDATION FOR CANCER RESEARCH, Tokyo 135-8550 (JP); Takeda Pharmaceutical Company Limited, Osaka-shi, Osaka 541-0045 (JP)
(72) Inventor: MASHIMA, Tetsuo, Tokyo 135-8550 (JP); TSURUO, Takashi, Tokyo 135-8550 (JP); TOJO, Hideaki, Tsukuba-shi Ibaraki 300-4293 (JP); SUMI, Hiroyuki, Tsukuba-shi Ibaraki 300-4293 (JP)
(74) Representative: Jones, Nicholas Andrew
(86) International application number: PCT/JP2007/058125
(87) International publication number: WO 2007/117038

(57) **Abstract**

The present invention provides a preventative/therapeutic agent for cancer capable of selectively and effectively killing cancer cells. A medicament of the present invention prepared by combining at least one selected from a substance that inhibits the activity of an enzyme belonging to the acyl-CoA synthase family and a substance that inhibits expression of a gene for an enzyme belonging to the acyl-CoA synthase family, with at least one selected from a substance that inhibits the activity of fatty acid synthase and a substance that inhibits expression of a fatty acid synthase gene and the like can be used as a preventative/therapeutic agent for cancer capable of selectively and effectively killing cancer cells.

SEQUENCE LISTING

<110> Japanese Foundation for Cancer Research
      Takeda Pharmaceutical Company Limited

<120> Prophylactic/therapeutic agent for cancer

<130> G08-0068

<140> PCT/JP2007/058125
<141> 2007-04-06

<150> JP2006-106928
<151> 2006-04-07

<160> 20

<210> 1
<211> 698
<212> PRT
<213> Homo sapiens

<400> 1
Met Gln Ala His Glu Leu Phe Arg Tyr Phe Arg Met Pro Glu Leu Val
                5                   10                  15
Asp Phe Arg Gln Tyr Val Arg Thr Leu Pro Thr Asn Thr Leu Met Gly
            20                  25                  30
Phe Gly Ala Phe Ala Ala Leu Thr Thr Phe Trp Tyr Ala Thr Arg Pro
        35                  40                  45
Lys Pro Leu Lys Pro Pro Cys Asp Leu Ser Met Gln Ser Val Glu Val
    50                  55                  60
Ala Gly Ser Gly Gly Ala Arg Arg Ser Ala Leu Leu Asp Ser Asp Glu
65                  70                  75                  80
Pro Leu Val Tyr Phe Tyr Asp Asp Val Thr Thr Leu Tyr Glu Gly Phe
                85                  90                  95
Gln Arg Gly Ile Gln Val Ser Asn Asn Gly Pro Cys Leu Gly Ser Arg
            100                 105                 110
Lys Pro Asp Gln Pro Tyr Glu Trp Leu Ser Tyr Lys Gln Val Ala Glu
        115                 120                 125
Leu Ser Glu Cys Ile Gly Ser Ala Leu Ile Gln Lys Gly Phe Lys Thr
    130                 135                 140
Ala Pro Asp Gln Phe Ile Gly Ile Phe Ala Gln Asn Arg Pro Glu Trp
145                 150                 155                 160
Val Ile Ile Glu Gln Gly Cys Phe Ala Tyr Ser Met Val Ile Val Pro
                165                 170                 175
Leu Tyr Asp Thr Leu Gly Asn Glu Ala Ile Thr Tyr Ile Val Asn Lys
            180                 185                 190
Ala Glu Leu Ser Leu Val Phe Val Asp Lys Pro Glu Lys Ala Lys Leu
        195                 200                 205
Leu Leu Glu Gly Val Glu Asn Lys Leu Ile Pro Gly Leu Lys Ile Ile
    210                 215                 220
Val Val Met Asp Ala Tyr Gly Ser Glu Leu Val Glu Arg Gly Gln Arg
225                 230                 235                 240
Cys Gly Val Glu Val Thr Ser Met Lys Ala Met Glu Asp Leu Gly Arg
                245                 250                 255
Ala Asn Arg Arg Lys Pro Lys Pro Pro Ala Pro Glu Asp Leu Ala Val
            260                 265                 270
Ile Cys Phe Thr Ser Gly Thr Thr Gly Asn Pro Lys Gly Ala Met Val
        275                 280                 285
Thr His Arg Asn Ile Val Ser Asp Cys Ser Ala Phe Val Lys Ala Thr
    290                 295                 300
Glu Asn Thr Val Asn Pro Cys Pro Asp Asp Thr Leu Ile Ser Phe Leu
305                 310                 315                 320
Pro Leu Ala His Met Phe Glu Arg Val Val Glu Cys Val Met Leu Cys
                325                 330                 335
His Gly Ala Lys Ile Gly Phe Phe Gln Gly Asp Ile Arg Leu Leu Met
            340                 345                 350
Asp Asp Leu Lys Val Leu Gln Pro Thr Val Phe Pro Val Val Pro Arg
        355                 360                 365
Leu Leu Asn Arg Met Phe Asp Arg Ile Phe Gly Gln Ala Asn Thr Thr

370                 375                 380
Leu Lys Arg Trp Leu Leu Asp Phe Ala Ser Lys Arg Lys Glu Ala Glu
385                 390                 395                 400
Leu Arg Ser Gly Ile Ile Arg Asn Asn Ser Leu Trp Asp Arg Leu Ile
                405                 410                 415
Phe His Lys Val Gln Ser Ser Leu Gly Gly Arg Val Arg Leu Met Val
            420                 425                 430
Thr Gly Ala Ala Pro Val Ser Ala Thr Val Leu Thr Phe Leu Arg Ala
        435                 440                 445
Ala Leu Gly Cys Gln Phe Tyr Glu Gly Tyr Gly Gln Thr Glu Cys Thr
    450                 455                 460
Ala Gly Cys Cys Leu Thr Met Pro Gly Asp Trp Thr Ala Gly His Val
465                 470                 475                 480
Gly Ala Pro Met Pro Cys Asn Leu Ile Lys Leu Val Asp Val Glu Glu
                485                 490                 495
Met Asn Tyr Met Ala Ala Glu Gly Glu Gly Glu Val Cys Val Lys Gly
            500                 505                 510
Pro Asn Val Phe Gln Gly Tyr Leu Lys Asp Pro Ala Lys Thr Ala Glu
        515                 520                 525
Ala Leu Asp Lys Asp Gly Trp Leu His Thr Gly Asp Ile Gly Lys Trp
    530                 535                 540
Leu Pro Asn Gly Thr Leu Lys Ile Ile Asp Arg Lys Lys His Ile Phe
545                 550                 555                 560
Lys Leu Ala Gln Gly Glu Tyr Ile Ala Pro Glu Lys Ile Glu Asn Ile
                565                 570                 575
Tyr Met Arg Ser Glu Pro Val Ala Gln Val Phe Val His Gly Glu Ser
            580                 585                 590
Leu Gln Ala Phe Leu Ile Ala Ile Val Val Pro Asp Val Glu Thr Leu
        595                 600                 605
Cys Ser Trp Ala Gln Lys Arg Gly Phe Glu Gly Ser Phe Glu Glu Leu
    610                 615                 620
Cys Arg Asn Lys Asp Val Lys Lys Ala Ile Leu Glu Asp Met Val Arg
625                 630                 635                 640
Leu Gly Lys Asp Ser Gly Leu Lys Pro Phe Glu Gln Val Lys Gly Ile
                645                 650                 655
Thr Leu His Pro Glu Leu Phe Ser Ile Asp Asn Gly Leu Leu Thr Pro
            660                 665                 670
Thr Met Lys Ala Lys Arg Pro Glu Leu Arg Asn Tyr Phe Arg Ser Gln
        675                 680                 685
Ile Asp Asp Leu Tyr Ser Thr Ile Lys Val
    690                 695

<210>  2
<211>  2094
<212>  DNA
<213>  Homo sapiens

<400>  2
atgcaagccc atgagctgtt ccggtatttt cgaatgccag agctggttga cttccgacag      60
tacgtgcgta ctcttccgac caacacgctt atgggcttcg gagcttttgc agcactcacc     120
accttctggt acgccacgag acccaaaccc ctgaagccgc catgcgacct ctccatgcag     180
tcagtggaag tggcgggtag tggtggtgca cgaagatccg cactacttga cagcgacgag     240
cccttggtgt atttctatga tgatgtcaca acattatacg aaggtttcca gaggggaata     300
caggtgtcaa ataatggccc ttgtttaggc tctcggaaac cagaccaacc ctatgaatgg     360
ctttcatata aacaggttgc agaattgtcg gagtgcatag gctcagcact gatccagaag     420
ggcttcaaga ctgccccaga tcagttcatt ggcatctttg ctcaaaatag acctgagtgg     480
gtgattattg aacaaggatg ctttgcttat tcgatggtga tcgttccact ttatgatacc     540
cttggaaatg aagccatcac gtacatagtc aacaaagctg aactctctct ggtttttgtt     600
gacaagccag agaaggccaa actcttatta gagggtgtag aaaataagtt aataccaggc     660
cttaaaatca tagttgtcat ggatgcctac ggcagtgaac tggtggaacg aggccagagg     720
tgtggggtgg aagtcaccag catgaaggcg atggaggacc tgggaagagc caacagacgg     780
aagcccaagc ctccagcacc tgaagatctt gcagtaattt gtttcacaag tggaactaca     840
ggcaacccca aaggagcaat ggtcactcac cgaaacatag tgagcgattg ttcagctttt     900
gtgaaagcaa cagagaatac agtcaatcct tgcccagatg atactttgat atctttcttg     960
cctctcgccc atatgtttga gagagttgta gagtgtgtaa tgctgtgtca tggagctaaa    1020
atcggatttt tccaaggaga tatcaggctg ctcatggatg acctcaaggt gcttcaaccc    1080
actgtcttcc ccgtggttcc aagactgctg aaccggatgt ttgaccgaat tttcggacaa    1140
gcaaacacca cgctgaagcg atggctcttg gactttgcct ccaagaggaa agaagcagag    1200
cttcgcagcg gcatcatcag aaacaacagc ctgtgggacc ggctgatctt ccacaaagta    1260

cagtcgagcc tgggcggaag agtccggctg atggtgacag gagccgcccc ggtgtctgcc 1320
actgtgctga cgttcctcag agcagccctg ggctgtcagt tttatgaagg atacggacag 1380
acagagtgca ctgccgggtg ctgcctgacc atgcctggag actggaccgc aggccatgtt 1440
ggggccccga tgccgtgcaa tttgataaaa cttgttgatg tggaagaaat gaattacatg 1500
gctgccgagg gcgagggcga ggtgtgtgtg aaagggccaa atgtatttca gggctacttg 1560
aaggacccag cgaaaacagc agaagctttg gacaaagacg gctggttaca cacaggggac 1620
attggaaaat ggttaccaaa tggcaccttg aaaattatcg accggaaaaa gcacatattt 1680
aagctggcac aaggagaata catagcccct gaaaagattg aaaatatcta catgcgaagt 1740
gagcctgttg ctcaggtgtt tgtccacgga gaaagcctgc aggcatttct cattgcaatt 1800
gtggtaccag atgttgagac attatgttcc tgggcccaaa agagaggatt tgaagggtcg 1860
tttgaggaac tgtgcagaaa taaggatgtc aaaaaagcta tcctcgaaga tatggtgaga 1920
cttgggaagg attctggtct gaaaccattt gaacaggtca aaggcatcac attgcaccct 1980
gaattatttt ctatcgacaa tggccttctg actccaacaa tgaaggcgaa aaggccagag 2040
ctgcggaact atttcaggtc gcagatagat gacctctatt ccactatcaa ggtt 2094

<210> 3
<211> 720
<212> PRT
<213> Homo sapiens

<400> 3
Met Asn Asn His Val Ser Ser Lys Pro Ser Thr Met Lys Leu Lys His
1               5                   10                  15
Thr Ile Asn Pro Ile Leu Leu Tyr Phe Ile His Phe Leu Ile Ser Leu
            20                  25                  30
Tyr Thr Ile Leu Thr Tyr Ile Pro Phe Tyr Phe Phe Ser Glu Ser Arg
        35                  40                  45
Gln Glu Lys Ser Asn Arg Ile Lys Ala Lys Pro Val Asn Ser Lys Pro
    50                  55                  60
Asp Ser Ala Tyr Arg Ser Val Asn Ser Leu Asp Gly Leu Ala Ser Val
65                  70                  75                  80
Leu Tyr Pro Gly Cys Asp Thr Leu Asp Lys Val Phe Thr Tyr Ala Lys
                85                  90                  95
Asn Lys Phe Lys Asn Lys Arg Leu Leu Gly Thr Arg Glu Val Leu Asn
            100                 105                 110
Glu Glu Asp Glu Val Gln Pro Asn Gly Lys Ile Phe Lys Lys Val Ile
        115                 120                 125
Leu Gly Gln Tyr Asn Trp Leu Ser Tyr Glu Asp Val Phe Val Arg Ala
    130                 135                 140
Phe Asn Phe Gly Asn Gly Leu Gln Met Leu Gly Gln Lys Pro Lys Thr
145                 150                 155                 160
Asn Ile Ala Ile Phe Cys Glu Thr Arg Ala Glu Trp Met Ile Ala Ala
                165                 170                 175
Gln Ala Cys Phe Met Tyr Asn Phe Gln Leu Val Thr Leu Tyr Ala Thr
            180                 185                 190
Leu Gly Gly Pro Ala Ile Val His Ala Leu Asn Glu Thr Glu Val Thr
        195                 200                 205
Asn Ile Ile Thr Ser Lys Glu Leu Leu Gln Thr Lys Leu Lys Asp Ile
    210                 215                 220
Val Ser Leu Val Pro Arg Leu Arg His Ile Ile Thr Val Asp Gly Lys
225                 230                 235                 240
Pro Pro Thr Trp Ser Glu Phe Pro Lys Gly Ile Ile Val His Thr Met
                245                 250                 255
Ala Ala Val Glu Ala Leu Gly Ala Lys Ala Ser Met Glu Asn Gln Pro
            260                 265                 270
His Ser Lys Pro Leu Pro Ser Asp Ile Ala Val Ile Met Tyr Thr Ser
        275                 280                 285
Gly Ser Thr Gly Leu Pro Lys Gly Val Met Ile Ser His Ser Asn Ile
    290                 295                 300
Ile Ala Gly Ile Thr Gly Met Ala Glu Arg Ile Pro Glu Leu Gly Glu
305                 310                 315                 320
Glu Asp Val Tyr Ile Gly Tyr Leu Pro Leu Ala His Val Leu Glu Leu
                325                 330                 335
Ser Ala Glu Leu Val Cys Leu Ser His Gly Cys Arg Ile Gly Tyr Ser
            340                 345                 350
Ser Pro Gln Thr Leu Ala Asp Gln Ser Ser Lys Ile Lys Lys Gly Ser
        355                 360                 365
Lys Gly Asp Thr Ser Met Leu Lys Pro Thr Leu Met Ala Ala Val Pro
    370                 375                 380

Glu Ile Met Asp Arg Ile Tyr Lys Asn Val Met Asn Lys Val Ser Glu
385                 390                 395                 400
Met Ser Ser Phe Gln Arg Asn Leu Phe Ile Leu Ala Tyr Asn Tyr Lys
                405                 410                 415
Met Glu Gln Ile Ser Lys Gly Arg Asn Thr Pro Leu Cys Asp Ser Phe
            420                 425                 430
Val Phe Arg Lys Val Arg Ser Leu Leu Gly Gly Asn Ile Arg Leu Leu
        435                 440                 445
Leu Cys Gly Gly Ala Pro Leu Ser Ala Thr Thr Gln Arg Phe Met Asn
    450                 455                 460
Ile Cys Phe Cys Cys Pro Val Gly Gln Gly Tyr Gly Leu Thr Glu Ser
465                 470                 475                 480
Ala Gly Ala Gly Thr Ile Ser Glu Val Trp Asp Tyr Asn Thr Gly Arg
                485                 490                 495
Val Gly Ala Pro Leu Val Cys Cys Glu Ile Lys Leu Lys Asn Trp Glu
            500                 505                 510
Glu Gly Gly Tyr Phe Asn Thr Asp Lys Pro His Pro Arg Gly Glu Ile
        515                 520                 525
Leu Ile Gly Gly Gln Ser Val Thr Met Gly Tyr Tyr Lys Asn Glu Ala
    530                 535                 540
Lys Thr Lys Ala Asp Phe Phe Glu Asp Glu Asn Gly Gln Arg Trp Leu
545                 550                 555                 560
Cys Thr Gly Asp Ile Gly Glu Phe Glu Pro Asp Gly Cys Leu Lys Ile
                565                 570                 575
Ile Asp Arg Lys Lys Asp Leu Val Lys Leu Gln Ala Gly Glu Tyr Val
            580                 585                 590
Ser Leu Gly Lys Val Glu Ala Ala Leu Lys Asn Leu Pro Leu Val Asp
        595                 600                 605
Asn Ile Cys Ala Tyr Ala Asn Ser Tyr His Ser Tyr Val Ile Gly Phe
    610                 615                 620
Val Val Pro Asn Gln Lys Glu Leu Thr Glu Leu Ala Arg Lys Lys Gly
625                 630                 635                 640
Leu Lys Gly Thr Trp Glu Glu Leu Cys Asn Ser Cys Glu Met Glu Asn
                645                 650                 655
Glu Val Leu Lys Val Leu Ser Glu Ala Ala Ile Ser Ala Ser Leu Glu
            660                 665                 670
Lys Phe Glu Ile Pro Val Lys Ile Arg Leu Ser Pro Glu Pro Trp Thr
        675                 680                 685
Pro Glu Thr Gly Leu Val Thr Asp Ala Phe Lys Leu Lys Arg Lys Glu
    690                 695                 700
Leu Lys Thr His Tyr Gln Ala Asp Ile Glu Arg Met Tyr Gly Arg Lys
705                 710                 715                 720


<210>  4
<211>  2160
<212>  DNA
<213>  Homo sapiens

<400>  4
atgaataacc acgtgtcttc aaaaccatct accatgaagc taaaacatac catcaaccct      60
attcttttat attttataca ttttctaata tcactttata ctattttaac atacattccg     120
ttttattttt tctccgagtc aagacaagaa aaatcaaacc gaattaaagc aaagcctgta     180
aattcaaaac ctgattctgc atacagatct gttaatagtt tggatggttt ggcttcagta     240
ttataccctg gatgtgatac tttagataaa gtttttacat atgcaaaaaa caaatttaag     300
aacaaaagac tcttgggaac acgtgaagtt ttaaatgagg aagatgaagt acaaccaaat     360
ggaaaaattt ttaaaaaggt tattcttgga cagtataatt ggctttccta tgaagatgtc     420
tttgttcgag cctttaattt tggaaatgga ttacagatgt tgggtcagaa accaaagacc     480
aacatcgcca tcttctgtga gaccagggcc gagtggatga tagctgcaca ggcgtgtttt     540
atgtataatt ttcagcttgt tacattatat gccactctag gaggtccagc cattgttcat     600
gcattaaatg aaacagaggt gaccaacatc attactagta aagaactctt acaaacaaag     660
ttgaaggata tagtttcttt ggtcccacgc ctgcggcaca tcatcactgt tgatggaaag     720
ccaccgacct ggtccgagtt ccccaagggc atcattgtgc ataccatggc tgcagtggag     780
gccctgggag ccaaggccag catggaaaac caacctcata gcaaaccatt gccctcagat     840
attgcagtaa tcatgtacac aagtggatcc acaggacttc caaagggagt catgatctca     900
catagtaaca ttattgctgg tataactggg atggcagaaa ggattccaga actaggagag     960
gaagatgtct acattggata tttgcctctg gcccatgttc tagaattaag tgctgagctt    1020
gtctgtcttt ctcacggatg ccgcattggt tactcttcac cacagacttt agcagatcag    1080
tcttcaaaaa ttaaaaaagg aagcaaaggg gatacatcca tgttgaaacc aacactgatg    1140
gcagcagttc cggaaatcat ggatcggatc tacaaaaatg tcatgaataa agtcagtgaa    1200

atgagtagtt ttcaacgtaa tctgtttatt ctggcctata attacaaaat ggaacagatt 1260
tcaaaaggac gtaatactcc actgtgcgac agctttgttt tccggaaagt tcgaagcttg 1320
ctagggggaa atattcgtct cctgttgtgt ggtggcgctc cactttctgc aaccacgcag 1380
cgattcatga acatctgttt ctgctgtcct gttggtcagg gatacgggct cactgaatct 1440
gctggggctg gaacaatttc cgaagtgtgg gactacaata ctggcagagt gggagcacca 1500
ttagtttgct gtgaaatcaa attaaaaaac tgggaggaag gtggatactt taatactgat 1560
aagccacacc ccaggggtga aattcttatt gggggccaaa gtgtgacaat ggggtactac 1620
aaaaatgaag caaaaacaaa agctgatttc tttgaagatg aaaatggaca aaggtggctc 1680
tgtactgggg atattggaga gtttgaaccc gatggatgct taaagattat tgatcgtaaa 1740
aaggaccttg taaaactaca ggcaggggaa tatgtttctc ttgggaaagt agaggcagct 1800
ttgaagaatc ttccactagt agataacatt tgtgcatatg caaacagtta tcattcttat 1860
gtcattggat ttgttgtgcc aaatcaaaag gaactaactg aactagctcg aaagaaagga 1920
cttaaaggga cttgggagga gctgtgtaac agttgtgaaa tggaaaatga ggtacttaaa 1980
gtgctttccg aagctgctat ttcagcaagt ctggaaaagt ttgaaattcc agtaaaaatt 2040
cgtttgagtc ctgaaccgtg gacccctgaa actggtctgg tgacagatgc cttcaagctg 2100
aaacgcaaag agcttaaaac acattaccag gcggacattg agcgaatgta tggaagaaaa 2160

<210> 5
<211> 670
<212> PRT
<213> Homo sapiens

<400> 5

<210>  6
<211>  2010
<212>  DNA
<213>  Homo sapiens

<400>  6
atggcaaaga gaataaaagc taagcccact tcagacaaac ctggaagtcc atatcgctct      60
gtcacacact tcgactcact agctgtaata gacatccctg gagcagatac tctggataaa     120
ttatttgacc atgctgtatc caagtttggg aagaaggaca gccttgggac cagggaaatc     180
ctaagtgaag aaaatgaaat gcagccaaat ggaaaagttt ttaagaagtt aattcttggg     240
aattataaat ggatgaacta tcttgaagtg aatcgcagag tgaataactt tggtagtgga     300
ctcactgcac tgggactaaa accaaagaac accattgcca tcttctgtga gaccagggcc     360
gaatggatga ttgcagcaca gacctgcttt aagtacaact ttcctcttgt gactttatat     420
gccacacttg gcaaagaagc agtagttcat gggctaaatg aatctgaggc ttcctatctg     480
attaccagtg ttgaacttct ggaaagtaaa cttaagactg cattgttaga tatcagttgt     540
gttaaacata tcatttatgt ggacaataag gctatcaata aagcagagta ccctgaagga     600
tttgagattc acagcatgca atcagtagaa gagttgggat ctaacccaga aaacttgggc     660
attcctccaa gtagaccaac gccttcagac atggccattg ttatgtatac tagtggttct     720
actggccgac ctaagggagt gatgatgcat catagcaatt tgatagctgg aatgacaggc     780
cagtgtgaaa gaatacctgg actgggaccg aaggacacat atattggcta cttgcctttg     840
gctcatgtgc tagaactgac agcagagata tcttgcttta cctatggctg caggattgga     900
tattcttctc cgcttacact ctctgaccag tccagcaaaa ttaaaaaagg aagcaaagga     960
gactgtactg tactgaagcc cacacttatg gctgctgttc cggaaatcat ggatagaatt    1020
tataagaatg ttatgagcaa agtccaagag atgaattata ttcagaaaac tctgttcaag    1080
atagggtatg attacaaatt ggaacagatc aaaaagggat atgatgcacc tctttgcaat    1140
ctgttactgt ttaaaaaggt caaggccctg ctgggaggga atgtccgcat gatgctgtct    1200
ggaggggccc cgctatctcc tcagacacac cgattcatga atgtctgctt ctgctgccca    1260
attggccagg gttatggact gacagaatca tgtggtgctg ggacagttac tgaagtaact    1320
gactatacta ctggcagagt tggagcacct cttatttgct gtgaaattaa gctaaaagac    1380
tggcaagaag gcggttatac aattaatgac aagccaaacc ccagaggtga aatcgtaatt    1440

ggtggacaga acatctccat gggatatttt aaaaatgaag agaaaacagc agaagattat 1500
tctgtggatg aaaatggaca aaggtggttt tgcactggtg atattggaga attccatccc 1560
gatggatgtt tacagattat agatcgtaag aaagatctag tgaagttaca agcaggagag 1620
tatgtatctc ttgggaaagt agaagctgca ctgaagaatt gtccacttat tgacaacatc 1680
tgtgcttttg ccaaaagtga tcagtcctat gtgatcagtt ttgtggttcc taaccagaaa 1740
aggttgacac ttttggcaca acagaaaggg gtagaaggaa cttgggttga tatctgcaat 1800
aatcctgcta tggaagctga aatactgaaa gaaattcgag aagctgcaaa tgccatgaaa 1860
ttggagcgat ttgaaattcc aatcaaggtt cgattaagcc cagagccatg gacccctgaa 1920
actggtttgg taactgatgc tttcaaactg aaaaggaagg agctgaggaa ccattacctc 1980
aaagacattg aacgaatgta tgggggcaaa 2010

<210> 7
<211> 711
<212> PRT
<213> Homo sapiens

<400> 7
Met Lys Leu Lys Leu Asn Val Leu Thr Ile Ile Leu Leu Pro Val His
1               5                   10                  15
Leu Leu Ile Thr Ile Tyr Ser Ala Leu Ile Phe Ile Pro Trp Tyr Phe
            20                  25                  30
Leu Thr Asn Ala Lys Lys Lys Asn Ala Met Ala Lys Arg Ile Lys Ala
        35                  40                  45
Lys Pro Thr Ser Asp Lys Pro Gly Ser Pro Tyr Arg Ser Val Thr His
    50                  55                  60
Phe Asp Ser Leu Ala Val Ile Asp Ile Pro Gly Ala Asp Thr Leu Asp
65                  70                  75                  80
Lys Leu Phe Asp His Ala Val Ser Lys Phe Gly Lys Lys Asp Ser Leu
                85                  90                  95
Gly Thr Arg Glu Ile Leu Ser Glu Glu Asn Glu Met Gln Pro Asn Gly
            100                 105                 110
Lys Val Phe Lys Lys Leu Ile Leu Gly Asn Tyr Lys Trp Met Asn Tyr
        115                 120                 125
Leu Glu Val Asn Arg Arg Val Asn Asn Phe Gly Ser Gly Leu Thr Ala
    130                 135                 140
Leu Gly Leu Lys Pro Lys Asn Thr Ile Ala Ile Phe Cys Glu Thr Arg
145                 150                 155                 160
Ala Glu Trp Met Ile Ala Ala Gln Thr Cys Phe Lys Tyr Asn Phe Pro
                165                 170                 175
Leu Val Thr Leu Tyr Ala Thr Leu Gly Lys Glu Ala Val Val His Gly
            180                 185                 190
Leu Asn Glu Ser Glu Ala Ser Tyr Leu Ile Thr Ser Val Glu Leu Leu
        195                 200                 205
Glu Ser Lys Leu Lys Thr Ala Leu Leu Asp Ile Ser Cys Val Lys His
    210                 215                 220
Ile Ile Tyr Val Asp Asn Lys Ala Ile Asn Lys Ala Glu Tyr Pro Glu
225                 230                 235                 240
Gly Phe Glu Ile His Ser Met Gln Ser Val Glu Glu Leu Gly Ser Asn
                245                 250                 255
Pro Glu Asn Leu Gly Ile Pro Pro Ser Arg Pro Thr Pro Ser Asp Met
            260                 265                 270
Ala Ile Val Met Tyr Thr Ser Gly Ser Thr Gly Arg Pro Lys Gly Val
        275                 280                 285
Met Met His His Ser Asn Leu Ile Ala Gly Met Thr Gly Gln Cys Glu
    290                 295                 300
Arg Ile Pro Gly Leu Gly Pro Lys Asp Thr Tyr Ile Gly Tyr Leu Pro
305                 310                 315                 320
Leu Ala His Val Leu Glu Leu Thr Ala Glu Ile Ser Cys Phe Thr Tyr
                325                 330                 335
Gly Cys Arg Ile Gly Tyr Ser Ser Pro Leu Thr Leu Ser Asp Gln Ser
            340                 345                 350
Ser Lys Ile Lys Lys Gly Ser Lys Gly Asp Cys Thr Val Leu Lys Pro
        355                 360                 365
Thr Leu Met Ala Ala Val Pro Glu Ile Met Asp Arg Ile Tyr Lys Asn
    370                 375                 380
Val Met Ser Lys Val Gln Glu Met Asn Tyr Ile Gln Lys Thr Leu Phe
385                 390                 395                 400
Lys Ile Gly Tyr Asp Tyr Lys Leu Glu Gln Ile Lys Lys Gly Tyr Asp
                405                 410                 415

Ala Pro Leu Cys Asn Leu Leu Leu Phe Lys Lys Val Lys Ala Leu Leu
            420                 425                 430
Gly Gly Asn Val Arg Met Met Leu Ser Gly Gly Ala Pro Leu Ser Pro
        435                 440                 445
Gln Thr His Arg Phe Met Asn Val Cys Phe Cys Cys Pro Ile Gly Gln
    450                 455                 460
Gly Tyr Gly Leu Thr Glu Ser Cys Gly Ala Gly Thr Val Thr Glu Val
465                 470                 475                 480
Thr Asp Tyr Thr Thr Gly Arg Val Gly Ala Pro Leu Ile Cys Cys Glu
                485                 490                 495
Ile Lys Leu Lys Asp Trp Gln Glu Gly Gly Tyr Thr Ile Asn Asp Lys
            500                 505                 510
Pro Asn Pro Arg Gly Glu Ile Val Ile Gly Gly Gln Asn Ile Ser Met
        515                 520                 525
Gly Tyr Phe Lys Asn Glu Glu Lys Thr Ala Glu Asp Tyr Ser Val Asp
    530                 535                 540
Glu Asn Gly Gln Arg Trp Phe Cys Thr Gly Asp Ile Gly Glu Phe His
545                 550                 555                 560
Pro Asp Gly Cys Leu Gln Ile Ile Asp Arg Lys Lys Asp Leu Val Lys
                565                 570                 575
Leu Gln Ala Gly Glu Tyr Val Ser Leu Gly Lys Val Glu Ala Ala Leu
            580                 585                 590
Lys Asn Cys Pro Leu Ile Asp Asn Ile Cys Ala Phe Ala Lys Ser Asp
        595                 600                 605
Gln Ser Tyr Val Ile Ser Phe Val Val Pro Asn Gln Lys Arg Leu Thr
    610                 615                 620
Leu Leu Ala Gln Gln Lys Gly Val Glu Gly Thr Trp Val Asp Ile Cys
625                 630                 635                 640
Asn Asn Pro Ala Met Glu Ala Glu Ile Leu Lys Glu Ile Arg Glu Ala
                645                 650                 655
Ala Asn Ala Met Lys Leu Glu Arg Phe Glu Ile Pro Ile Lys Val Arg
            660                 665                 670
Leu Ser Pro Glu Pro Trp Thr Pro Glu Thr Gly Leu Val Thr Asp Ala
        675                 680                 685
Phe Lys Leu Lys Arg Lys Glu Leu Arg Asn His Tyr Leu Lys Asp Ile
    690                 695                 700
Glu Arg Met Tyr Gly Gly Lys
705                 710


<210>  8
<211>  2133
<212>  DNA
<213>  Homo sapiens

<400>  8
   atgaaactta agctaaatgt gctcaccatt attttgctgc ctgtccactt gttaataaca      60
   atatacagtg cccttatatt tattccatgg tattttctta ccaatgccaa gaagaaaaac     120
   gctatggcaa agagaataaa agctaagccc acttcagaca aacctggaag tccatatcgc     180
   tctgtcacac acttcgactc actagctgta atagacatcc ctggagcaga tactctggat     240
   aaattatttg accatgctgt atccaagttt gggaagaagg acagccttgg gaccagggaa     300
   atcctaagtg aagaaaatga aatgcagcca aatggaaaag tttttaagaa gttaattctt     360
   gggaattata aatggatgaa ctatcttgaa gtgaatcgca gagtgaataa ctttggtagt     420
   ggactcactg cactgggact aaaaccaaag aacaccattg ccatcttctg tgagaccagg     480
   gccgaatgga tgattgcagc acagacctgc tttaagtaca actttcctct tgtgacttta     540
   tatgccacac ttggcaaaga agcagtagtt catgggctaa atgaatctga ggcttcctat     600
   ctgattacca gtgttgaact tctggaaagt aaacttaaga ctgcattgtt agatatcagt     660
   tgtgttaaac atatcattta tgtggacaat aaggctatca ataaagcaga gtaccctgaa     720
   ggatttgaga ttcacagcat gcaatcagta gaagagttgg gatctaaccc agaaaacttg     780
   ggcattcctc caagtagacc aacgccttca gacatggcca ttgttatgta tactagtggt     840
   tctactggcc gacctaaggg agtgatgatg catcatagca atttgatagc tggaatgaca     900
   ggccagtgtg aaagaatacc tggactggga ccgaaggaca catatattgg ctacttgcct     960
   ttggctcatg tgctagaact gacagcagag atatcttgct ttacctatgg ctgcaggatt    1020
   ggatattctt ctccgcttac actctctgac cagtccagca aaattaaaaa aggaagcaaa    1080
   ggagactgta ctgtactgaa gcccacactt atggctgctg ttccggaaat catggataga    1140
   atttataaga atgttatgag caaagtccaa gagatgaatt atattcagaa aactctgttc    1200
   aagataggtt atgattacaa attggaacag atcaaaaagg gatatgatgc acctctttgc    1260
   aatctgttac tgtttaaaaa ggtcaaggcc ctgctgggag ggaatgtccg catgatgctg    1320
   tctggagggg ccccgctatc tcctcagaca caccgattca tgaatgtctg cttctgctgc    1380
   ccaattggcc agggttatgg actgacagaa tcatgtggtg ctgggacagt tactgaagta    1440
   actgactata ctactggcag agttggagca cctcttattt gctgtgaaat taagctaaaa 1500
   gactggcaag aaggcggtta tacaattaat gacaagccaa accccagagg tgaaatcgta 1560
   attggtggac agaacatctc catgggatat tttaaaaatg aagagaaaac agcagaagat 1620
   tattctgtgg atgaaaatgg acaaaggtgg ttttgcactg gtgatattgg agaattccat 1680
   cccgatggat gtttacagat tatagatcgt aagaaagatc tagtgaagtt acaagcagga 1740
   gagtatgtat ctcttgggaa agtagaagct gcactgaaga attgtccact tattgacaac 1800
   atctgtgctt ttgccaaaag tgatcagtcc tatgtgatca gttttgtggt tcctaaccag 1860
   aaaaggttga cacttttggc acaacagaaa ggggtagaag gaacttgggt tgatatctgc 1920
   aataatcctg ctatggaagc tgaaatactg aaagaaattc gagaagctgc aaatgccatg 1980
   aaattggagc gatttgaaat tccaatcaag gttcgattaa gcccagagcc atggacccct 2040
   gaaactggtt tggtaactga tgctttcaaa ctgaaaagga aggagctgag gaaccattac 2100
   ctcaaagaca ttgaacgaat gtatgggggc aaa 2133

<210> 9
<211> 739
<212> PRT
<213> Homo sapiens

<400> 9
Met Asp Ala Leu Lys Pro Pro Cys Leu Trp Arg Asn His Glu Arg Gly
1               5                   10                  15
Lys Lys Asp Arg Asp Ser Cys Gly Arg Lys Asn Ser Glu Pro Gly Ser
            20                  25                  30
Pro His Ser Leu Glu Ala Leu Arg Asp Ala Ala Pro Ser Gln Gly Leu
        35                  40                  45
Asn Phe Leu Leu Leu Phe Thr Lys Met Leu Phe Ile Phe Asn Phe Leu
    50                  55                  60
Phe Ser Pro Leu Pro Thr Pro Ala Leu Ile Cys Ile Leu Thr Phe Gly
65                  70                  75                  80
Ala Ala Ile Phe Leu Trp Leu Ile Thr Arg Pro Gln Pro Val Leu Pro
                85                  90                  95
Leu Leu Asp Leu Asn Asn Gln Ser Val Gly Ile Glu Gly Gly Ala Arg
            100                 105                 110
Lys Gly Val Ser Gln Lys Asn Asn Asp Leu Thr Ser Cys Cys Phe Ser
        115                 120                 125
Asp Ala Lys Thr Met Tyr Glu Val Phe Gln Arg Gly Leu Ala Val Ser
    130                 135                 140
Asp Asn Gly Pro Cys Leu Gly Tyr Arg Lys Pro Asn Gln Pro Tyr Arg
145                 150                 155                 160
Trp Leu Ser Tyr Lys Gln Val Ser Asp Arg Ala Glu Tyr Leu Gly Ser
                165                 170                 175
Cys Leu Leu His Lys Gly Tyr Lys Ser Ser Pro Asp Gln Phe Val Gly
            180                 185                 190
Ile Phe Ala Gln Asn Arg Pro Glu Trp Ile Ile Ser Glu Leu Ala Cys
        195                 200                 205
Tyr Thr Tyr Ser Met Val Ala Val Pro Leu Tyr Asp Thr Leu Gly Pro
    210                 215                 220
Glu Ala Ile Val His Ile Val Asn Lys Ala Asp Ile Ala Met Val Ile
225                 230                 235                 240
Cys Asp Thr Pro Gln Lys Ala Leu Val Leu Ile Gly Asn Val Glu Lys
                245                 250                 255
Gly Phe Thr Pro Ser Leu Lys Val Ile Ile Leu Met Asp Pro Phe Asp
            260                 265                 270
Asp Asp Leu Lys Gln Arg Gly Glu Lys Ser Gly Ile Glu Ile Leu Ser
        275                 280                 285
Leu Tyr Asp Ala Glu Asn Leu Gly Lys Glu His Phe Arg Lys Pro Val
    290                 295                 300
Pro Pro Ser Pro Glu Asp Leu Ser Val Ile Cys Phe Thr Ser Gly Thr
305                 310                 315                 320
Thr Gly Asp Pro Lys Gly Ala Met Ile Thr His Gln Asn Ile Val Ser
                325                 330                 335
Asn Ala Ala Ala Phe Leu Lys Cys Val Glu His Ala Tyr Glu Pro Thr
            340                 345                 350
Pro Asp Asp Val Ala Ile Ser Tyr Leu Pro Leu Ala His Met Phe Glu
        355                 360                 365
Arg Ile Val Gln Ala Val Val Tyr Ser Cys Gly Ala Arg Val Gly Phe
    370                 375                 380
Phe Gln Gly Asp Ile Arg Leu Leu Ala Asp Asp Met Lys Thr Leu Lys
385                 390                 395                 400

Pro Thr Leu Phe Pro Ala Val Pro Arg Leu Leu Asn Arg Ile Tyr Asp
                405                 410                 415
Lys Val Gln Asn Glu Ala Lys Thr Pro Leu Lys Lys Phe Leu Leu Lys
            420                 425                 430
Leu Ala Val Ser Ser Lys Phe Lys Glu Leu Gln Lys Gly Ile Ile Arg
        435                 440                 445
His Asp Ser Phe Trp Asp Lys Leu Ile Phe Ala Lys Ile Gln Asp Ser
    450                 455                 460
Leu Gly Gly Arg Val Arg Val Ile Val Thr Gly Ala Ala Pro Met Ser
465                 470                 475                 480
Thr Ser Val Met Thr Phe Phe Arg Ala Ala Met Gly Cys Gln Val Tyr
                485                 490                 495
Glu Ala Tyr Gly Gln Thr Glu Cys Thr Gly Gly Cys Thr Phe Thr Leu
            500                 505                 510
Pro Gly Asp Trp Thr Ser Gly His Val Gly Val Pro Leu Ala Cys Asn
        515                 520                 525
Tyr Val Lys Leu Glu Asp Val Ala Asp Met Asn Tyr Phe Thr Val Asn
    530                 535                 540
Asn Glu Gly Glu Val Cys Ile Lys Gly Thr Asn Val Phe Lys Gly Tyr
545                 550                 555                 560
Leu Lys Asp Pro Glu Lys Thr Gln Glu Ala Leu Asp Ser Asp Gly Trp
                565                 570                 575
Leu His Thr Gly Asp Ile Gly Arg Trp Leu Pro Asn Gly Thr Leu Lys
            580                 585                 590
Ile Ile Asp Arg Lys Lys Asn Ile Phe Lys Leu Ala Gln Gly Glu Tyr
        595                 600                 605
Ile Ala Pro Glu Lys Ile Glu Asn Ile Tyr Asn Arg Ser Gln Pro Val
    610                 615                 620
Leu Gln Ile Phe Val His Gly Glu Ser Leu Arg Ser Ser Leu Val Gly
625                 630                 635                 640
Val Val Val Pro Asp Thr Asp Val Leu Pro Ser Phe Ala Ala Lys Leu
                645                 650                 655
Gly Val Lys Gly Ser Phe Glu Glu Leu Cys Gln Asn Gln Val Val Arg
            660                 665                 670
Glu Ala Ile Leu Glu Asp Leu Gln Lys Ile Gly Lys Glu Ser Gly Leu
        675                 680                 685
Lys Thr Phe Glu Gln Val Lys Ala Ile Phe Leu His Pro Glu Pro Phe
    690                 695                 700
Ser Ile Glu Asn Gly Leu Leu Thr Pro Thr Leu Lys Ala Lys Arg Gly
705                 710                 715                 720
Glu Leu Ser Lys Tyr Phe Arg Thr Gln Ile Asp Ser Leu Tyr Glu His
                725                 730                 735
Ile Gln Asp


<210>  10
<211>  2217
<212>  DNA
<213>  Homo sapiens

<400>  10
   atggacgctc tgaagccacc ctgtctctgg aggaaccacg agcgagggaa gaaggacagg      60
   gactcgtgtg gcaggaagaa ctcagagccg ggaagccccc attcactaga agcactgaga     120
   gatgcggccc cctcgcaggg tctgaatttc ctgctgctgt tcacaaagat gctttttatc     180
   tttaactttt tgttttcccc acttccgacc ccggcgttga tctgcatcct gacatttgga     240
   gctgccatct tcttgtggct gatcaccaga cctcaacccg tcttacctct tcttgacctg     300
   aacaatcagt ctgtgggaat tgagggagga gcacggaagg gggtttccca gaagaacaat     360
   gacctaacaa gttgctgctt ctcagatgcc aagactatgt atgaggtttt ccaaagagga     420
   ctcgctgtgt ctgacaatgg gccctgcttg ggatatagaa aaccaaacca gccctacaga     480
   tggctatctt acaaacaggt gtctgataga gcagagtacc tgggttcctg tctcttgcat     540
   aaaggttata aatcatcacc agaccagttt gtcggcatct ttgctcagaa taggccagag     600
   tggatcatct ccgaattggc ttgttacacg tactctatgg tagctgtacc tctgtatgac     660
   accttgggac cagaagccat cgtacatatt gtcaacaagg ctgatatcgc catggtgatc     720
   tgtgacacac cccaaaaggc attggtgctg atagggaatg tagagaaagg cttcaccccg     780
   agcctgaagg tgatcatcct tatggacccc tttgatgatg acctgaagca aagaggggag     840
   aagagtggaa ttgagatctt atccctatat gatgctgaga acctaggcaa agagcacttc     900
   agaaaacctg tgcctcctag cccagaagac ctgagcgtca tctgcttcac cagtgggacc     960
   acaggtgacc ccaaaggagc catgataacc catcaaaata ttgtttcaaa tgctgctgcc    1020
   tttctcaaat gtgtggagca tgcttatgag cccactcctg atgatgtggc catatcctac    1080
   ctccctctgg ctcatatgtt tgagaggatt gtacaggctg ttgtgtacag ctgtggagcc    1140
   agagttggat tcttccaagg ggatattcgg ttgctggctg acgacatgaa gactttgaag 1200
   cccacattgt ttcccgcggt gcctcgactc cttaacagga tctacgataa ggtacaaaat 1260
   gaggccaaga cacccttgaa gaagttcttg ttgaagctgg ctgtttccag taaattcaaa 1320
   gagcttcaaa agggtatcat caggcatgat agtttctggg acaagctcat ctttgcaaag 1380
   atccaggaca gcctgggcgg aagggttcgt gtaattgtca ctggagctgc ccccatgtcc 1440
   acttcagtca tgacattctt ccgggcagca atgggatgtc aggtgtatga agcttatggt 1500
   caaacagaat gcacaggtgg ctgtacattt acattacctg gggactggac atcaggtcac 1560
   gttggggtgc ccctggcttg caattacgtg aagctggaag atgtggctga catgaactac 1620
   tttacagtga ataatgaagg agaggtctgc atcaagggta caaacgtgtt caaaggatac 1680
   ctgaaggacc ctgagaagac acaggaagcc ctggacagtg atggctggct tcacacagga 1740
   gacattggtc gctggctccc gaatggaact ctgaagatca tcgaccgtaa aaagaacatt 1800
   ttcaagctgg cccaaggaga atacattgca ccagagaaga tagaaaatat ctacaacagg 1860
   agtcaaccag tgttacaaat ttttgtacac ggggagagct tacggtcatc cttagtagga 1920
   gtggtggttc ctgacacaga tgtacttccc tcatttgcag ccaagcttgg ggtgaagggc 1980
   tcctttgagg aactgtgcca aaaccaagtt gtaagggaag ccattttaga agacttgcag 2040
   aaaattggga aagaaagtgg ccttaaaact tttgaacagg tcaaagccat ttttcttcat 2100
   ccagagccat tttccattga aaatgggctc ttgacaccaa cattgaaagc aaagcgagga 2160
   gagctttcca aatactttcg gacccaaatt gacagcctgt atgagcacat ccaggat 2217

<210> 11
<211> 683
<212> PRT
<213> Homo sapiens

<400> 11
Met Leu Phe Ile Phe Asn Phe Leu Phe Ser Pro Leu Pro Thr Pro Ala
1               5                   10                  15
Leu Ile Cys Ile Leu Thr Phe Gly Ala Ala Ile Phe Leu Trp Leu Ile
            20                  25                  30
Thr Arg Pro Gln Pro Val Leu Pro Leu Leu Asp Leu Asn Asn Gln Ser
        35                  40                  45
Val Gly Ile Glu Gly Gly Ala Arg Lys Gly Val Ser Gln Lys Asn Asn
    50                  55                  60
Asp Leu Thr Ser Cys Cys Phe Ser Asp Ala Lys Thr Met Tyr Glu Val
65                  70                  75                  80
Phe Gln Arg Gly Leu Ala Val Ser Asp Asn Gly Pro Cys Leu Gly Tyr
                85                  90                  95
Arg Lys Pro Asn Gln Pro Tyr Arg Trp Leu Ser Tyr Lys Gln Val Ser
            100                 105                 110
Asp Arg Ala Glu Tyr Leu Gly Ser Cys Leu Leu His Lys Gly Tyr Lys
        115                 120                 125
Ser Ser Pro Asp Gln Phe Val Gly Ile Phe Ala Gln Asn Arg Pro Glu
    130                 135                 140
Trp Ile Ile Ser Glu Leu Ala Cys Tyr Thr Tyr Ser Met Val Ala Val
145                 150                 155                 160
Pro Leu Tyr Asp Thr Leu Gly Pro Glu Ala Ile Val His Ile Val Asn
                165                 170                 175
Lys Ala Asp Ile Ala Met Val Ile Cys Asp Thr Pro Gln Lys Ala Leu
            180                 185                 190
Val Leu Ile Gly Asn Val Glu Lys Gly Phe Thr Pro Ser Leu Lys Val
        195                 200                 205
Ile Ile Leu Met Asp Pro Phe Asp Asp Asp Leu Lys Gln Arg Gly Glu
    210                 215                 220
Lys Ser Gly Ile Glu Ile Leu Ser Leu Tyr Asp Ala Glu Asn Leu Gly
225                 230                 235                 240
Lys Glu His Phe Arg Lys Pro Val Pro Pro Ser Pro Glu Asp Leu Ser
                245                 250                 255
Val Ile Cys Phe Thr Ser Gly Thr Thr Gly Asp Pro Lys Gly Ala Met
            260                 265                 270
Ile Thr His Gln Asn Ile Val Ser Asn Ala Ala Ala Phe Leu Lys Cys
        275                 280                 285
Val Glu His Ala Tyr Glu Pro Thr Pro Asp Asp Val Ala Ile Ser Tyr
    290                 295                 300
Leu Pro Leu Ala His Met Phe Glu Arg Ile Val Gln Ala Val Val Tyr
305                 310                 315                 320
Ser Cys Gly Ala Arg Val Gly Phe Phe Gln Gly Asp Ile Arg Leu Leu
                325                 330                 335
Ala Asp Asp Met Lys Thr Leu Lys Pro Thr Leu Phe Pro Ala Val Pro
            340                 345                 350

Arg Leu Leu Asn Arg Ile Tyr Asp Lys Val Gln Asn Glu Ala Lys Thr
        355                 360                 365
Pro Leu Lys Lys Phe Leu Leu Lys Leu Ala Val Ser Ser Lys Phe Lys
    370                 375                 380
Glu Leu Gln Lys Gly Ile Ile Arg His Asp Ser Phe Trp Asp Lys Leu
385                 390                 395                 400
Ile Phe Ala Lys Ile Gln Asp Ser Leu Gly Gly Arg Val Arg Val Ile
                405                 410                 415
Val Thr Gly Ala Ala Pro Met Ser Thr Ser Val Met Thr Phe Phe Arg
            420                 425                 430
Ala Ala Met Gly Cys Gln Val Tyr Glu Ala Tyr Gly Gln Thr Glu Cys
        435                 440                 445
Thr Gly Gly Cys Thr Phe Thr Leu Pro Gly Asp Trp Thr Ser Gly His
    450                 455                 460
Val Gly Val Pro Leu Ala Cys Asn Tyr Val Lys Leu Glu Asp Val Ala
465                 470                 475                 480
Asp Met Asn Tyr Phe Thr Val Asn Asn Glu Gly Glu Val Cys Ile Lys
                485                 490                 495
Gly Thr Asn Val Phe Lys Gly Tyr Leu Lys Asp Pro Glu Lys Thr Gln
            500                 505                 510
Glu Ala Leu Asp Ser Asp Gly Trp Leu His Thr Gly Asp Ile Gly Arg
        515                 520                 525
Trp Leu Pro Asn Gly Thr Leu Lys Ile Ile Asp Arg Lys Lys Asn Ile
    530                 535                 540
Phe Lys Leu Ala Gln Gly Glu Tyr Ile Ala Pro Glu Lys Ile Glu Asn
545                 550                 555                 560
Ile Tyr Asn Arg Ser Gln Pro Val Leu Gln Ile Phe Val His Gly Glu
                565                 570                 575
Ser Leu Arg Ser Ser Leu Val Gly Val Val Val Pro Asp Thr Asp Val
            580                 585                 590
Leu Pro Ser Phe Ala Ala Lys Leu Gly Val Lys Gly Ser Phe Glu Glu
        595                 600                 605
Leu Cys Gln Asn Gln Val Val Arg Glu Ala Ile Leu Glu Asp Leu Gln
    610                 615                 620
Lys Ile Gly Lys Glu Ser Gly Leu Lys Thr Phe Glu Gln Val Lys Ala
625                 630                 635                 640
Ile Phe Leu His Pro Glu Pro Phe Ser Ile Glu Asn Gly Leu Leu Thr
                645                 650                 655
Pro Thr Leu Lys Ala Lys Arg Gly Glu Leu Ser Lys Tyr Phe Arg Thr
            660                 665                 670
Gln Ile Asp Ser Leu Tyr Glu His Ile Gln Asp
        675                 680


<210>  12
<211>  2049
<212>  DNA
<213>  Homo sapiens

<400>  12
atgcttttta tctttaactt tttgttttcc ccacttccga ccccggcgtt gatctgcatc      60
ctgacatttg gagctgccat cttcttgtgg ctgatcacca gacctcaacc cgtcttacct     120
cttcttgacc tgaacaatca gtctgtggga attgagggag gagcacggaa gggggtttcc     180
cagaagaaca atgacctaac aagttgctgc ttctcagatg ccaagactat gtatgaggtt     240
ttccaaagag gactcgctgt gtctgacaat gggccctgct tgggatatag aaaaccaaac     300
cagccctaca gatggctatc ttacaaacag gtgtctgata gagcagagta cctgggttcc     360
tgtctcttgc ataaaggtta taaatcatca ccagaccagt ttgtcggcat ctttgctcag     420
aataggccag agtggatcat ctccgaattg gcttgttaca cgtactctat ggtagctgta     480
cctctgtatg acaccttggg accagaagcc atcgtacata ttgtcaacaa ggctgatatc     540
gccatggtga tctgtgacac accccaaaag gcattggtgc tgatagggaa tgtagagaaa     600
ggcttcaccc cgagcctgaa ggtgatcatc cttatggacc cctttgatga tgacctgaag     660
caaagagggg agaagagtgg aattgagatc ttatccctat atgatgctga gaacctaggc     720
aaagagcact tcagaaaacc tgtgcctcct agcccagaag acctgagcgt catctgcttc     780
accagtggga ccacaggtga ccccaaagga gccatgataa cccatcaaaa tattgtttca     840
aatgctgctg cctttctcaa atgtgtggag catgcttatg agcccactcc tgatgatgtg     900
gccatatcct acctccctct ggctcatatg tttgagagga ttgtacaggc tgttgtgtac     960
agctgtggag ccagagttgg attcttccaa ggggatattc ggttgctggc tgacgacatg    1020
aagactttga agcccacatt gtttcccgcg gtgcctcgac tccttaacag gatctacgat    1080
aaggtacaaa atgaggccaa gacacccttg aagaagttct tgttgaagct ggctgtttcc    1140
agtaaattca aagagcttca aaagggtatc atcaggcatg atagtttctg ggacaagctc    1200

atctttgcaa agatccagga cagcctgggc ggaagggttc gtgtaattgt cactggagct 1260
gcccccatgt ccacttcagt catgacattc ttccgggcag caatgggatg tcaggtgtat 1320
gaagcttatg gtcaaacaga atgcacaggt ggctgtacat ttacattacc tggggactgg 1380
acatcaggtc acgttggggt gcccctggct tgcaattacg tgaagctgga agatgtggct 1440
gacatgaact actttacagt gaataatgaa ggagaggtct gcatcaaggg tacaaacgtg 1500
ttcaaaggat acctgaagga ccctgagaag acacaggaag ccctggacag tgatggctgg 1560
cttcacacag gagacattgg tcgctggctc ccgaatggaa ctctgaagat catcgaccgt 1620
aaaaagaaca ttttcaagct ggcccaagga gaatacattg caccagagaa gatagaaaat 1680
atctacaaca ggagtcaacc agtgttacaa atttttgtac acggggagag cttacggtca 1740
tccttagtag gagtggtggt tcctgacaca gatgtacttc cctcatttgc agccaagctt 1800
ggggtgaagg gctcctttga ggaactgtgc caaaaccaag ttgtaaggga agccatttta 1860
gaagacttgc agaaaattgg gaaagaaagt ggccttaaaa cttttgaaca ggtcaaagcc 1920
atttttcttc atccagagcc attttccatt gaaaatgggc tcttgacacc aacattgaaa 1980
gcaaagcgag gagagctttc caaatacttt cggacccaaa ttgacagcct gtatgagcac 2040
atccaggat 2049

<210> 13
<211> 722
<212> PRT
<213> Homo sapiens

<400> 13
Met Leu Thr Phe Phe Leu Val Ser Gly Gly Ser Leu Trp Leu Phe Val
1               5                   10                  15
Glu Phe Val Leu Ser Leu Leu Glu Lys Met Gln Thr Gln Glu Ile Leu
            20                  25                  30
Arg Ile Leu Arg Leu Pro Glu Leu Gly Asp Leu Gly Gln Phe Phe Arg
        35                  40                  45
Ser Leu Ser Ala Thr Thr Leu Val Ser Met Gly Ala Leu Ala Ala Ile
    50                  55                  60
Leu Ala Tyr Trp Phe Thr His Arg Pro Lys Ala Leu Gln Pro Pro Cys
65                  70                  75                  80
Asn Leu Leu Met Gln Ser Glu Glu Val Glu Asp Ser Gly Gly Ala Arg
                85                  90                  95
Arg Ser Val Ile Gly Ser Gly Pro Gln Leu Leu Thr His Tyr Tyr Asp
            100                 105                 110
Asp Ala Arg Thr Met Tyr Gln Val Phe Arg Arg Gly Leu Ser Ile Ser
        115                 120                 125
Gly Asn Gly Pro Cys Leu Gly Phe Arg Lys Pro Lys Gln Pro Tyr Gln
    130                 135                 140
Trp Leu Ser Tyr Gln Glu Val Ala Asp Arg Ala Glu Phe Leu Gly Ser
145                 150                 155                 160
Gly Leu Leu Gln His Asn Cys Lys Ala Cys Thr Asp Gln Phe Ile Gly
                165                 170                 175
Val Phe Ala Gln Asn Arg Pro Glu Trp Ile Ile Val Glu Leu Ala Cys
            180                 185                 190
Tyr Thr Tyr Ser Met Val Val Val Pro Leu Tyr Asp Thr Leu Gly Pro
        195                 200                 205
Gly Ala Ile Arg Tyr Ile Ile Asn Thr Ala Asp Ile Ser Thr Val Ile
    210                 215                 220
Val Asp Lys Pro Gln Lys Ala Val Leu Leu Leu Glu His Val Glu Arg
225                 230                 235                 240
Lys Glu Thr Pro Gly Leu Lys Leu Ile Ile Leu Met Asp Pro Phe Glu
                245                 250                 255
Glu Ala Leu Lys Glu Arg Gly Gln Lys Cys Gly Val Val Ile Lys Ser
            260                 265                 270
Met Gln Ala Val Glu Asp Cys Gly Gln Glu Asn His Gln Ala Pro Val
        275                 280                 285
Pro Pro Gln Pro Asp Asp Leu Ser Ile Val Cys Phe Thr Ser Gly Thr
    290                 295                 300
Thr Gly Asn Pro Lys Gly Ala Met Leu Thr His Gly Asn Val Val Ala
305                 310                 315                 320
Asp Phe Ser Gly Phe Leu Lys Val Thr Glu Ser Gln Trp Ala Pro Thr
                325                 330                 335
Cys Ala Asp Val His Ile Ser Tyr Leu Pro Leu Ala His Met Phe Glu
            340                 345                 350
Arg Met Val Gln Ser Val Val Tyr Cys His Gly Gly Arg Val Gly Phe
        355                 360                 365
Phe Gln Gly Asp Ile Arg Leu Leu Ser Asp Asp Met Lys Ala Leu Cys

370                 375                 380
Pro Thr Ile Phe Pro Val Val Pro Arg Leu Leu Asn Arg Met Tyr Asp
385                 390                 395                 400
Lys Ile Phe Ser Gln Ala Asn Thr Pro Leu Lys Arg Trp Leu Leu Glu
                405                 410                 415
Phe Ala Ala Lys Arg Lys Gln Ala Glu Val Arg Ser Gly Ile Ile Arg
            420                 425                 430
Asn Asp Ser Ile Trp Asp Glu Leu Phe Phe Asn Lys Ile Gln Ala Ser
        435                 440                 445
Leu Gly Gly Cys Val Arg Met Ile Val Thr Gly Ala Ala Pro Ala Ser
    450                 455                 460
Pro Thr Val Leu Gly Phe Leu Arg Ala Ala Leu Gly Cys Gln Val Tyr
465                 470                 475                 480
Glu Gly Tyr Gly Gln Thr Glu Cys Thr Ala Gly Cys Thr Phe Thr Thr
                485                 490                 495
Pro Gly Asp Trp Thr Ser Gly His Val Gly Ala Pro Leu Pro Cys Asn
            500                 505                 510
His Ile Lys Leu Val Asp Val Glu Glu Leu Asn Tyr Trp Ala Cys Lys
        515                 520                 525
Gly Glu Gly Glu Ile Cys Val Arg Gly Pro Asn Val Phe Lys Gly Tyr
    530                 535                 540
Leu Lys Asp Pro Asp Arg Thr Lys Glu Ala Leu Asp Ser Asp Gly Trp
545                 550                 555                 560
Leu His Thr Gly Asp Ile Gly Lys Trp Leu Pro Ala Gly Thr Leu Lys
                565                 570                 575
Ile Ile Asp Arg Lys Lys His Ile Phe Lys Leu Ala Gln Gly Glu Tyr
            580                 585                 590
Val Ala Pro Glu Lys Ile Glu Asn Ile Tyr Ile Arg Ser Gln Pro Val
        595                 600                 605
Ala Gln Ile Tyr Val His Gly Asp Ser Leu Lys Ala Phe Leu Val Gly
    610                 615                 620
Ile Val Val Pro Asp Pro Glu Val Met Pro Ser Trp Ala Gln Lys Arg
625                 630                 635                 640
Gly Ile Glu Gly Thr Tyr Ala Asp Leu Cys Thr Asn Lys Asp Leu Lys
                645                 650                 655
Lys Ala Ile Leu Glu Asp Met Val Arg Leu Gly Lys Glu Ser Gly Leu
            660                 665                 670
His Ser Phe Glu Gln Val Lys Ala Ile His Ile His Ser Asp Met Phe
        675                 680                 685
Ser Val Gln Asn Gly Leu Leu Thr Pro Thr Leu Lys Ala Lys Arg Pro
    690                 695                 700
Glu Leu Arg Glu Tyr Phe Lys Lys Gln Ile Glu Glu Leu Tyr Ser Ile
705                 710                 715                 720
Ser Met


<210>  14
<211>  2166
<212>  DNA
<213>  Homo sapiens

<400>  14
atgctgacct tcttcctcgt gtcggggggc tccctctggc tattcgtaga gtttgtcctc      60
tcacttctgg agaagatgca gacacaggag atcctgagga tactgcgact gcctgagcta     120
ggtgacttgg gacagttttt ccgcagcctc tcggccacca ccctcgtgag tatgggtgcc     180
ctggctgcca tccttgccta ctggttcact caccggccaa aggccttgca gccgccatgc     240
aacctcctga tgcagtcaga agaagtagag gacagtggcg gggcacggcg atctgtgatt     300
gggtctggcc ctcagctact tacccactac tatgatgatg cccggaccat gtaccaggtg     360
ttccgccgtg ggcttagcat ctcagggaat gggccctgtc ttggtttcag gaagcctaag     420
cagccttacc agtggctgtc ctaccaggag gtggccgaca gggctgaatt tctggggtcc     480
ggacttctcc agcacaattg taaagcatgc actgatcagt ttattggtgt ttttgcacaa     540
aatcggccag agtggatcat tgtggagctg gcctgctaca catattccat ggtggtggtc     600
ccgctctatg acaccctggg ccctggggct atccgctaca tcatcaatac agcggacatc     660
agcaccgtga ttgtggacaa acctcagaag gctgtgcttc tgctagagca tgtggagagg     720
aaggagactc caggcctcaa gctgatcatc ctcatggacc cattcgaaga agccctgaaa     780
gagagagggc agaagtgcgg ggtggtcatt aagtccatgc aggccgtgga ggactgtggc     840
caagagaatc accaggctcc tgtgcccccg cagcctgatg acctctccat tgtgtgtttc     900
acaagcggca cgacagggaa cccaaaaggt gcgatgctca cccatgggaa cgtggtggct     960
gatttctcag gctttctgaa agtgacagag agtcagtggg ctcccacttg tgcggatgtg    1020
cacatttcct atttgccttt agcacacatg tttgagcgaa tggtgcagtc tgtcgtctat    1080

tgccacggag ggcgtgttgg cttcttccag ggagatatcc gccttctctc agatgacatg 1140
aaggctctat gccccaccat cttccctgtg gtcccacgac tgctgaaccg gatgtacgac 1200
aagatcttca gccaggcaaa cacaccatta aagcgctggc tcctggagtt tgcagcaaag 1260
cgtaagcaag ccgaggtccg gagtggaatc atcaggaatg atagtatctg ggatgaactc 1320
ttctttaata agattcaggc cagtcttggt gggtgtgtgc ggatgattgt tactggagca 1380
gccccagcat caccaacagt tctgggattt ctccgggcag ctctagggtg ccaggtttat 1440
gaaggttatg gccaaactga gtgcacagct ggatgtacct tcaccactcc tggcgactgg 1500
acctcagggc acgtaggggc gccacttccc tgcaatcata tcaagctcgt tgatgttgag 1560
gaactgaact actgggcctg caaaggagag ggagagatat gtgtgagagg accaaatgtg 1620
ttcaaaggct acttgaaaga tccagacagg acgaaggagg ccctggacag cgatggctgg 1680
cttcacactg gagacatcgg aaaatggctg ccggcaggaa ctcttaaaat tattgatcgg 1740
aaaaagcata tatttaaact tgctcaggga gaatatgttg cacccgagaa gattgagaac 1800
atctacatcc ggagccaacc tgtggcgcaa atctatgtcc atggggacag cttaaaggcc 1860
tttttggtag gcattgttgt gcctgaccct gaagttatgc cctcctgggc ccagaagaga 1920
ggaattgaag gaacatatgc agatctctgc acaaataagg atctgaagaa agccattttg 1980
gaagatatgg tgaggttagg aaaagaaagt ggactccatt cttttgagca ggttaaagcc 2040
attcacatcc attctgacat gttctcagtt caaaatggct tgctgacacc aacactaaaa 2100
gctaagagac ctgagctgag agagtacttc aaaaaacaaa tagaagagct ttactcaatc 2160
tccatg 2166

<210> 15
<211> 722
<212> PRT
<213> Homo sapiens

<400> 15
Met Leu Thr Phe Phe Leu Val Ser Gly Gly Ser Leu Trp Leu Phe Val
1               5                   10                  15
Glu Phe Val Leu Ser Leu Leu Glu Lys Met Gln Thr Gln Glu Ile Leu
            20                  25                  30
Arg Ile Leu Arg Leu Pro Glu Leu Gly Asp Leu Gly Gln Phe Phe Arg
        35                  40                  45
Ser Leu Ser Ala Thr Thr Leu Val Ser Met Gly Ala Leu Ala Ala Ile
    50                  55                  60
Leu Ala Tyr Trp Phe Thr His Arg Pro Lys Ala Leu Gln Pro Pro Cys
65                  70                  75                  80
Asn Leu Leu Met Gln Ser Glu Glu Val Glu Asp Ser Gly Gly Ala Arg
                85                  90                  95
Arg Ser Val Ile Gly Ser Gly Pro Gln Leu Leu Thr His Tyr Tyr Asp
            100                 105                 110
Asp Ala Arg Thr Met Tyr Gln Val Phe Arg Arg Gly Leu Ser Ile Ser
        115                 120                 125
Gly Asn Gly Pro Cys Leu Gly Phe Arg Lys Pro Lys Gln Pro Tyr Gln
    130                 135                 140
Trp Leu Ser Tyr Gln Glu Val Ala Asp Arg Ala Glu Phe Leu Gly Ser
145                 150                 155                 160
Gly Leu Leu Gln His Asn Cys Lys Ala Cys Thr Asp Gln Phe Ile Gly
                165                 170                 175
Val Phe Ala Gln Asn Arg Pro Glu Trp Ile Ile Val Glu Leu Ala Cys
            180                 185                 190
Tyr Thr Tyr Ser Met Val Val Val Pro Leu Tyr Asp Thr Leu Gly Pro
        195                 200                 205
Gly Ala Ile Arg Tyr Ile Ile Asn Thr Ala Asp Ile Ser Thr Val Ile
    210                 215                 220
Val Asp Lys Pro Gln Lys Ala Val Leu Leu Leu Glu His Val Glu Arg
225                 230                 235                 240
Lys Glu Thr Pro Gly Leu Lys Leu Ile Ile Leu Met Asp Pro Phe Glu
                245                 250                 255
Glu Ala Leu Lys Glu Arg Gly Gln Lys Cys Gly Val Val Ile Lys Ser
            260                 265                 270
Met Gln Ala Val Glu Asp Cys Gly Gln Glu Asn His Gln Ala Pro Val
        275                 280                 285
Pro Pro Gln Pro Asp Asp Leu Ser Ile Val Cys Phe Thr Ser Gly Thr
    290                 295                 300
Thr Gly Asn Pro Lys Gly Ala Met Leu Thr His Gly Asn Val Val Ala
305                 310                 315                 320
Asp Phe Ser Gly Phe Leu Lys Val Thr Glu Lys Val Ile Phe Pro Arg
                325                 330                 335
Gln Asp Asp Val Leu Ile Ser Phe Leu Pro Leu Ala His Met Phe Glu

            340                 345                 350
Arg Val Ile Gln Ser Val Val Tyr Cys His Gly Gly Arg Val Gly Phe
        355                 360                 365
Phe Gln Gly Asp Ile Arg Leu Leu Ser Asp Asp Met Lys Ala Leu Cys
    370                 375                 380
Pro Thr Ile Phe Pro Val Val Pro Arg Leu Leu Asn Arg Met Tyr Asp
385                 390                 395                 400
Lys Ile Phe Ser Gln Ala Asn Thr Pro Leu Lys Arg Trp Leu Leu Glu
                405                 410                 415
Phe Ala Ala Lys Arg Lys Gln Ala Glu Val Arg Ser Gly Ile Ile Arg
            420                 425                 430
Asn Asp Ser Ile Trp Asp Glu Leu Phe Phe Asn Lys Ile Gln Ala Ser
        435                 440                 445
Leu Gly Gly Cys Val Arg Met Ile Val Thr Gly Ala Ala Pro Ala Ser
    450                 455                 460
Pro Thr Val Leu Gly Phe Leu Arg Ala Ala Leu Gly Cys Gln Val Tyr
465                 470                 475                 480
Glu Gly Tyr Gly Gln Thr Glu Cys Thr Ala Gly Cys Thr Phe Thr Thr
                485                 490                 495
Pro Gly Asp Trp Thr Ser Gly His Val Gly Ala Pro Leu Pro Cys Asn
            500                 505                 510
His Ile Lys Leu Val Asp Val Glu Glu Leu Asn Tyr Trp Ala Cys Lys
        515                 520                 525
Gly Glu Gly Glu Ile Cys Val Arg Gly Pro Asn Val Phe Lys Gly Tyr
    530                 535                 540
Leu Lys Asp Pro Asp Arg Thr Lys Glu Ala Leu Asp Ser Asp Gly Trp
545                 550                 555                 560
Leu His Thr Gly Asp Ile Gly Lys Trp Leu Pro Ala Gly Thr Leu Lys
                565                 570                 575
Ile Ile Asp Arg Lys Lys His Ile Phe Lys Leu Ala Gln Gly Glu Tyr
            580                 585                 590
Val Ala Pro Glu Lys Ile Glu Asn Ile Tyr Ile Arg Ser Gln Pro Val
        595                 600                 605
Ala Gln Ile Tyr Val His Gly Asp Ser Leu Lys Ala Phe Leu Val Gly
    610                 615                 620
Ile Val Val Pro Asp Pro Glu Val Met Pro Ser Trp Ala Gln Lys Arg
625                 630                 635                 640
Gly Ile Glu Gly Thr Tyr Ala Asp Leu Cys Thr Asn Lys Asp Leu Lys
                645                 650                 655
Lys Ala Ile Leu Glu Asp Met Val Arg Leu Gly Lys Glu Ser Gly Leu
            660                 665                 670
His Ser Phe Glu Gln Val Lys Ala Ile His Ile His Ser Asp Met Phe
        675                 680                 685
Ser Val Gln Asn Gly Leu Leu Thr Pro Thr Leu Lys Ala Lys Arg Pro
    690                 695                 700
Glu Leu Arg Glu Tyr Phe Lys Lys Gln Ile Glu Glu Leu Tyr Ser Ile
705                 710                 715                 720
Ser Met

<210> 16
<211> 2166
<212> DNA
<213> Homo sapiens

<400> 16
atgctgacct tcttcctcgt gtcggggggc tccctctggc tattcgtaga gtttgtcctc 60
tcacttctgg agaagatgca gacacaggag atcctgagga tactgcgact gcctgagcta 120
ggtgacttgg gacagttttt ccgcagcctc tcggccacca ccctcgtgag tatgggtgcc 180
ctggctgcca tccttgccta ctggttcact caccggccaa aggccttgca gccgccatgc 240
aacctcctga tgcagtcaga agaagtagag gacagtggcg gggcacggcg atctgtgatt 300
gggtctggcc ctcagctact tacccactac tatgatgatg cccggaccat gtaccaggtg 360
ttccgccgtg ggcttagcat ctcagggaat gggccctgtc ttggtttcag gaagcctaag 420
cagccttacc agtggctgtc ctaccaggag gtggccgaca gggctgaatt tctggggtcc 480
ggacttctcc agcacaattg taaagcatgc actgatcagt ttattggtgt ttttgcacaa 540
aatcggccag agtggatcat tgtggagctg gcctgctaca catattccat ggtggtggtc 600
ccgctctatg acaccctggg ccctggggct atccgctaca tcatcaatac agcggacatc 660
agcaccgtga ttgtggacaa acctcagaag gctgtgcttc tgctagagca tgtggagagg 720
aaggagactc caggcctcaa gctgatcatc ctcatggacc cattcgaaga agccctgaaa 780
gagagagggc agaagtgcgg ggtggtcatt aagtccatgc aggccgtgga ggactgtggc 840

caagagaatc accaggctcc tgtgcccccg cagcctgatg acctctccat tgtgtgtttc 900
acaagcggca cgacagggaa cccaaaaggt gcgatgctca cccatgggaa cgtggtggct 960
gatttctcag gctttctgaa agtgacagag aaagtgatct ttccgagaca ggacgatgtg 1020
ctcatctcct tcctgcctct ggctcacatg tttgagagag taatccagtc tgtcgtctat 1080
tgccacggag ggcgtgttgg cttcttccag ggagatatcc gccttctctc agatgacatg 1140
aaggctctat gccccaccat cttccctgtg gtcccacgac tgctgaaccg gatgtacgac 1200
aagatcttca gccaggcaaa cacaccatta aagcgctggc tcctggagtt tgcagcaaag 1260
cgtaagcaag ccgaggtccg gagtggaatc atcaggaatg atagtatctg ggatgaactc 1320
ttctttaata agattcaggc cagtcttggt gggtgtgtgc ggatgattgt tactggagca 1380
gccccagcat caccaacagt tctgggattt ctccgggcag ctctagggtg ccaggtttat 1440
gaaggttatg gccaaactga gtgcacagct ggatgtacct tcaccactcc tggcgactgg 1500
acctcagggc acgtaggggc gccacttccc tgcaatcata tcaagctcgt tgatgttgag 1560
gaactgaact actgggcctg caaaggagag ggagagatat gtgtgagagg accaaatgtg 1620
ttcaaaggct acttgaaaga tccagacagg acgaaggagg ccctggacag cgatggctgg 1680
cttcacactg gagacatcgg aaaatggctg ccggcaggaa ctcttaaaat tattgatcgg 1740
aaaaagcata tatttaaact tgctcaggga gaatatgttg cacccgagaa gattgagaac 1800
atctacatcc ggagccaacc tgtggcgcaa atctatgtcc atggggacag cttaaaggcc 1860
tttttggtag gcattgttgt gcctgaccct gaagttatgc cctcctgggc ccagaagaga 1920
ggaattgaag gaacatatgc agatctctgc acaaataagg atctgaagaa agccattttg 1980
gaagatatgg tgaggttagg aaaagaaagt ggactccatt cttttgagca ggttaaagcc 2040
attcacatcc attctgacat gttctcagtt caaaatggct tgctgacacc aacactaaaa 2100
gctaagagac ctgagctgag agagtacttc aaaaaacaaa tagaagagct ttactcaatc 2160
tccatg 2166

<210> 17
<211> 2511
<212> PRT
<213> Homo sapiens

<400> 17
Met Glu Glu Val Val Ile Ala Gly Met Ser Gly Lys Leu Pro Glu Ser
1               5                   10                  15
Glu Asn Leu Gln Glu Phe Trp Asp Asn Leu Ile Gly Gly Val Asp Met
            20                  25                  30
Val Thr Asp Asp Asp Arg Arg Trp Lys Ala Gly Leu Tyr Gly Leu Pro
        35                  40                  45
Arg Arg Ser Gly Lys Leu Lys Asp Leu Ser Arg Phe Asp Ala Ser Phe
    50                  55                  60
Phe Gly Val His Pro Lys Gln Ala His Thr Met Asp Pro Gln Leu Arg
65                  70                  75                  80
Leu Leu Leu Glu Val Thr Tyr Glu Ala Ile Val Asp Gly Gly Ile Asn
                85                  90                  95
Pro Asp Ser Leu Arg Gly Thr His Thr Gly Val Trp Val Gly Val Ser
            100                 105                 110
Gly Ser Glu Thr Ser Glu Ala Leu Ser Arg Asp Pro Glu Thr Leu Val
        115                 120                 125
Gly Tyr Ser Met Val Gly Cys Gln Arg Ala Met Met Ala Asn Arg Leu
    130                 135                 140
Ser Phe Phe Phe Asp Phe Arg Gly Pro Ser Ile Ala Leu Asp Thr Ala
145                 150                 155                 160
Cys Ser Ser Ser Leu Met Ala Leu Gln Asn Ala Tyr Gln Ala Ile His
                165                 170                 175
Ser Gly Gln Cys Pro Ala Ala Ile Val Gly Gly Ile Asn Val Leu Leu
            180                 185                 190
Lys Pro Asn Thr Ser Val Gln Phe Leu Arg Leu Gly Met Leu Ser Pro
        195                 200                 205
Glu Gly Thr Cys Lys Ala Phe Asp Thr Ala Gly Asn Gly Tyr Cys Arg
    210                 215                 220
Ser Glu Gly Val Val Ala Val Leu Leu Thr Lys Lys Ser Leu Ala Arg
225                 230                 235                 240
Arg Val Tyr Ala Thr Ile Leu Asn Ala Gly Thr Asn Thr Asp Gly Phe
                245                 250                 255
Lys Glu Gln Gly Val Thr Phe Pro Ser Gly Asp Ile Gln Glu Gln Leu
            260                 265                 270
Ile Arg Ser Leu Tyr Gln Ser Ala Gly Val Ala Pro Glu Ser Phe Glu
        275                 280                 285
Tyr Ile Glu Ala His Gly Thr Gly Thr Lys Val Gly Asp Pro Gln Glu
    290                 295                 300
Leu Asn Gly Ile Thr Arg Ala Leu Cys Ala Thr Arg Gln Glu Pro Leu

305                 310                 315                 320
Leu Ile Gly Ser Thr Lys Ser Asn Met Gly His Pro Glu Pro Ala Ser
                325                 330                 335
Gly Leu Ala Ala Leu Ala Lys Val Leu Leu Ser Leu Glu His Gly Leu
            340                 345                 350
Trp Ala Pro Asn Leu His Phe His Ser Pro Asn Pro Glu Ile Pro Ala
        355                 360                 365
Leu Leu Asp Gly Arg Leu Gln Val Val Asp Gln Pro Leu Pro Val Arg
    370                 375                 380
Gly Gly Asn Val Gly Ile Asn Ser Phe Gly Phe Gly Gly Ser Asn Val
385                 390                 395                 400
His Ile Ile Leu Arg Pro Asn Thr Gln Pro Pro Pro Ala Pro Ala Pro
                405                 410                 415
His Ala Thr Leu Pro Arg Leu Leu Arg Ala Ser Gly Arg Thr Pro Glu
            420                 425                 430
Ala Val Gln Lys Leu Leu Glu Gln Gly Leu Arg His Ser Gln Asp Leu
        435                 440                 445
Ala Phe Leu Ser Met Leu Asn Asp Ile Ala Ala Val Pro Ala Thr Ala
    450                 455                 460
Met Pro Phe Arg Gly Tyr Ala Val Leu Gly Gly Glu Arg Gly Gly Pro
465                 470                 475                 480
Glu Val Gln Gln Val Pro Ala Gly Glu Arg Pro Leu Trp Phe Ile Cys
                485                 490                 495
Ser Gly Met Gly Thr Gln Trp Arg Gly Met Gly Leu Ser Leu Met Arg
            500                 505                 510
Leu Asp Arg Phe Arg Asp Ser Ile Leu Arg Ser Asp Glu Ala Val Lys
        515                 520                 525
Pro Phe Gly Leu Lys Val Ser Gln Leu Leu Leu Ser Thr Asp Glu Ser
    530                 535                 540
Thr Phe Asp Asp Ile Val His Ser Phe Val Ser Leu Thr Ala Ile Gln
545                 550                 555                 560
Ile Gly Leu Ile Asp Leu Leu Ser Cys Met Gly Leu Arg Pro Asp Gly
                565                 570                 575
Ile Val Gly His Ser Leu Gly Glu Val Ala Cys Gly Tyr Ala Asp Gly
            580                 585                 590
Cys Leu Ser Gln Glu Glu Ala Val Leu Ala Ala Tyr Trp Arg Gly Gln
        595                 600                 605
Cys Ile Lys Glu Ala His Leu Pro Pro Gly Ala Met Ala Ala Val Gly
    610                 615                 620
Leu Ser Trp Glu Glu Cys Lys Gln Arg Cys Pro Pro Gly Val Val Pro
625                 630                 635                 640
Ala Cys His Asn Ser Lys Asp Thr Val Thr Ile Ser Gly Pro Gln Ala
                645                 650                 655
Pro Val Phe Glu Phe Val Glu Gln Leu Arg Lys Glu Gly Val Phe Ala
            660                 665                 670
Lys Glu Val Arg Thr Gly Gly Met Ala Phe His Ser Tyr Phe Met Glu
        675                 680                 685
Ala Ile Ala Pro Pro Leu Leu Gln Glu Leu Lys Lys Val Ile Arg Glu
    690                 695                 700
Pro Lys Pro Arg Ser Ala Arg Trp Leu Ser Thr Ser Ile Pro Glu Ala
705                 710                 715                 720
Gln Trp His Ser Ser Leu Ala Arg Thr Ser Ser Ala Glu Tyr Asn Val
                725                 730                 735
Asn Asn Leu Val Ser Pro Val Leu Phe Gln Glu Ala Leu Trp His Val
            740                 745                 750
Pro Glu His Ala Val Val Leu Glu Ile Ala Pro His Ala Leu Leu Gln
        755                 760                 765
Ala Val Leu Lys Arg Gly Leu Lys Pro Ser Cys Thr Ile Ile Pro Leu
    770                 775                 780
Met Lys Lys Asp His Arg Asp Asn Leu Glu Phe Phe Leu Ala Gly Ile
785                 790                 795                 800
Gly Arg Leu His Leu Ser Gly Ile Asp Ala Asn Pro Asn Ala Leu Phe
                805                 810                 815
Pro Pro Val Glu Phe Pro Ala Pro Arg Gly Thr Pro Leu Ile Ser Pro
            820                 825                 830
Leu Ile Lys Trp Asp His Ser Leu Ala Trp Asp Val Pro Ala Ala Glu
        835                 840                 845
Asp Phe Pro Asn Gly Ser Gly Ser Pro Ser Ala Ala Ile Tyr Asn Ile
    850                 855                 860
Asp Thr Ser Ser Glu Ser Pro Asp His Tyr Leu Val Asp His Thr Leu

865                 870                 875                 880
Asp Gly Arg Val Leu Phe Pro Ala Thr Gly Tyr Leu Ser Ile Val Trp
                885                 890                 895
Lys Thr Leu Ala Arg Ala Leu Gly Leu Gly Val Glu Gln Leu Pro Val
            900                 905                 910
Val Phe Glu Asp Val Val Leu His Gln Ala Thr Ile Leu Pro Lys Thr
        915                 920                 925
Gly Thr Val Ser Leu Glu Val Arg Leu Leu Glu Ala Ser Arg Ala Phe
    930                 935                 940
Glu Val Ser Glu Asn Gly Asn Leu Val Val Ser Gly Lys Val Tyr Gln
945                 950                 955                 960
Trp Asp Asp Pro Asp Pro Arg Leu Phe Asp His Pro Glu Ser Pro Thr
                965                 970                 975
Pro Asn Pro Thr Glu Pro Leu Phe Leu Ala Gln Ala Glu Val Tyr Lys
            980                 985                 990
Glu Leu Arg Leu Arg Gly Tyr Asp Tyr Gly Pro His Phe Gln Gly Ile
        995                 1000                1005
Leu Glu Ala Ser Leu Glu Gly Asp Ser Gly Arg Leu Leu Trp Lys Asp
    1010                1015                1020
Asn Trp Val Ser Phe Met Asp Thr Met Leu Gln Met Ser Ile Leu Gly
1025                1030                1035                1040
Ser Ala Lys His Gly Leu Tyr Leu Pro Thr Arg Val Thr Ala Ile His
                1045                1050                1055
Ile Asp Pro Ala Thr His Arg Gln Lys Leu Tyr Thr Leu Gln Asp Lys
            1060                1065                1070
Ala Gln Val Ala Asp Val Val Val Ser Arg Trp Leu Arg Val Thr Val
        1075                1080                1085
Ala Gly Gly Val His Ile Ser Gly Leu His Thr Glu Ser Ala Pro Arg
    1090                1095                1100
Arg Gln Gln Glu Gln Gln Val Pro Ile Leu Glu Lys Phe Cys Phe Thr
1105                1110                1115                1120
Pro His Thr Glu Glu Gly Cys Leu Ser Glu Arg Ala Ala Leu Gln Glu
                1125                1130                1135
Glu Leu Gln Leu Cys Lys Gly Leu Val Gln Ala Leu Gln Thr Lys Val
            1140                1145                1150
Thr Gln Gln Gly Leu Lys Met Val Val Pro Gly Leu Asp Gly Ala Gln
        1155                1160                1165
Ile Pro Arg Asp Pro Ser Gln Gln Glu Leu Pro Arg Leu Leu Ser Ala
    1170                1175                1180
Ala Cys Arg Leu Gln Leu Asn Gly Asn Leu Gln Leu Glu Leu Ala Gln
1185                1190                1195                1200
Val Leu Ala Gln Glu Arg Pro Lys Leu Pro Glu Asp Pro Leu Leu Ser
                1205                1210                1215
Gly Leu Leu Asp Ser Pro Ala Leu Lys Ala Cys Leu Asp Thr Ala Val
            1220                1225                1230
Glu Asn Met Pro Ser Leu Lys Met Lys Val Val Glu Val Leu Ala Gly
        1235                1240                1245
His Gly His Leu Tyr Ser Arg Ile Pro Gly Leu Leu Ser Pro His Pro
    1250                1255                1260
Leu Leu Gln Leu Ser Tyr Thr Ala Thr Asp Arg His Pro Gln Ala Leu
1265                1270                1275                1280
Glu Ala Ala Gln Ala Glu Leu Gln Gln His Asp Val Ala Gln Gly Gln
                1285                1290                1295
Trp Asp Pro Ala Asp Pro Ala Pro Ser Ala Leu Gly Ser Ala Asp Leu
            1300                1305                1310
Leu Val Cys Asn Cys Ala Val Ala Ala Leu Gly Asp Pro Ala Ser Ala
        1315                1320                1325
Leu Ser Asn Met Val Ala Ala Leu Arg Glu Gly Gly Phe Leu Leu Leu
    1330                1335                1340
His Thr Leu Leu Arg Gly His Pro Leu Gly Asp Ile Val Ala Phe Leu
1345                1350                1355                1360
Thr Ser Thr Glu Pro Gln Tyr Gly Gln Gly Ile Leu Ser Gln Asp Ala
                1365                1370                1375
Trp Glu Ser Leu Phe Ser Arg Val Ser Leu Arg Leu Val Gly Leu Lys
            1380                1385                1390
Lys Ser Phe Tyr Gly Ser Thr Leu Phe Leu Cys Arg Arg Pro Thr Pro
        1395                1400                1405
Gln Asp Ser Pro Ile Phe Leu Pro Val Asp Asp Thr Ser Phe Arg Trp
    1410                1415                1420
Val Glu Ser Leu Lys Gly Ile Leu Ala Asp Glu Asp Ser Ser Arg Pro

1425                1430                1435                1440
Val Trp Leu Lys Ala Ile Asn Cys Ala Thr Ser Gly Val Val Gly Leu
                1445                1450                1455
Val Asn Cys Leu Arg Arg Glu Pro Gly Gly Asn Arg Leu Arg Cys Val
            1460                1465                1470
Leu Leu Ser Asn Leu Ser Ser Thr Ser His Val Pro Glu Val Asp Pro
        1475                1480                1485
Gly Ser Ala Glu Leu Gln Lys Val Leu Gln Gly Asp Leu Val Met Asn
    1490                1495                1500
Val Tyr Arg Asp Gly Ala Trp Gly Ala Phe Arg His Phe Leu Leu Glu
1505                1510                1515                1520
Glu Asp Lys Pro Glu Glu Pro Thr Ala His Ala Phe Val Ser Thr Leu
                1525                1530                1535
Thr Arg Gly Asp Leu Ser Ser Ile Arg Trp Val Cys Ser Ser Leu Arg
            1540                1545                1550
His Ala Gln Pro Thr Cys Pro Gly Ala Gln Leu Cys Thr Val Tyr Tyr
        1555                1560                1565
Ala Ser Leu Asn Phe Arg Asp Ile Met Leu Ala Thr Gly Lys Leu Ser
    1570                1575                1580
Pro Asp Ala Ile Pro Gly Lys Trp Thr Ser Gln Asp Ser Leu Leu Gly
1585                1590                1595                1600
Met Glu Phe Ser Gly Arg Asp Ala Ser Gly Lys Arg Val Met Gly Leu
                1605                1610                1615
Val Pro Ala Lys Gly Leu Ala Thr Ser Val Leu Leu Ser Pro Asp Phe
            1620                1625                1630
Leu Trp Asp Val Pro Ser Asn Trp Thr Leu Glu Glu Ala Ala Ser Val
        1635                1640                1645
Pro Val Val Tyr Ser Thr Ala Tyr Tyr Ala Leu Val Val Arg Gly Arg
    1650                1655                1660
Val Arg Pro Gly Glu Thr Leu Leu Ile His Ser Gly Ser Gly Gly Val
1665                1670                1675                1680
Gly Gln Ala Ala Ile Ala Ile Ala Leu Ser Leu Gly Cys Arg Val Phe
                1685                1690                1695
Thr Thr Val Gly Ser Ala Glu Lys Arg Ala Tyr Leu Gln Ala Arg Phe
            1700                1705                1710
Pro Gln Leu Asp Ser Thr Ser Phe Ala Asn Ser Arg Asp Thr Ser Phe
        1715                1720                1725
Glu Gln His Val Leu Trp His Thr Gly Gly Lys Gly Val Asp Leu Val
    1730                1735                1740
Leu Asn Ser Leu Ala Glu Glu Lys Leu Gln Ala Ser Val Arg Cys Leu
1745                1750                1755                1760
Ala Thr His Gly Arg Phe Leu Glu Ile Gly Lys Phe Asp Leu Ser Gln
                1765                1770                1775
Asn His Pro Leu Gly Met Ala Ile Phe Leu Lys Asn Val Thr Phe His
            1780                1785                1790
Gly Val Leu Leu Asp Ala Phe Phe Asn Glu Ser Ser Ala Asp Trp Arg
        1795                1800                1805
Glu Val Trp Ala Leu Val Gln Ala Gly Ile Arg Asp Gly Val Val Arg
    1810                1815                1820
Pro Leu Lys Cys Thr Val Phe His Gly Ala Gln Val Glu Asp Ala Phe
1825                1830                1835                1840
Arg Tyr Met Ala Gln Gly Lys His Ile Gly Lys Val Val Val Gln Val
                1845                1850                1855
Leu Ala Glu Glu Pro Glu Ala Val Leu Lys Gly Ala Lys Pro Lys Leu
            1860                1865                1870
Met Ser Ala Ile Ser Lys Thr Phe Cys Pro Ala His Lys Ser Tyr Ile
        1875                1880                1885
Ile Ala Gly Gly Leu Gly Gly Phe Gly Leu Glu Leu Ala Gln Trp Leu
    1890                1895                1900
Ile Gln Arg Gly Val Gln Lys Leu Val Leu Thr Ser Arg Ser Gly Ile
1905                1910                1915                1920
Arg Thr Gly Tyr Gln Ala Lys Gln Val Arg Arg Trp Arg Arg Gln Gly
                1925                1930                1935
Val Gln Val Gln Val Ser Thr Ser Asn Ile Ser Ser Leu Glu Gly Ala
            1940                1945                1950
Arg Gly Leu Ile Ala Glu Ala Ala Gln Leu Gly Pro Val Gly Gly Val
        1955                1960                1965
Phe Asn Leu Ala Val Val Leu Arg Asp Gly Leu Leu Glu Asn Gln Thr
    1970                1975                1980
Pro Glu Phe Phe Gln Asp Val Cys Lys Pro Lys Tyr Ser Gly Thr Leu

1985                1990                1995                2000
Asn Leu Asp Arg Val Thr Arg Glu Ala Cys Pro Glu Leu Asp Tyr Phe
                2005                2010                2015
Val Val Phe Ser Ser Val Ser Cys Gly Arg Gly Asn Ala Gly Gln Ser
            2020                2025                2030
Asn Tyr Gly Phe Ala Asn Ser Ala Met Glu Arg Ile Cys Glu Lys Arg
        2035                2040                2045
Arg His Glu Gly Leu Pro Gly Leu Ala Val Gln Trp Gly Ala Ile Gly
    2050                2055                2060
Asp Val Gly Ile Leu Val Glu Thr Met Ser Thr Asn Asp Thr Ile Val
2065                2070                2075                2080
Ser Gly Thr Leu Pro Gln Arg Met Ala Ser Cys Leu Glu Val Leu Asp
                2085                2090                2095
Leu Phe Leu Asn Gln Pro His Met Val Leu Ser Ser Phe Val Leu Ala
            2100                2105                2110
Glu Lys Ala Ala Ala Tyr Arg Asp Arg Asp Ser Gln Arg Asp Leu Val
        2115                2120                2125
Glu Ala Val Ala His Ile Leu Gly Ile Arg Asp Leu Ala Ala Val Asn
    2130                2135                2140
Leu Asp Ser Ser Leu Ala Asp Leu Gly Leu Asp Ser Leu Met Ser Val
2145                2150                2155                2160
Glu Val Arg Gln Thr Leu Glu Arg Glu Leu Asn Leu Val Leu Ser Val
                2165                2170                2175
Arg Glu Val Arg Gln Leu Thr Leu Arg Lys Leu Gln Glu Leu Ser Ser
            2180                2185                2190
Lys Ala Asp Glu Ala Ser Glu Leu Ala Cys Pro Thr Pro Lys Glu Asp
        2195                2200                2205
Gly Leu Ala Gln Gln Gln Thr Gln Leu Asn Leu Arg Ser Leu Leu Val
    2210                2215                2220
Asn Pro Glu Gly Pro Thr Leu Met Arg Leu Asn Ser Val Gln Ser Ser
2225                2230                2235                2240
Glu Arg Pro Leu Phe Leu Val His Pro Ile Glu Gly Ser Thr Thr Val
                2245                2250                2255
Phe His Ser Leu Ala Ser Arg Leu Ser Ile Pro Thr Tyr Gly Leu Gln
            2260                2265                2270
Cys Thr Arg Ala Ala Pro Leu Asp Ser Ile His Ser Leu Ala Ala Tyr
        2275                2280                2285
Tyr Ile Asp Cys Ile Arg Gln Val Gln Pro Glu Gly Pro Tyr Arg Val
    2290                2295                2300
Ala Gly Tyr Ser Tyr Gly Ala Cys Val Ala Phe Glu Met Cys Ser Gln
2305                2310                2315                2320
Leu Gln Ala Gln Gln Ser Pro Ala Pro Thr His Asn Ser Leu Phe Leu
                2325                2330                2335
Phe Asp Gly Ser Pro Thr Tyr Val Leu Ala Tyr Thr Gln Ser Tyr Arg
            2340                2345                2350
Ala Lys Leu Thr Pro Gly Cys Glu Ala Glu Ala Glu Thr Glu Ala Ile
        2355                2360                2365
Cys Phe Phe Val Gln Gln Phe Thr Asp Met Glu His Asn Arg Val Leu
    2370                2375                2380
Glu Ala Leu Leu Pro Leu Lys Gly Leu Glu Glu Arg Val Ala Ala Ala
2385                2390                2395                2400
Val Asp Leu Ile Ile Lys Ser His Gln Gly Leu Asp Arg Gln Glu Leu
                2405                2410                2415
Ser Phe Ala Ala Arg Ser Phe Tyr Tyr Lys Leu Arg Ala Ala Glu Gln
            2420                2425                2430
Tyr Thr Pro Lys Ala Lys Tyr His Gly Asn Val Met Leu Leu Arg Ala
        2435                2440                2445
Lys Thr Gly Gly Ala Tyr Gly Glu Asp Leu Gly Ala Asp Tyr Asn Leu
    2450                2455                2460
Ser Gln Val Cys Asp Gly Lys Val Ser Val His Val Ile Glu Gly Asp
2465                2470                2475                2480
His Arg Thr Leu Leu Glu Gly Ser Gly Leu Glu Ser Ile Ile Ser Ile
                2485                2490                2495
Ile His Ser Ser Leu Ala Glu Pro Arg Val Ser Val Arg Glu Gly
            2500                2505                2510

<210>  18
<211>  7533
<212>  DNA

<213>  Homo sapiens

<400>  18
atggaggagg tggtgattgc cggcatgtcc gggaagctgc cagagtcgga gaacttgcag      60
gagttctggg acaacctcat cggcggtgtg gacatggtca cggacgatga ccgtcgctgg     120
aaggcggggc tctacggcct gccccggcgg tccggcaagc tgaaggacct gtctaggttt     180
gatgcctcct tcttcggagt ccaccccaag caggcacaca cgatggaccc tcagctgcgg     240
ctgctgctgg aagtcaccta tgaagccatc gtggacggag gcatcaaccc agattcactc     300
cgaggaacac acactggcgt ctgggtgggc gtgagcggct ctgagacctc ggaggccctg     360
agccgagacc ccgagacact cgtgggctac agcatggtgg gctgccagcg agcgatgatg     420
gccaaccggc tctccttctt cttcgacttc agagggccca gcatcgcact ggacacagcc     480
tgctcctcca gcctgatggc cctgcagaac gcctaccagg ccatccacag cgggcagtgc     540
cctgccgcca tcgtgggggg catcaatgtc ctgctgaagc ccaacacctc cgtgcagttc     600
ttgaggctgg ggatgctcag ccccgagggc acctgcaagg ccttcgacac agcggggaat     660
gggtactgcc gctcggaggg tgtggtggcc gtcctgctga ccaagaagtc cctggcccgg     720
cgggtgtacg ccaccatcct gaacgccggc accaatacag atggcttcaa ggagcaaggc     780
gtgaccttcc cctcagggga tatccaggag cagctcatcc gctcgttgta ccagtcggcc     840
ggagtggccc ctgagtcatt tgaatacatc gaagcccacg gcacaggcac caaggtgggc     900
gacccccagg agctgaatgg catcacccga gccctgtgcg ccacccgcca ggagccgctg     960
ctcatcggct ccaccaagtc caacatgggg cacccggagc cagcctcggg gctggcagcc    1020
ctggccaagg tgctgctgtc cctggagcac gggctctggg cccccaacct gcacttccat    1080
agccccaacc ctgagatccc agcgctgttg gatgggcggc tgcaggtggt ggaccagccc    1140
ctgcccgtcc gtggcggcaa cgtgggcatc aactcctttg gcttcggggg ctccaacgtg    1200
cacatcatcc tgaggcccaa cacgcagccg ccccccgcac ccgccccaca tgccaccctg    1260
ccccgtctgc tgcgggccag cggacgcacc cctgaggccg tgcagaagct gctggagcag    1320
ggcctccggc acagccagga cctggctttc ctgagcatgc tgaacgacat cgcggctgtc    1380
cccgccaccg ccatgccctt ccgtggctac gctgtgctgg gtggtgagcg cggtggccca    1440
gaggtgcagc aggtgcccgc tggcgagcgc ccgctctggt tcatctgctc tgggatgggc    1500
acacagtggc gcgggatggg gctgagcctc atgcgcctgg accgcttccg agattccatc    1560
ctacgctccg atgaggctgt gaagccattc ggcctgaagg tgtcacagct gctgctgagc    1620
acagacgaga gcacctttga tgacatcgtc cattcgtttg tgagcctgac tgccatccag    1680
ataggcctca tagacctgct gagctgcatg gggctgaggc cagatggcat cgtcggccac    1740
tccctggggg aggtggcctg tggctacgcc gacggctgcc tgtcccagga ggaggccgtc    1800
ctcgctgcct actggagggg acagtgcatc aaagaagccc atctcccgcc gggcgccatg    1860
gcagccgtgg gcttgtcctg ggaggagtgt aaacagcgct gccccccggg cgtggtgccc    1920
gcctgccaca actccaagga cacagtcacc atctcgggac ctcaggcccc ggtgtttgag    1980
ttcgtggagc agctgaggaa ggagggtgtg tttgccaagg aggtgcggac cggcggtatg    2040
gccttccact cctacttcat ggaggccatc gcacccccac tgctgcagga gctcaagaag    2100
gtgatccggg agccgaagcc acgttcagcc cgctggctca gcacctctat ccccgaggcc    2160
cagtggcaca gcagcctggc acgcacgtcc tccgccgagt acaatgtcaa caacctggtg    2220
agccctgtgc tgttccagga ggccctgtgg cacgtgcctg agcacgcggt ggtgctggag    2280
atcgcgcccc acgccctgct gcaggctgtc ctgaagcgtg gcctgaagcc gagctgcacc    2340
atcatccccc tgatgaagaa ggatcacagg gacaacctgg agttcttcct ggccggcatc    2400
ggcaggctgc acctctcagg catcgacgcc aaccccaatg ccttgttccc acctgtggag    2460
ttcccagctc cccgaggaac tcccctcatc tccccactca tcaagtggga ccacagcctg    2520
gcctgggacg tgccggccgc cgaggacttc cccaacggtt caggttcccc ctcagccgcc    2580
atctacaaca tcgacaccag ctccgagtct cctgaccact acctggtgga ccacaccctc    2640
gacggtcgcg tcctcttccc cgccactggc tacctgagca tagtgtggaa gacgctggcc    2700
cgcgccctgg gcctgggcgt cgagcagctg cctgtggtgt ttgaggatgt ggtgctgcac    2760
caggccacca tcctgcccaa gactgggaca gtgtccctgg aggtacggct cctggaggcc    2820
tcccgtgcct tcgaggtgtc agagaacggc aacctggtag tgagtgggaa ggtgtaccag    2880
tgggatgacc ctgaccccag gctcttcgac cacccggaaa gccccacccc caaccccacg    2940
gagcccctct tcctggccca ggctgaagtt tacaaggagc tgcgtctgcg tggctacgac    3000
tacggccctc atttccaggg catcctggag gccagcctgg aaggtgactc ggggaggctg    3060
ctgtggaagg ataactgggt gagcttcatg gacaccatgc tgcagatgtc catcctgggc    3120
tcggccaagc acggcctgta cctgcccacc cgtgtcaccg ccatccacat cgaccctgcc    3180
acccacaggc agaagctgta cacactgcag gacaaggccc aagtggctga cgtggtggtg    3240
agcaggtggc tgagggtcac agtggccgga ggcgtccaca tctccgggct ccacactgag    3300
tcggccccgc ggcggcagca ggagcagcag gtgcccatcc tggagaagtt ttgcttcact    3360
ccccacacgg aggaggggtg cctgtctgag cgcgctgccc tgcaggagga gctgcaactg    3420
tgcaaggggc tggtgcaggc actgcagacc aaggtgaccc agcaggggct gaagatggtg    3480
gtgcccggac tggatggggc ccagatcccc cgggacccct cacagcagga actgccccgg    3540
ctgttgtcgg ctgcctgcag gcttcagctc aacgggaacc tgcagctgga gctggcgcag    3600
gtgctggccc aggagaggcc caagctgcca gaggaccctc tgctcagcgg cctcctggac    3660
tccccggcac tcaaggcctg cctggacact gccgtggaga acatgcccag cctgaagatg    3720
aaggtggtgg aggtgctggc tggccacggt cacctgtatt cccgcatccc aggcctgctc    3780
agcccccatc ccctgctgca gctgagctac acggccaccg accgccaccc ccaggccctg    3840
gaggctgccc aggccgagct gcagcagcac gacgttgccc agggccagtg ggatcccgca    3900
gaccctgccc ccagcgccct gggcagcgcc gacctcctgg tgtgcaactg tgctgtggct    3960
gccctcgggg acccggcctc agctctcagc aacatggtgg ctgccctgag agaagggggc     4020

tttctgctcc tgcacacact gctccggggg cacccccctcg gggacatcgt ggccttcctc 4080
acctccactg agccgcagta tggccagggc atcctgagcc aggacgcgtg ggagagcctc 4140
ttctccaggg tgtcgctgcg cctggtgggc ctgaagaagt ccttctacgg ctccacgctc 4200
ttcctgtgcc gccggcccac cccgcaggac agccccatct tcctgccggt ggacgatacc 4260
agcttccgct gggtggagtc tctgaagggc atcctggctg acgaagactc ttcccggcct 4320
gtgtggctga aggccatcaa ctgtgccacc tcgggcgtgg tgggcttggt gaactgtctc 4380
cgccgagagc ccggcgggaa ccgcctccgg tgtgtgctgc tctccaacct cagcagcacc 4440
tcccacgtcc cggaggtgga cccgggctcc gcagaactgc agaaggtgtt gcagggagac 4500
ctggtgatga acgtctaccg cgacggggcc tggggggctt tccgccactt cctgctggag 4560
gaggacaagc ctgaggagcc gacggcacat gcctttgtga gcaccctcac ccggggggac 4620
ctgtcctcca tccgctgggt ctgctcctcg ctgcgccatg cccagcccac ctgccctggc 4680
gcccagctct gcacggtcta ctacgcctcc ctcaacttcc gcgacatcat gctggccact 4740
ggcaagctgt cccctgatgc catcccaggg aagtggacct cccaggacag cctgctaggt 4800
atggagttct cgggccgaga cgccagcggc aagcgtgtga tgggactggt gcctgccaag 4860
ggcctggcca cctctgtcct gctgtcaccg gacttcctct gggatgtgcc ttccaactgg 4920
acgctggagg aggcggcctc ggtgcctgtc gtctacagca cggcctacta cgcgctggtg 4980
gtgcgtgggc gggtgcgccc cggggagacg ctgctcatcc actcgggctc gggcggcgtg 5040
ggccaggccg ccatcgccat cgccctcagt ctgggctgcc gcgtcttcac caccgtgggg 5100
tcggctgaga agcgggcgta cctccaggcc aggttccccc agctcgacag caccagcttc 5160
gccaactccc gggacacatc cttcgagcag catgtgctgt ggcacacggg cgggaagggc 5220
gttgacctgg tcttgaactc cttggcggaa gagaagctgc aggccagcgt gaggtgcttg 5280
gctacgcacg gtcgcttcct ggaaattggc aaattcgacc tttctcagaa ccacccgctc 5340
ggcatggcta tcttcctgaa gaacgtgaca ttccacgggg tcctactgga tgcgttcttc 5400
aacgagagca gtgctgactg gcgggaggtg tgggcgcttg tgcaggccgg catccgggat 5460
ggggtggtac ggcccctcaa gtgcacggtg ttccatgggg cccaggtgga ggacgccttc 5520
cgctacatgg cccaagggaa gcacattggc aaagtcgtcg tgcaggtgct tgcggaggag 5580
ccggaggcag tgctgaaggg ggccaaaccc aagctgatgt cggccatctc caagaccttc 5640
tgcccggccc acaagagcta catcatcgct ggtggtctgg gtggcttcgg cctggagttg 5700
gcgcagtggc tgatacagcg tggggtgcag aagctcgtgt tgacttctcg ctccgggatc 5760
cggacaggct accaggccaa gcaggtccgc cggtggaggc gccagggcgt acaggtgcag 5820
gtgtccacca gcaacatcag ctcactggag ggggcccggg gcctcattgc cgaggcggcg 5880
cagcttgggc ccgtgggcgg cgtcttcaac ctggccgtgg tcttgagaga tggcttgctg 5940
gagaaccaga ccccagagtt cttccaggac gtctgcaagc ccaagtacag cggcaccctg 6000
aacctggaca gggtgacccg agaggcgtgc cctgagctgg actactttgt ggtcttctcc 6060
tctgtgagct gcgggcgtgg caatgcggga cagagcaact acggctttgc caattccgcc 6120
atggagcgta tctgtgagaa acgccggcac gaaggcctcc caggcctggc cgtgcagtgg 6180
ggcgccatcg gcgacgtggg cattttggtg gagacgatga gcaccaacga cacgatcgtc 6240
agtggcacgc tgccccagcg catggcgtcc tgcctggagg tgctggacct cttcctgaac 6300
cagccccaca tggtcctgag cagctttgtg ctggctgaga aggctgcggc ctatagggac 6360
agggacagcc agcgggacct ggtggaggcc gtggcacaca tcctgggcat ccgcgacttg 6420
gctgctgtca acctggacag ctcactggcg gacctgggcc tggactcgct catgagcgtg 6480
gaggtgcgcc agacgctgga gcgtgagctc aacctggtgc tgtccgtgcg cgaggtgcgg 6540
caactcacgc tccggaaact gcaggagctg tcctcaaagg cggatgaggc cagcgagctg 6600
gcatgcccca cgcccaagga ggatggtctg gcccagcagc agactcagct gaacctgcgc 6660
tccctgctgg tgaacccgga gggccccacc ctgatgcggc tcaactccgt gcagagctcg 6720
gagcggcccc tgttcctggt gcacccaatc gagggctcca ccaccgtgtt ccacagcctg 6780
gcctcccggc tcagcatccc cacctatggc ctgcagtgca cccgagctgc gccccttgac 6840
agcatccaca gcctggctgc ctactacatc gactgcatca ggcaggtgca gcccgagggc 6900
ccctaccgcg tggccggcta ctcctacggg gcctgcgtgg cctttgaaat gtgctcccag 6960
ctgcaggccc agcagagccc agcccccacc cacaacagcc tcttcctgtt cgacggctcg 7020
cccacctacg tactggccta cacccagagc taccgggcaa agctgacccc aggctgtgag 7080
gctgaggctg agacggaggc catatgcttc ttcgtgcagc agttcacgga catggagcac 7140
aacagggtgc tggaggcgct gctgccgctg aagggcctag aggagcgtgt ggcagccgcc 7200
gtggacctga tcatcaagag ccaccagggc ctggaccgcc aggagctgag ctttgcggcc 7260
cggtccttct actacaagct gcgtgccgct gagcagtaca cacccaaggc caagtaccat 7320
ggcaacgtga tgctactgcg cgccaagacg ggtggcgcct acggcgagga cctgggcgcg 7380
gactacaacc tctcccaggt atgcgacggg aaagtatccg tccacgtcat cgagggtgac 7440
caccgcacgc tgctggaggg cagcggcctg gagtccatca tcagcatcat ccacagctcc 7500
ctggctgagc cacgcgtgag cgtgcgggag ggc 7533

<210> 19
<211> 41
<212> DNA
<213> Artificial Sequence

<220>
<223> Primer

<400> 19

aaagaattct atgcttttta tctttaactt tttgttttcc c 41

<210> 20
<211> 35
<212> DNA
<213> Artificial Sequence

<220>
<223> Primer

<400> 20
aaaggatcca taatcctgga tgtgctcata caggc 35

## Description

### TECHNICAL FIELD

The present invention relates to a preventative/therapeutic agent for cancer.

### BACKGROUND OF THE INVENTION

Fatty acid synthase (FAS) is an enzyme responsible for conversion of malonyl-CoA into long-chain fatty acids, which is an early reaction in fatty acid biosynthesis (Wakil, S. J., Biochemistry, Vol. 28, p. 4523-4530, 1989). FAS is overexpressed in many cancer cells (Kuhajda, F. P. et al., Proc. Natl. Acad. Sci. USA, Vol. 91, p. 6379-6383, 1994). Inhibition of FAS expression or FAS activity selectivity suppresses proliferation and induces cell death of cancer cells, with little toxicity towards normal cells (Kuhajda, F. P. et al., Proc. Natl. Acad. Sci. USA, Vol. 97, p. 3450-3454, 2000; De Schrijver, E. et al., Cancer Res., Vol. 63, p. 3799-3804, 2003). Known FAS inhibitors include Cerulenin, a natural low-molecular-weight compound derived from *Cephalosporium caerulence* (Vance, D. et al., Biochem. Biophys. Res. Commun., Vol. 48, p. 649-656, 1972) and the synthetic low-molecular weight compound C75 (Pizer, E. S. et al., Cancer Res., Vol. 60, p. 213-218, 2000).

On the other hand, enzymes in the acyl-CoA synthase (hereunder abbreviated as ACS) family are responsible for converting long-chain fatty acids into acyl-CoA. Because acyl-CoA is a substrate for intercellular lipid synthesis and fatty acid degradation/elongation reactions, ACS play a central role in intercellular lipid metabolism and also in intercellular signaling by means of lipids. Enzymes in ACS family are also involved in extracellular fatty acid uptake (Faergemen, N. J. & Knudsen, J., Biochemical J., Vol. 323, p. 1-12, 1997). Five isozymes (ACS 1, 3, 4, 5, 6) with different substrate specificities and intracellular locations have already been identified in humans and rodents (Coleman, R. A. et al., J. Nutr., Vol. 132, p. 2123-2126, 2002). Of these, ACS4 and ACS5 are overexpressed in human colon cancer and human gliomas (Cao, Y. et al., Cancer Res., Vol. 61, p. 8429-8434, 2001; Yamashita, Y. et al., Oncogene, Vol. 19, p. 5919-5925, 2000). ACS inhibition also induces cancer-specific cell death (Mashima, T. et al., J. Natl. Cancer Inst., Vol. 97, p. 765-777, 2005). Triacsin C, a natural low-molecular-weight compound derived from *Streptomyces sp.,* is known as an ACS inhibitor (Tomoda, H. et al., Biochem. Biophys. Acta, Vol. 921, p. 595-598, 1987). Triacsin C is known to inhibit ACS1 and ACS4 (Kim, J.-H. et al., J. Biol. Chem., Vol. 276, p. 24667-24673, 2001).

### DISCLOSURE OF THE INVENTION

By means of the current state of cancer therapy, many cancers cannot be cured with existing methods and there is demand for development of new therapies. A particular problem is the side-effects produced by existing anti-cancer agents in normal human tissue. There is strong demand for development of safe and advanced method which is selectively and powerfully inducing cancer cell death however showing low toxicity in normal cells in order to improve therapeutic results against cancer.

As a result of exhaustive research aimed at solving these problems, the inventors discovered that cancer cells could be killed more forcefully by simultaneously inhibiting both ACS activity and FAS activity. The present invention was perfected as a result of further research based on this finding.

That is, the present invention provides, such as:
[1] A preventative/therapeutic agent for cancer prepared by combining (i) at least one selected from a substance that inhibits the activity of an enzyme belonging to the acyl-CoA synthase family and a substance that inhibits expression of a gene for an enzyme belonging to the acyl-CoA synthase family, with (ii) at least one selected from a substance that inhibits the activity of fatty acid synthase and a substance that inhibits expression of a fatty acid synthase gene,
[2] The preventative/therapeutic agent according to [1] above, wherein the enzyme belonging to the acyl-CoA synthase family is at least one selected from the proteins comprising the same or substantially the same amino acid sequences represented by SEQ ID NO:1, SEQ ID NO:3, SEQ ID NO:5, SEQ ID NO:7, SEQ ID NO:9, SEQ ID NO:11, SEQ ID NO:13 and SEQ ID NO:15, or partial peptides or salts thereof,
[3] The preventative/therapeutic agent according to [1] above, wherein the enzyme belonging to the acyl-CoA synthase family is at least one selected from the proteins comprising the same or substantially the same amino acid sequences represented by SEQ ID NO:1, SEQ ID NO:3, SEQ ID NO:5, SEQ ID NO:7, SEQ ID NO:9 and SEQ ID NO:11, or partial peptides or salts thereof,
[4] The preventative/therapeutic agent according to [1] above, wherein the fatty acid synthase is a protein comprising the same or substantially the same amino acid sequence represented by SEQ ID NO:17, or a partial peptide or salt thereof,
[5] The preventative/therapeutic agent according to [1] above, prepared by combining (i) at least one selected from (a) a compound or salt thereof that inhibits the activity of an enzyme belonging to the acyl-CoA synthase family, (b) a compound or salt thereof that inhibits expression of a gene for an enzyme belonging to the acyl-CoA synthase family, (c) an antibody to an enzyme belonging to the acyl-CoA synthase family, (d) an antisense polynucleotide comprising a nucleotide sequence or part of a nucleotide sequence complementary or substantially complementary to the nucleotide sequence of a polynucleotide coding for an enzyme belonging to the acyl-CoA synthase family and (e) siRNA or shRNA for a polynucleotide coding for an enzyme belonging to the acyl-CoA synthase family, with (ii) at least one selected from (a) a compound or salt thereof that inhibits the activity of fatty acid synthase, (b) a compound or salt thereof that inhibit expression of a fatty acid synthase gene, (c) an antibody to fatty acid synthase, (d) an antisense polynucleotide comprising a nucleotide sequence or part of a nucleotide sequence complementary or substantially complementary to the nucleotide sequence of a polynucleotide coding for fatty acid synthase and (e) siRNA or shRNA for a polynucleotide coding for fatty acid synthase,
[6] The preventative/therapeutic agent according to [1] above, prepared by combining a compound or salt thereof that inhibits the activity of an enzyme belonging to the acyl-CoA synthase family with a compound or salt thereof that inhibits the activity of fatty acid synthase,
[7] A preventative/therapeutic agent for cancer comprising (i) a substance that inhibits the activities of an enzyme belonging to the acyl-CoA synthase family and fatty acid synthase, and/or (ii) a substance that inhibits expression of a gene for an enzyme belonging to the acyl-CoA synthase family and expression of a fatty acid synthase gene,
[8] The preventative/therapeutic agent according to Claim 1, wherein the preventative/therapeutic agent for cancer is a combination preparation,
[9] The preventative/therapeutic agent according to Claim 1, wherein the preventative/therapeutic agent is a kit comprising (i) a medicament comprising at least one selected from a substance that inhibits the activity of an enzyme belonging to the acyl-CoA family and a substance that inhibits expression of a gene for an enzyme belonging to the acyl-CoA family and (ii) a medicament comprising at least one selected from a substance that inhibits the activity of fatty acid synthase and a substance that inhibits expression of a fatty acid synthase gene,
[10] A method for preventing/treating cancer wherein (i) the activity of an enzyme belonging to the acyl-CoA family and/or expression of a gene for the enzyme is inhibited and (ii) the activity of fatty acid synthase and/or expression of a gene for the enzyme is inhibited,
[11] A method for preventing/treating cancer wherein (i) an effective dose of at least one selected from a substance that inhibits the activity of an enzyme belonging to the acyl-CoA synthase family and a substance that inhibits expression of a gene for an enzyme belonging to the acyl-CoA synthase family, and (ii) an effective dose of at least one selected from a substance that inhibits the activity of fatty acid synthase and a substance that inhibits expression of a fatty acid synthase gene, are administered to a mammal,
[12] A use of (i) at least one selected from a substance that inhibits the activity of an enzyme belonging to the acyl-CoA synthase family and a substance that inhibits expression of a gene for an enzyme belonging to the acyl-CoA synthase family, and (ii) at least one selected from a substance that inhibits the activity of fatty acid synthase and a substance that inhibits expression of a fatty acid synthase gene, to manufacture a preventative/therapeutic agent for cancer,
[13] A cancer cell apoptosis promoting agent or cancer cell proliferation inhibiting agent prepared by combining (i) at least one selected from a substance that inhibits the activity of an enzyme belonging to the acyl-CoA synthase family and a substance that inhibits expression of a gene for an enzyme belonging to the acyl-CoA synthase family, with (ii) at least one selected from a substance that inhibits the activity of fatty acid synthase and a substance that inhibits expression of a fatty acid synthase gene,
[14] The agent according to [13] above, prepared by combining (i) at least one selected from (a) a compound or salt thereof that inhibits the activity of an enzyme belonging to the acyl-CoA synthase family, (b) a compound or salt thereof that inhibits expression of a gene for an enzyme belonging to the acyl-CoA synthase family, (c) an antibody to an enzyme belonging to the acyl-CoA synthase family, (d) an antisense polynucleotide comprising a nucleotide sequence or part of a nucleotide sequence complementary or substantially complementary to the nucleotide sequence of a polynucleotide coding for an enzyme belonging to the acyl-CoA synthase family and (e) siRNA or shRNA for a polynucleotide coding for an enzyme belonging to the acyl-CoA synthase family, with (ii) at least one selected from (a) a compound or salt thereof that inhibits the activity of fatty acid synthase, (b) a compound or salt thereof that inhibits expression of a fatty acid synthase gene, (c) an antibody to fatty acid synthase, (d) an antisense polynucleotide comprising a nucleotide sequence or part of a nucleotide sequence complementary or substantially complementary to the nucleotide sequence of a polynucleotide coding for fatty acid synthase and (e) siRNA or shRNA for a polynucleotide coding for fatty acid synthase,
[15] The agent according to [13] above, prepared by combining a compound or salt thereof that inhibits the activity of an enzyme belonging to the acyl-CoA synthase family with a compound or salt thereof that inhibits the activity of fatty acid synthase,
[16] A cancer cell apoptosis promoting agent or cancer cell proliferation inhibiting agent, comprising (i) a substance that inhibits the activities of an enzyme belonging to the acyl-CoA synthase family and fatty acid synthase, and/or (ii) a substance that inhibits expression of a gene for an enzyme belonging to the acyl-CoA synthase family and expression of a fatty acid synthase gene,
[16'] A cancer cell apoptosis promotion method or cancer cell proliferation inhibition method wherein (i) the activities of an enzyme belonging to the acyl-CoA synthase family and fatty acid synthase are inhibited, and/or (ii) expression of a gene for an enzyme belonging to the acyl-CoA synthase family and expression of a fatty acid synthase gene are inhibited,
[16"] A cancer cell apoptosis promotion method or cancer cell proliferation inhibition method wherein (i) an effective dose of a substance that inhibits the activities of an enzyme belonging to the acyl-CoA synthase family and fatty acid synthase, and/or (ii) an effective dose of a substance that inhibits expression of a gene for an enzyme belonging to the acyl-CoA synthase family and expression of a fatty acid synthase gene, are administered to a mammal,
[17] A cancer cell apoptosis promotion method or cancer cell proliferation inhibition method wherein (i) the activity of an enzyme belonging to the acyl-CoA synthase family and/or expression of a gene for the enzyme is inhibited, and (ii) the activity of fatty acid synthase and/or expression of a gene for the enzyme is inhibited.
[17'] A cancer cell apoptosis promotion method or cancer cell proliferation inhibition method wherein (i) an effective dose of a substance that inhibits the activity of an enzyme belonging to the acyl-CoA synthase family and/or expression of a gene for the enzyme and (ii) an effective dose of a substance that inhibits the activity of fatty acid synthase and/or expression of a gene for the enzyme are administered to a mammal.

A "preventative/therapeutic agent for cancer" may itself be a substance having a preventative/therapeutic effect against cancer (such as a synthetic compound, peptide, protein, antibody, nonpeptidal compound, fermentation product, cell extract, plant extract, animal tissue extract, serum or the like), or may be a medicament containing such a substance. "Cancer prevention" includes prevention of metastasis and/or reoccurrence of cancer.

A "cancer cell apoptosis promoting medicament" may itself be a substance having a cancer cell apoptosis-promoting effect, or may be a medicament containing such a substance (such as a synthetic compound, peptide, protein, antibody, noneptidal compound, fermentation product, cell extract, plant extract, animal tissue extract, serum or the like).

A "cancer cell proliferation inhibiting medicament" may itself be a substance having a cancer cell proliferation inhibition effect (such as a synthetic compound, peptide, protein, antibody, noneptidal compound, fermentation product, cell extract, plant extract, animal tissue extract, serum or the like), or may be a medicament containing such a substance.

"Inhibiting expression of a gene" means that production of the protein coded for by that gene is inhibited by inhibiting any of the series of events (including transcription (mRNA production) and translation (protein production) for example) leading from the gene to protein production.

"Inhibiting expression of a protein" means that production of the protein is inhibited by inhibiting any of the series of events leading from the gene coding for the protein to protein production (including transcription (mRNA production) and translation (protein production) for example).

"Low-molecular-weight compounds" are organic and inorganic compounds with molecular weights of 10,000 or less (preferably 5,000 or less, more preferably 2,000 or less, still more preferably 700 or less).

### BRIEF DESCRIPTION OF THE DRAWINGS

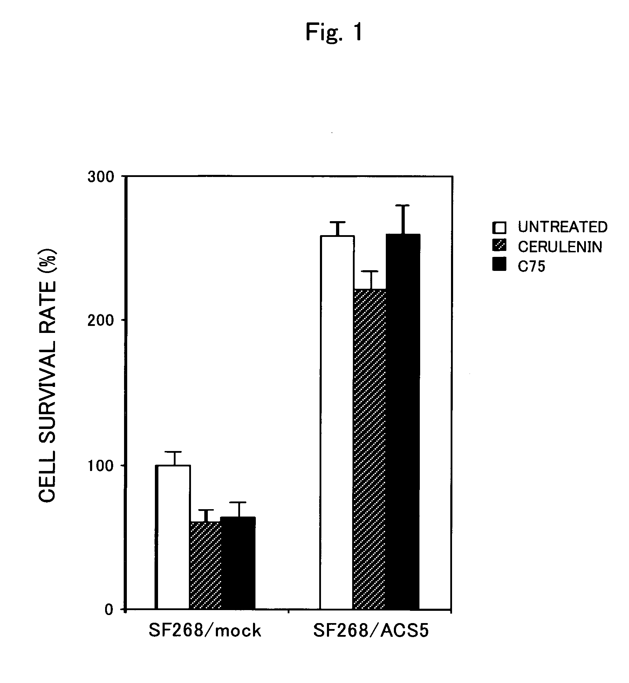

Fig. 1 shows the results for inhibition of cell proliferation when the FAS inhibitor Cerulenin (15 µg/ml) or C75 (15 µg/ml) was added to SF268/mock or SF268/ACS5 cells and cultured for 24 hours. The cell survival rate (%) using 100% untreated SF268/mock is shown on the vertical axis.

The enzyme belonging to the acyl-CoA synthase family and the fatty acid synthase (sometimes abbreviated below as proteins used in the present invention) may be synthetic proteins or proteins derived from the cells (for example, retinal cells, liver cells, spleen cells, nerve cells, glial cells, pancreatic cells, bone marrow cells, mesangial cells, Langerhans cells, epidermal cells, epithelial cells, endothelial cells, fibroblasts, fiber cells, muscle cells, fat cells, immune cells (such as macrophages, T-cells, B-cells, natural killer cells, mast cells, neutrophils, basophils, eosinophils, monocytes, platelets, etc.), megakaryocytes, synovial cells, cartilage cells, bone cells, osteoblasts, osteoclasts, mammary cells, liver cells or mesenchymal cells, or precursor cells, stem cells or cancer cells of these) of humans or warm-blooded animals (for example, guinea pigs, rats, mice, chickens, rabbits, pigs, sheep, cows and monkeys) or from tissues in which these cells are present, such as the brain, various parts of the brain (for example the retina, olfactory bulb, amygdaloid nucleus, basal ganglia, hippocampus, thalamus, hypothalamus, cerebral cortex, medulla oblongata and cerebellum), spinal cord, pituitary gland, stomach, pancreas, kidneys, liver, reproductive glands, thyroid gland, gallbladder, bone marrow, adrenal gland, skin, muscle, lungs, digestive tract (for example the large intestine and small intestine), blood vessels, heart, thymus, spleen, submandibular gland, peripheral blood, prostate gland, testicles, ovaries, placenta, uterus, bone, joints, skeletal muscle and the like, or from blood cells or cultured cells thereof (for example, MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01 and the like).

The enzyme belonging to the acyl-CoA synthase family may be at least one selected from the proteins containing the same or substantially the same amino acid sequences represented by SEQ ID NO:1, SEQ ID NO:3, SEQ ID NO:5, SEQ ID NO:7, SEQ ID NO:9, SEQ ID NO:11, SEQ ID NO:13 and SEQ ID NO:15, or the partial peptides or salts thereof

The fatty acid synthase may be at least one selected from the proteins containing the same or substantially the same amino acid sequence represented by SEQ ID NO:17, or the partial peptides or salts thereof.

The same or substantially the same amino acid sequence represented by SEQ ID NO:1, SEQ ID NO:3, SEQ ID NO:5, SEQ ID NO:7, SEQ ID NO:9, SEQ ID NO:11, SEQ ID NO:13, SEQ ID NO:15 or SEQ ID NO:17 may be an amino acid sequence having about 50% or greater or preferably about 60% or greater or more preferably about 70% or greater or still more preferably about 80% or greater or especially about 90% or greater or ideally about 95% or greater homology with an amino acid sequence represented by SEQ ID NO:1, SEQ ID NO:3, SEQ ID NO:5, SEQ ID NO:7, SEQ ID NO:9, SEQ ID NO:11, SEQ ID NO:13, SEQ ID NO:15 or SEQ ID NO:17.

Amino acid sequence homology may be calculated using the homology calculation algorithm NCBI BLAST (National Center for Biotechnology Information Basic Local Alignment Search Tool), under the following conditions: expected value = 10; gaps allowed; matrix = BLOSUM62; filtering OFF.

A protein containing substantially the same amino acid sequence represented by SEQ ID NO:1, SEQ ID NO:3, SEQ ID NO:5, SEQ ID NO:7, SEQ ID NO:9, SEQ ID NO:11, SEQ ID NO:13 or SEQ ID NO:15 is for example preferably a protein that contains substantially the same amino acid sequence represented by the aforementioned SEQ ID NO:1, SEQ ID NO:3, SEQ ID NO:5, SEQ ID NO:7, SEQ ID NO:9, SEQ ID NO:11, SEQ ID NO:13 or SEQ ID NO:15, and that has substantially the same activity as a protein containing an amino acid sequence represented by SEQ ID NO:1, SEQ ID NO:3, SEQ ID NO:5, SEQ ID NO:7, SEQ ID NO:9, SEQ ID NO:11, SEQ ID NO:13 or SEQ ID NO:15.

The activity that is substantially the same may be acyl-CoA synthase activity for example. Substantially the same means that the properties are of the same kind (that is, physiologically or pharmacologically the same). Thus, preferably the acyl-CoA synthase activity is equivalent (such as about 0.01 to 100 times or preferably about 0.1 to 10 times or more preferably 0.5 to 2 times), but quantitative factors such as the degree of such activity and the molecular weight of the protein may be different.

Acyl-CoA synthase activity can be measured by known methods, such as the methods described in J. Biol. Chem., Vol. 256, p. 5702-5707, 1981 or equivalent methods. Specifically, the protein used in the present invention is reacted for 10 minutes at 35°C in 0.5 ml of solution containing 0.2 M Tris-HCl buffer (pH 7.5), 2.5 mM ATP, 8 mM MgCl₂, 2 mM EDTA, 20 mM NaF, 0.1% (w/v) Triton X-100, 10 µM [1-¹⁴C] palmitic acid (5 µCi/µmol) and 0.5 mM coenzyme A (CoA). The reaction begins when the CoA is added, and is stopped by adding 2.5 ml of isopropanol:n-heptane:1 M sulfuric acid (40:10:1 v/v). After the reaction has been stopped, 0.5 ml of water and 2.5 ml of n-heptane are added to remove an organic solvent layer containing unreacted fatty acids, the water layer is washed three times with 2.5 ml n-heptane, and the radioactivity remaining in the water layer is measured with a scintillation counter.

The protein containing substantially the same amino acid sequence represented by SEQ ID NO:17 is for example preferably a protein that contains substantially the same amino acid sequence represented by the aforementioned SEQ ID NO:17, and that has substantially the same activity as a protein containing the amino acid sequence represented by SEQ ID NO:17.

The activity that is substantially the same may be fatty acid synthase activity for example. Substantially the same means that the properties are of the same kind (that is, physiologically or pharmacologically the same). Thus, preferably the fatty acid synthase activity is equivalent (such as about 0.01 to 100 times or preferably about 0.1 to 10 times or more preferably 0.5 to 2 times), but quantitative factors such as the degree of such activity and the molecular weight of the protein may be different.

Fatty acid synthase activity can be measured by known methods, such as those described in Nepokroeff, C. M. et al., Methods Enzymol., Vol. 26, p. 37-39, 1975 or equivalent methods. Specifically, the protein used in the present invention is reacted for 5 minutes at 30°C in 1 ml of reaction solution containing 0.5 M potassium phosphate buffer (pH 7.0), 0.2 mM malonyl-CoA, 0.066 mM acetyl-CoA and 0.2 mM NADPH, absorbancy at 340 nm is measured before and after the reaction, and enzyme activity is calculated from the difference between the two absorbancy values.

A protein used in the present invention may also be for example (1) a so-called mutein such as a protein containing (i) the amino acid sequence represented by SEQ ID NO:1, SEQ ID NO:3, SEQ ID NO:5, SEQ ID NO:7, SEQ ID NO:9, SEQ ID NO:11, SEQ ID NO:13 or SEQ ID NO:15 with 1 or 2 or more (such as about 1 to 100 or preferably 1 to 30 or more preferably 1 to 10 or still more preferably a few (1 to 5)) amino acids deleted therefrom, (ii) the amino acid sequence represented by SEQ ID NO:1, SEQ ID NO:3, SEQ ID NO:5, SEQ ID NO:7, SEQ ID NO:9, SEQ ID NO:11, SEQ ID NO:13 or SEQ ID NO:15 with 1 or 2 or more (such as about 1 to 100 or preferably 1 to 30 or more preferably 1 to 10 or still more preferably a few (1 to 5)) amino acids added thereto, (iii) the amino acid sequence represented by SEQ ID NO:1, SEQ ID NO:3, SEQ ID NO:5, SEQ ID NO:7, SEQ ID NO:9, SEQ ID NO:11, SEQ ID NO:13 or SEQ ID NO:15 with 1 or 2 or more (such as about 1 to 100 or preferably 1 to 30 or more preferably 1 to 10 or still more preferably a few (1 to 5)) amino acids inserted therein, (iv) the amino acid sequence represented by SEQ ID NO:1, SEQ ID NO:3, SEQ ID NO:5, SEQ ID NO:7, SEQ ID NO:9, SEQ ID NO:11, SEQ ID NO:13 or SEQ ID NO:15 with 1 or 2 or more (such as about 1 to 100 or preferably 1 to 30 or more preferably 1 to 10 or still more preferably a few (1 to 5)) amino acids replaced with other amino acids or (v) an amino acid sequence consisting of a combination of these, (2) a so-called mutein such as a protein containing (i) the amino acid sequence represented by SEQ ID NO:17 with 1 or 2 or more (such as about 1 to 100 or preferably 1 to 30 or more preferably 1 to 10 or still more preferably a few (1 to 5)) amino acids deleted therefrom, (ii) the amino acid sequence represented by SEQ ID NO:17 with 1 or 2 or more (such as about 1 to 100 or preferably 1 to 30 or more preferably 1 to 10 or still more preferably a few (1 to 5)) amino acids added thereto, (iii) the amino acid sequence represented by SEQ ID NO:17 with 1 or 2 or more (such as about 1 to 100 or preferably 1 to 30 or more preferably 1 to 10 or still more preferably a few (1 to 5)) amino acids inserted therein, (iv) the amino acid sequence represented by SEQ ID NO:17 with 1 or 2 or more (such as about 1 to 100 or preferably 1 to 30 or more preferably 1 to 10 or still more preferably a few (1 to 5)) amino acids replaced with other amino acids or (v) an amino acid sequence consisting of a combination of these.

When there are insertions, deletions or substitutions in an amino acid sequence as described above, the locations of such insertions, deletions or substitutions are not particularly limited.

In this Description, the left end of a protein is called the N-terminal (amino terminal) and the right end is called the C-terminal (carboxyl terminal) in accordance with the rules of peptide nomenclature. In proteins containing the amino acid sequence represented by SEQ ID NO:1 and other proteins used in the present invention, the C-terminal may be either a carboxyl group (-COOH), carboxylate (-COO⁻), amide (-CONH₂) or ester (-COOR).

In this case, a methyl, ethyl, n-propyl, isopropyl, n-butyl or other C₁₋₆ alkyl group for example, a cyclopentyl, cyclohexyl or other C₃₋₈ cycloalkyl group for example, a phenyl, α-naphthyl or other C₆₋₁₂ aryl group for example, a benzyl, phenethyl or other phenyl-C₁₋₂ alkyl group for example o r an α-naphthylmethyl or other α-naphthyl-C₁₋₂ alkyl group or other C₇₋₁₄ aralkyl group or pivaloyloxymethyl group or the like for example can be used as the R in the ester.

When a protein used in the present invention has a carboxyl group (or carboxylate) in a position other than the C-terminal, it is included as a protein used in the present invention if the carboxyl group is amidated or esterified. The ester in this case may be one such as the C-terminal ester described above or the like.

A protein used in the present invention may also be one in which an amino group of an amino acid residue (methionine residue for example) of the N-terminal is protected with a protective group (such as a formyl group, acetyl group or other C₁₋₆ alkanoyl or other C₁₋₆ acyl group), one in which a glutamine residue of the N-terminal produced by cleavage in vivo is converted to pyroglutamate, one in which a substituent (-OH, -SH, amino group, imidazole group, indole group, guanidino group or the like) on the side chain of an amino acid in the molecule is protected with a suitable protective group (such as a formyl group, acetyl group or other C₁₋₆ alkanoyl or other C₁₋₆ acyl group), or a fused protein such as a so-called glycoprotein having a sugar chain bound thereto.

Specific examples of proteins used in the present invention include proteins containing the amino acid sequence represented by SEQ ID NO:1, proteins containing the amino acid sequence represented by SEQ ID NO:3, proteins containing the amino acid sequence represented by SEQ ID NO:5, proteins containing the amino acid sequence represented by SEQ ID NO:7, proteins containing the amino acid sequence represented by SEQ ID NO:9, proteins containing the amino acid sequence represented by SEQ ID NO:11, proteins containing the amino acid sequence represented by SEQ ID NO:13, proteins containing the amino acid sequence represented by SEQ ID NO:15 and proteins containing the amino acid sequence represented by SEQ ID NO:17 and the like for example.

A partial peptide of a protein used in the present invention is a partial peptide of an aforementioned protein used in the present invention, and may preferably be any having properties equivalent to those of the aforementioned proteins used in the present invention.

For example, a peptide having an amino acid sequence of at least 20 or more or preferably 50 or more or more preferably 70 or more or still more preferably 100 or more or ideally 200 or more amino acids out of the constituent amino acid sequence of a protein used in the present invention can be used.

In a partial peptide used in the present invention, 1 or 2 or more (preferably about 1 to 20 or more preferably 1 to 10 or still more preferably a few (1 to 5)) amino acids may be deleted, 1 or 2 or more (preferably about 1 to 20 or more preferably 1 to 10 or still more preferably a few (1 to 5)) amino acids may be added, 1 or 2 or more (preferably about 1 to 20 or more preferably 1 to 10 or still more preferably a few (1 to 5)) amino acids may be inserted, or 1 or 2 or more (preferably about 1 to 20 or more preferably 1 to 10 or still more preferably a few (1 to 5)) amino acids may be replaced with other amino acids in the amino acid sequence.

Moreover, in a partial peptide used in the present invention the C-terminal may be a carboxyl group (-COOH), carboxylate (-COO⁻), amide (-CONH₂) or ester (-COOR).

Like a protein used in the present invention, moreover, a partial peptide used in the present invention may be one having a carboxyl group (or carboxylate) at a position other than the C-terminal, one in which an amino group of an N-terminal amino acid residue (such as a methionine residue) is protected, one in which a glutamine residue produced by cleavage of the N-terminal in vivo is converted to pyroglutamate, one in which a substituent on the side chain of an amino acid in the molecule is protected with a suitable protective group, or a so-called glycopeptide having a sugar chain bound thereto.

A partial peptide used in the present invention can be used as an antigen for antibody preparation.

A salt with a physiologically acceptable acid (inorganic or organic acid for example) or base (alkali metal salt for example) or the like can be used as a salt of a protein or partial peptide used in the present invention, and a physiologically acceptable acid addition salt is particularly desirable. For example, salts with inorganic acids (such as hydrochloric acid, phosphoric acid, hydrobromic acid and sulfuric acid) or organic acids (such as acetic acid, formic acid, propionic acid, fumaric acid, maleic acid, succinic acid, tartaric acid, citric acid, malic acid, oxalic acid, benzoic acid, methanesulfonic acid and benzenesulfonic acid) and the like can be used as such salts.

A protein or its partial peptide of the present invention, or a salt thereof, can be manufactured by known protein preparation methods from the aforementioned cells or tissues of humans or warm-blooded animals, or can be manufactured by culturing a transformant containing DNA coding for the protein. It may also be manufactured in accordance with the peptide synthesis methods described below.

In the case of manufacture from tissue or cells of humans or mammals, the human or mammal tissue or cell can be homogenized and extracted with acid or the like, and the resulting extract can then be purified and isolated by a combination of reverse-phase chromatography, ion-exchange chromatography and other chromatography methods.

A commercial resin for protein synthesis can normally be used for synthesizing a protein or its partial peptide of the present invention, or a salt or amide thereof. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2'-4'-dimethoxyphenyl-hydroxymethyl)phenoxy resin, 4-(2',4'-dimethoxyphenyl-Fmoc aminoethyl)phenoxy resin and the like. Using such a resin, amino acids having the α-amino groups and side-chain functional groups suitably protected are condensed on the resin by various known condensation methods according to the sequence of the target protein. At the end of the reaction, the protein or partial peptide is excised from the resin while the various protective groups are removed at the same time, and an intramolecular disulfide bond-forming reaction is then performed in a highly diluted solution to obtain the target protein or its partial peptide of the present invention, or an amide thereof.

A variety of activating reagents used in protein synthesis can be used when condensing the aforementioned protected amino acids, and carbodiimides are particularly desirable. Carbodiimides that can be used include DCC, N,N'-diisopropylcarbodiimide, N-ethyl-N'-(3-dimethylaminopropyl)carbodiimide and the like. When activating with these, the protected amino acids can be added directly to the resin together with a racemization suppressor (such as HOBt or HOOBt), or else the protected amino acids can be first activated as symmetrical acid anhydrides or HOBt or HOOBt esters, and then added to the resin.

The solvent used for activating the protected amino acids and condensing with the resin can be selected appropriately from known solvents used in protein synthesis reactions. For example, an acid amide such as N,N-dimethylformamide, N,N-dimethylacetamide or N-methylpyrrolidone, a halogenated hydrocarbon such as methylene chloride or chloroform, an alcohol such as trifluoroethanol, a sulfoxide such as dimethylsulfoxide, an ether such a pyridine, dioxane or tetrahydrofuran, a nitrile such as acetonitrile or propionitrile, an ester such as methyl acetate or ethyl acetate or a suitable mixture of these can be used. The reaction temperature can be selected appropriately from the range known to be suitable for protein bond-forming reactions, and is normally selected from the range of about -20°C to 50°C. The activated amino acid derivatives are normally used in an excess of 1.5 to 4 times. When a test using a ninhydrin reaction shows condensation to be insufficient, thorough condensation can be achieved by repeating the condensation reaction without removing the protective groups. If sufficient condensation is not obtained even by repeating the reaction, the unreacted amino acids can be acetylated using anhydrous acetic acid or acetylimidazole so as not to affect subsequent reactions.

Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like can be used as protective groups for the amino groups in the raw material.

Carboxyl groups can be protected for example by alkyl esterification (for example, methyl, ethyl, propyl, butyl, t-butyl, cyclopentyl, cyclohexyl, cycloheptyl, cyclooctyl, 2-adamantyl or other straight, branched or cyclic alkyl esterification), aralkyl esterification (for example, benzyl ester, 4-nitrobenzyl ester, 4-methoxybenzyl ester, 4-chlorobenzyl ester or benzhydryl esterification), phenacyl esterification, benzyloxycarbonyl hydrazidation, t-butoxycarbonyl hydrazidation, trityl hydrazidation or the like.

A serine hydroxyl can be protected for example by esterification or etherification. Suitable groups that can be used for such esterification include acetyl and other lower (C₁₋₆) alkanoyl group, benzoyl and other aroyl groups, and benzyloxycarbonyl, ethoxycarbonyl and other groups derived from carbonic acid and the like. Groups suited to etherification include for example benzyl, tetrahydropyranyl and t-butyl groups and the like.

Bzl, Cl₂-Bzl, 2-nitrobenzyl, Br-Z, t-butyl and the like for example can be used as protective groups for the phenolic hydroxyl group of tyrosine.

Tos, 4-methoxy-2,3,6-trimethylbenzenesulfonyl, DNP, benzyloxymethyl, Bum, Boc, Trt, Fmoc and the like for example can be used as protective groups for the imidazole of histidine.

The corresponding acid anhydride, azide, active ester (ester of an alcohol such as pentachlorophenol, 2,4,5-trichlorophenol, 2,4-dinitrophenol, cyanomethyl alcohol, paranitrophenol, HONB, N-hydroxysuccinimide, N-hydroxyphthalimide or HOBt for example) or the like for example can be used as a material in which the carboxyl groups of the raw material have been activated. The corresponding phosphoric acid amide can be used as a material in which the amino groups of the raw material have been activated.

Methods of removing (eliminating) protective groups include for example contact reduction in a flow of hydrogen gas in the presence of a catalyst such as Pd-black or Pd-carbon, acid treatment with anhydrous hydrogen fluoride, methanesulfonic acid, trifluoromethanesulfonic acid, trifluoracetic acid or a mixed liquid of these or the like, base treatment with diisopropyl ethylamine, triethylamine, piperidine, piperazine or the like, or reduction with sodium in liquid ammonia. A elimination reaction by such acid treatment is generally performed at a temperature of about -20°C to 40°C, and in the case of acid treatment it is useful to add a cation capture agent such as anisole, phenol, thioanisole, metacresol, paracresol, dimethylsulfide, 1,4-butanedithiol, 1,2-ethanedithiol or the like. Moreover, a 2,4-dinitrophenyl group used as the imidazole protective group of histidine is removed by thiophenol treatment, while a formyl group used as an indole protective group of tryptophan can be deprotected by acid treatment in the presence of 1,2-ethanedithiol, 1,4-butanedithiol or the like or removed by alkali treatment using diluted sodium hydroxide solution, diluted ammonia or the like.

Known groups and methods can be selected appropriately for protection and protective groups of functional groups that should not contribute to reaction of the raw material, elimination of such groups, and activation of functional groups contributing to the reaction.

Another method for obtaining an amide of a protein or partial peptide is for example to first amidate and protect the α-carboxy groups of the carboxy terminal amino acids, then elongate the peptide (protein) chains to the desired length on the amino group side to manufacture a protein or partial peptide with only the protective groups of the α-amino groups removed at the N-terminal of the peptide chain and a protein or partial peptide with only the protective groups of the carboxyl groups removed at the C-terminal, and condense these proteins in a mixed solvent as described above. The details of the condensation reaction are similar to those described above. The protected protein or peptide obtained by condensation can then be purified, and all the protective groups can then be removed by the aforementioned methods to obtain the desired crude protein or peptide. This crude protein or peptide can be purified using various known purification techniques, and the principal fraction can then be freeze-dried to obtain an amide of the desired protein or peptide.

To obtain an ester of a protein or peptide, for example the α-carboxyl groups of the carboxy-terminal amino acids can be condensed with an alcohol to obtain an amino acid ester, and an ester of the desired protein or peptide can be obtained in the same way as an amide of the protein or peptide.

A partial peptide or salt thereof used in the present invention can be manufactured by well-known peptide synthesis methods, or by cleaving the protein used in the present invention with a suitable peptidase. The peptide synthesis method can be either a solid-phase synthesis method or liquid-phase synthesis method for example. That is, partial peptides or amino acids capable of forming the partial peptide used in the present invention can be condensed with the residual part, and if the product has protective groups these can be desorbed to manufacture the target peptide. Examples of known condensation methods and protective group elimination methods are described for example in (i) through (v) below.
(i) M. Bodanszky and M. A. Ondetti, Peptide Synthesis, Interscience Publishers, New York (1966)
(ii) Schroeder and Luebke, The Peptide, Academic Press, New York (1965)
(iii) Izumiya, Nobuo et al., Peptide Gosei no Kiso to Jikken, Maruzen (1975)
(iv) Yajima, Haruaki and Sakakibara, Shumpei Seikagaku Jikken Koza 1, Tanpakushitsu no Kagaku IV, 205 (1977)
(v) Yajima, Haruaki Ed., Zoku-Iyakuhin no Kaihatsu, Vol. 14, Peptide Gosei, Hirokawa Shoten

After the reaction, the partial peptide used in the present invention can be purified and isolated by normal purification methods, such as a combination of solvent extraction, distillation, column chromatography, liquid chromatography, recrystallization and the like. When the partial peptide obtained by these methods is in a free form, it can be converted into the corresponding salt by known methods or similar methods, or conversely if it is obtained as a salt, it can be converted into the free form or another salt by known methods or similar methods.

A polynucleotide coding for a protein used in the present invention can be any containing a nucleotide sequence coding for the protein used in the present invention described above. Preferably it is DNA. DNA in this case may be genome DNA, a genome DNA library, cDNA from the aforementioned cells or tissues, a cDNA library from the aforementioned cells or tissue or synthetic DNA.

The vector used for the library may be a bacteriophage, plasmid, cosmid, phagemid or the like. A total RNA or mRNA fraction can also be purified from the aforementioned cells or tissues and amplified by direct Reverse Transcription Polymerase Chain Reaction (hereunder abbreviated as "RT-PCR").

DNA coding for a protein used in the present invention may be for example (1) DNA containing a nucleotide sequence represented by SEQ ID NO:2, SEQ ID NO:4, SEQ ID NO:6, SEQ ID NO:8, SEQ ID NO:10, SEQ ID NO:12, SEQ ID NO:14 or SEQ ID NO:16 or DNA that contains a nucleotide sequence that hybridizes under highly stringent conditions with a nucleotide sequence represented by SEQ ID NO:2, SEQ ID NO:4, SEQ ID NO:6, SEQ ID NO:8, SEQ ID NO:10, SEQ ID NO:12, SEQ ID NO:14 or SEQ ID NO:16 and that codes for a protein having substantially the same properties as a protein containing an amino acid sequence represented by the aforementioned SEQ ID NO:1, SEQ ID NO:3, SEQ ID NO:5, SEQ ID NO:7, SEQ ID NO:9, SEQ ID NO:11, SEQ ID NO:13 or SEQ ID NO:15, or (2) DNA containing the nucleotide sequence represented by SEQ ID NO:18, or DNA that contains a nucleotide sequence that hybridizes under highly stringent conditions with the nucleotide sequence represented by SEQ ID NO:18, and that codes for a protein having substantially the same properties as a protein containing an amino acid sequence represented by the aforementioned SEQ ID NO:17.

For example, DNA containing a nucleotide sequence having about 50% or greater or preferably about 60% or greater or more preferably about 70% or greater or still more preferably about 80% or greater or especially about 90% or greater or ideally about 95% or greater homology with a nucleotide sequence represented by SEQ ID NO:2, SEQ ID NO:4, SEQ ID NO:6, SEQ ID NO:8, SEQ ID NO:10, SEQ ID NO:12, SEQ ID NO:14 or SEQ ID NO:16 can be used as the DNA that hybridizes under highly stringent conditions with a nucleotide sequence represented by SEQ ID NO:2, SEQ ID NO:4, SEQ ID NO:6, SEQ ID NO:8, SEQ ID NO:10, SEQ ID NO:12, SEQ ID NO:14 or SEQ ID NO:16.

For example, DNA containing a nucleotide sequence having about 50% or greater or preferably about 60% or greater or more preferably about 70% or greater or still more preferably about 80% or greater or especially about 90% or greater or ideally about 95% or greater homology with the nucleotide sequence represented by SEQ ID NO:18 can be used as the DNA sequence that hybridizes under highly stringent conditions with the nucleotide sequence represented by SEQ ID NO:18.

Nucleotide sequence homology may be calculated using the homology calculation algorithm NCBI BLAST (National Center for Biotechnology Information Basic Local Alignment Search Tool), under the following conditions (expected value = 10; gaps allowed; filtering ON; match score = 1, mismatch score =-3).

Hybridization can be accomplished by known methods or similar methods, such as for example the methods described in Molecular Cloning 2nd (J. Sambrook et al., Cold Spring Harbor Lab. Press, 1989). When using a commercial library, it can be accomplished according to the methods described in the attached library. More preferably, it is accomplished under highly stringent conditions.

Highly stringent conditions are for example conditions of sodium concentration about 19 to 40 mM or preferably about 19 to 20 mM, and temperature about 50 to 70°C or preferably about 60 to 65°C. In particular, a sodium concentration of about 19 mM and a temperature of about 65°C are the most desirable.

More specifically, DNA containing the nucleotide sequence represented by SEQ ID NO:2 can be used as DNA coding for a protein containing the amino acid sequence represented by SEQ ID NO:1, DNA containing the nucleotide sequence represented by SEQ ID NO:4 as DNA coding for a protein containing the amino acid sequence represented by SEQ ID NO:3, DNA containing the nucleotide sequence represented by SEQ ID NO:6 as DNA coding for a protein containing the amino acid sequence represented by SEQ ID NO:5, DNA containing the nucleotide sequence represented by SEQ ID NO:8 as DNA coding for a protein containing the amino acid sequence represented by SEQ ID NO:7, DNA containing the nucleotide sequence represented by SEQ ID NO:10 as DNA coding for a protein containing the amino acid sequence represented by SEQ ID NO:9, DNA containing the nucleotide sequence represented by SEQ ID NO:12 as DNA coding for a protein containing the amino acid sequence represented by SEQ ID NO:11, DNA containing the nucleotide sequence represented by SEQ ID NO:14 as DNA coding for a protein containing the amino acid sequence represented by SEQ ID NO:13, DNA containing the nucleotide sequence represented by SEQ ID NO:16 as DNA coding for a protein containing the amino acid sequence represented by SEQ ID NO:15, and DNA containing the nucleotide sequence represented by SEQ ID NO:18 as DNA coding for a protein containing the amino acid sequence represented by SEQ ID NO:17.

DNA coding for a partial peptide used in the present invention may be any containing a nucleotide sequence coding for a partial peptide used in the present invention as described above. It may be either genome DNA, a genome DNA library, cDNA from the aforementioned cells or tissues, a cDNA library from the aforementioned cells or tissues or synthetic DNA.

DNA including part of DNA containing a nucleotide sequence represented by SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18, or DNA that contains a nucleotide sequence that hybridizes under highly stringent conditions with a nucleotide sequence represented by SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18 and that contains part of DNA coding for a protein having substantially equivalent activity to that of a protein used in the present invention or the like can be used as DNA coding for a partial peptide used in the present invention.

DNA capable of hybridizing with a nucleotide sequence represented by SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18 is defined as above.

Hybridization methods and highly stringent hybridization methods similar to those described above can be used.

DNA coding completely for a protein or partial peptide used in the present invention (which may be simply called a "protein used in the present invention" when explaining the cloning and expression of DNA coding therefor) can be cloned either by PCR amplification using synthetic DNA primers having part of a nucleotide sequence coding for the protein used in the present invention, or by selecting by hybridization DNA incorporated into a suitable vector that has been labeled with a DNA fragment or synthetic DNA coding for some or all regions of the protein used in the present invention. Hybridization can be accomplished for example by the methods described in Molecular Cloning 2nd Edition (J. Sambrook et al., Cold Spring Harbor Lab. Press, 1989). When using a commercial library, it can be accomplished in accordance with the methods described in the attached manual.

A DNA nucleotide sequence can be altered by PCR using a known kit such as Mutan^{™}-super Express Km (Takara), or Mutan™-K (Takara), in accordance with a known method such as ODA-LA PCR, Gapped duplex, Kunkel or the like or a similar method.

According to the objective, DNA coding for a cloned sequence can be used as is or after digestion with a restriction enzyme or addition of a linker as desired. This DNA may have ATG as a translation initiation codon at the 5' end or TAA, TGA or TAG as a translation termination codon at the 3' end. This translation initiation codon and translation termination codon can be added using suitable synthetic DNA adapters.

An expression vector for a protein used in the present invention can be manufactured for example by (i) excising a target DNA fragment from DNA coding for the protein used in the present invention, and (ii) linking this DNA fragment downstream from a promoter in a suitable expression vector.

Vectors that can be used include *E*. coli-derived plasmids (such as pBR322, pBR325, pUC12 or pUC 13), *B. subtilis-derived* plasmids (such as pUB110, pTP5 or pC 194), yeast-derived plasmids (such as pSH19 or pSH15), λ-phages and other bacteriophages, retroviruses, vaccinia virus, baculoviruses and other animal viruses and the like as well as pA1-11, pXT1, pRc/CMV, pRc/RSV, pcDNAI/Neo and the like.

Any suitable promoter corresponding to the host used to express the gene can be used as a promoter in the present invention. When an animal cell is used as the host, examples include the SRα promoter, SV40 promoter, LTR promoter, CMV promoter, HSV-TK promoter and the like.

Of these, it is desirable to use a CMV (cytomegalovirus) promoter, SRα promoter or the like. When the host is an Escherichia, it is desirable to use a trp promoter, lac promoter, recA promoter, λP_{L} promoter, lpp promoter, T7 promoter or the like, while when the host is a Bacillus it is desirable to use an SPO1 promoter, SPO2 promoter, penP promoter or the like, and when the host is a yeast it is desirable to use PHO5 promoter, PGK promoter, GAP promoter, ADH promoter or the like. When the host is an insect cell, a polyhedrin promoter, P10 promoter or the like is preferred.

In addition to these, enhancers, splicing signals, poly-A addition signals, selection markers, SV40 replication origins (sometimes abbreviated as SV40 ori) and the like may also be included in the vector as desired. Examples of selection signals include the dihydrofolate reductase (hereunder sometimes abbreviated as dhfr) gene [methotrexate (MTX) resistant], ampicillin resistance gene (sometimes abbreviated below as Amp^{r}), neomycin resistance gene (G418 resistant, sometimes abbreviated below as Neo^{r}) and the like. In particular, when using dhfr gene-deficient Chinese hamster cells with the dhfr gene as the selection marker, the target gene can be selected even with medium containing no thymidine.

A signal sequence matching the host can also be added as necessary to the N-terminal end of a protein used in the present invention. When the host is an Escherichia, PhoA signal sequence, OmpA signal sequence or the like can be used, and when the host is a Bacillus, an α-amylase signal sequence, subtilisin signal sequence or the like can be used, while when the host is a yeast, an MFα signal sequence, SUC2 signal sequence or the like can be used, and when the host is an animal cells an insulin signal sequence, α-interferon signal sequence, antibody molecule signal sequence or the like can be used.

A transformant can be produced using a vector constructed in this way that contains DNA coding for a protein used in the present invention.

Hosts that can be used include Escherichia bacteria, Bacillus bacteria, yeasts, insect cells, insects, animal cells and the like.

Specific examples of Escherichia bacteria include *Escherichia coli* K12-DH1 (Proc. Natl. Acad. Sci. USA, Vol. 60, 160 (1968)), JM103 (Nucleic Acids Research Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)) and the like.

Examples of Bacillus bacteria include *Bacillus subtilis* MI114 (Gene, Vol. 24, 255 (1983)), 207-21 (Journal of Biochemistry, Vol. 95, 87 (1984)) and the like.

Examples of yeasts include *Saccharomyces cerevisiae* AH22, AH22R⁻, NA87-11A, DKD-5D and 20B-12, *Schizosaccharomyces pombe* NCYC1913 and NCYC2036, and *Pichia pastoris* KM71 and the like.

Examples of insect cells include cell strains derived from *Spodoptera frugiperda* larvae (Sf cells), MG1 cells from the midgut of *Trichoplusia ni,* High Five^{™} cells from *Trichoplusia ni* eggs, cells from *Mamestra brassicae* or cells from *Estigmena acrea* and the like when the virus is AcNPV. When the virus is BmNPV, cell strains from silkworms *(Bombyx mori* N cells; BmN cells) and the like can be used. Examples of such Sf cells include Sf9 cells (ATCC CRL1711), Sf21 cells (Vaughn, J. L. et al., In Vivo, 13, 213-217, (1977)) and the like.

Silkworm larvae or the like can be used as insects (Maeda et al., Nature, Vol. 315, 592 (1985)).

Animal cells that can be used include monkey COS-7 cells, Vero cells, Chinese hamster CHO cells (hereunder abbreviated as CHO cells), dhfr gene-deficient Chinese hambster CHO cells (hereunder abbreviated as CHO (dhfr⁻) cells, mouse L cells, mouse AtT-20, mouse myeloma cells, mouse ATDC5 cells, rat GH3, human FL cells and the like.

Escherichia bacteria can be transformed in accordance with the methods described in Proc. Natl. Acad. Sci. USA, Vol. 69, 2110 (1972) and Gene, Vol 17, 107 (1982) and the like for example.

Bacillus bacteria can be transformed in accordance with the methods described in Molecular 7 General Genetics, Vol. 168, 111 (1979) and the like for example.

Yeasts can be transformed in accordance with the methods described in Methods in Enzymology, Vol. 194, 182-187 (1991) and Proc. Natl. Acad. Sci. USA, Vol. 75, 1929 (1978) and the like for example.

Insect cells or insects can be transformed in accordance with the methods described in Bio/Technology, 6, 47-55 (1988) and the like for example.

Animal cells can be transformed in accordance with the methods described in Saibo Kogaku Bessatsu 8, Shin Saibo Kogaku Jikken Protocol 263-267 (1995) (Shujunsha) and Virology, Vol. 52, 456 (1973) for example.

In this way, a transformant can be obtained that has been transformed with an expression vector containing DNA coding for a protein.

When culturing a transformant for which the host is an Escherichia or Bacillus bacterium, liquid medium is suitable as the medium for culture, with the carbon sources, nitrogen sources, inorganic substance and the like necessary for growth of the transformant included therein. Examples of carbon sources include glucose, dextrin, soluble starch, sucrose and the like for example, while examples of nitrogen sources include ammonium salts, nitrates, corn steep liquor, peptone, casein, meat extract, soybean cake, potato extract and other inorganic and organic materials, and examples of inorganic substance include calcium chloride, sodium dihydrogenphosphate, magnesium chloride and the like. Yeast extract, vitamins, growth promotion factors and the like may also be added. The pH of the medium is preferably about 5 to 8.

The medium for culturing an Escherichia bacterium is preferably M9 medium (Miller, Journal of Experiments in Molecular Genetics, 431-433, Cold Spring Harbor Laboratory, New York, 1972) containing glucose and casamino acids. A chemical such as 3β-indolylacrylic acid can also be added as necessary to cause the promoter to act efficiently.

When the host is an Escherichia, culture is normally performed for about 3 to 24 hours at about 15 to 43°C, with aeration and agitation as necessary.

When the host is a Bacillus, culture is normally performed for about 6 to 24 hours at about 30 to 40°C, with aeration and agitation as necessary.

When culturing a transformant for which the host is a yeast, the medium may be for example Burkholder minimal medium (Bostian, K. L. et al., Proc. Natl. Acad. Sci. USA, Vol. 77, 4505 (1980)) or SD medium containing 0.5% casamino acids (Bitter, G. A. et al., Proc. Natl. Acad. Sci. USA, Vol. 81, 5330 (1984)). The pH of the medium is preferably adjusted to about 5 to 8. Culture is normally performed for about 24 to 72 hours at about 20°C to 35°C, with aeration and agitation as necessary.

When culturing a transformant for which the host is an insect cell or insect, Grace's Insect Medium (Grace, T. C. C., Nature, 195, 788 (1962) with 10% immobilized bovine serum or other additives added thereto as appropriate can be used as the medium. The pH of the medium is preferably adjusted to about 6.2 to 6.4. Culture is normally performed for about 3 to 5 days at about 27°C, with aeration and agitation as necessary.

When culturing a transformant for which the host is an animal cells, the medium may be for example MEM medium containing about 5 to 20% fetal bovine serum (Science, Vol. 122, 501 (1952)), DMEM medium (Virology, Vol. 8, 396 (1959)), RPMI 1640 medium (the Journal of the American Medical Association, Vol. 199, 519 (1967)), 199 medium (Proceeding of the Society for the Biological Medicine, Vol. 73, 1 (1950)) or the like. The pH is preferably adjusted to about 6 to 8. Culture is normally performed for about 15 to 60 hours at about 30°C to 40°C, with aeration and agitation as necessary.

In this way, a protein used in the present invention can be produced in the cells or cell membranes or outside the cells of a transformant.

The protein used in the present invention can be isolated and purified from such a culture by the following methods for example.

To extract a protein used in the present invention from cultured bacteria or cells, a method can be used in which the bacteria or cells are collected after culture by known methods, suspended in a suitable buffer and disrupted by ultrasound, lisozyme and/or freeze-drying treatment, and a crude protein extract is then obtained by centrifugation or filtration. A protein denaturant such as urea or guanidine hydrochloride or a surfactant such as Triton X-100^{™} can also be included in the buffer. When the protein is excreted in the culture liquid, after completion of culture the bacteria or cells can be separated by known methods from the supernatant, which is then collected.

The protein contained in culture supernatant or extract thus obtained can be purified by a suitable combination of well-known separation and purification methods. Examples of such known separation and purification methods include salting out, solvent precipitation and other methods using solubility, dialysis, ultrafiltration, gel filtration, SDS-polyacrylamide gel electrophoresis and other methods using primarily differences in molecular weight, ion exchange chromatography and other methods using differences in charge, affinity chromatography and other methods using differences in hydrophobicity, isoelectric point electrophoresis and other methods using differences in isoelectric point and the like.

When the protein obtained in this way is obtained in a free form, it can be converted into a salt by known methods or their equivalents, or conversely when it is obtained as a salt it can be converted by known methods or their equivalents into a free form or another salt.

By applying a suitable protein-modifying enzyme to the protein produced by a recombinant, either before or after purification it is also possible to modify the protein as desired or partially remove polypeptides. Trypsin, chymotrypsin, arginyl endopeptidase, protein kinase, glycosidase or the like can be used as the protein-modifying enzyme.

The presence of a protein used in the present invention produced in this way can be measured by Western blotting or enzyme immunoassay using a specific antibody.

An antibody to a protein used in the present invention, or a partial peptide or salt thereof, may be either a polyclonal antibody or a monoclonal antibody as long as it is an antibody that recognizes the protein used in the present invention or a partial peptide or salt thereof.

An antibody to a protein used in the present invention or a partial peptide or salt thereof (which may be abbreviated simply as "a protein used in the present invention" in the following explanation of antibodies) can be manufactured by known antibody or antiserum manufacturing methods using a protein used in the present invention as the antigen.

### [Preparation of Monoclonal Antibody]

### (a) Preparation of monoclonal antibody-producing cells

A protein used in the present invention is administered by itself or together with a carrier or diluent to a warm-blooded animal at a site capable of antibody production. Complete Freund's adjuvant or incomplete Freund's adjuvant can also be administered at the time of administration in order to enhance antibody production. The protein is normally administered a total of 2 to 10 times at 2 to 6 week intervals. Examples of warm-blooded animals that can be used include monkeys, rabbits, dogs, guinea pigs, mice, rats, sheep, goats and chickens, but mice and rats are preferred.

When preparing monoclonal antibody-producing cells, individuals with confirmed antibody titer can be selected from warm-blooded animals (such as mice) that have been immunized with the antigen, the spleens or lymph nodes can be extracted 2 to 5 days after the final immunization, and the antibody-producing cells contained in these can be fused to myeloma cells from the same or different animal species to prepare a monoclonal antibody-producing hybridoma. Antibody titer in antiserum can be measured for example by first reacting the labeled protein (described below) with antiserum, and then measuring the activity of a labeling agent bound to the antibody. The fusing operation can be performed by known methods, such as those of Köhler and Milstein (Nature, 256, 495 (1975)). Examples of fusion promoters include polyethylene glycol (PEG) and sendai virus, but PEG is preferred.

Examples of myeloma cells include NS-1, P3U1, SP2/0, AP-1 and other myeloma cells from warm-blooded animals, but P3U1 is used by preference. With the ratio of number of antibody-producing cells (spleen cells) used to myeloma cells in the range of about 1:1 to 20:1, PEG (preferably PEG1000 to PEG 6000) is added at a concentration of about 10 to 80%, and the cells are incubated for 1 to 10 minutes at 20 to 40°C or preferably 30 to 37°C to efficiently fuse the cells.

The monoclonal antibody-producing hybridoma can be screened by a variety of methods, such as for example by a method of adding hybridoma culture serum to a solid phase (such as a microplate) having the protein antigen adsorbed thereon directly or together with a carrier, then adding protein A or an anti-immunoglobulin antibody (an anti-mouse immunoglobulin antibody when the cells used for cell fusion are mouse cells) labeled with a radioactive substance or enzyme, and detecting the monoclonal antibody bound to the solid phase, or by a method of adding hybridoma culture serum to a solid phase having an anti-immunoglobulin antibody or protein A bound thereon, adding the protein labeled with a radioactive substance or enzyme, and detecting the monoclonal antibody bound to the solid phase.

Monoclonal antibody selection can be accomplished by known methods or similar methods. It is normally performed in animal cell medium with HAT (hypoxanthine, aminopterin, thymidine) added thereto. The medium for selection and breeding can be may be any medium in which the hybridoma can grow. For example, RMPI 1640 medium containing 1 to 20% or preferably 10 to 20% bovine fetal serum, GIT medium containing 1 to 10% bovine fetal serum (Wako Pure Chemical Industries) or serum-free medium for hybridoma culture (SFM-101, Nissui Pharmaceutical Co.) or the like can be used. The culture temperature is normally 20 to 40°C or preferably about 37°C. The culture time is normally 5 days to 3 weeks or preferably 1 to 2 weeks. Culture is normally performed in 5% carbon dioxide gas. The antibody titer of the hybridoma culture serum can be measured in the same way as the antibody titer of antiserum as described above.

### (b) Purification of monoclonal antibody

The monoclonal antibody can be separated and purified by known methods, such as for example by immunoglobulin separation and purification methods (for example, salting out, alcohol precipitation, isoelectric point precipitation, electrophoresis, adsorption-elimination with an ion-exchange body (such as DEAE), ultracentrifugation, gel filtration, or a specific purification method in which the antibody alone is collected with an antigen-bound solid phase or an active adsorbent such as protein A or protein G, and the bonds are released to obtain the antibody).

### [Preparation of polyclonal antibody]

A polyclonal antibody of the present invention can be manufactured by known methods or similar methods. For example, the immune antigen (protein antigen) can be prepared by itself or as a fused body together with a carrier protein, a warm-blooded animal can be immunized by the same methods used for the monoclonal antibody above, matter containing an antibody to the protein used in the present invention can be collected from this immune animal, and the antibody can then be separated and purified.

When the immune antigen used to immunize the warm-blooded animal is fused to a carrier protein, the type of carrier protein and the ratio of carrier and hapten can be any that allow the antibody to be efficiently produced in response to immunization with the hapten crosslinked to the carrier, but for example bovine serum albumin, bovine thyroglobulin, hemocyanin or the like can be coupled at a weight ratio of about 0.1 to 20 or preferably about 1 to 5 per 1 of hapten.

A variety of condensation agents can be used to couple the hapten and carrier protein, and active ester reagents containing glutaraldehyde, carbodiimide, maleimide active ester, thiol groups and dithiopyridine groups and the like are used.

The condensation product is administered by itself or together with a carrier or diluent to a warm-blooded animal at a site capable of antibody production. Complete Freund's adjuvant or incomplete Freund's adjuvant can be administered at the same time to enhance antibody-producing ability. Administration is normally performed a total of about 3 to 10 times at 2 to 6 week intervals.

The polyclonal antibody can be collected from the blood or ascites or the like and preferably from the blood of a warm-blood animal immunized by the methods described above.

Polyclonal antibody titer in antiserum can be measured in the same way as antibody titer in antiserum as described above. The polyclonal antibody can be separated and purified by immunoglobulin separation and purification methods in the same way as the monoclonal antibody described above.

An antisense polynucleotide having a nucleotide sequence or part of a nucleotide sequence complementary or substantially complementary to the nucleotide sequence of a polynucleotide (preferably DNA) (such DNA is sometimes called "DNA used in the present invention" in the following explanation of antisense polynucleotides) coding for a protein used in the present invention or its polypeptide may be any antisense polynucleotide having a nucleotide sequence or part of a nucleotide sequence complementary or substantially complementary to the nucleotide sequence of DNA used in the present invention, but is preferably antisense DNA.

A nucleotide sequence substantially complementary to DNA used in the present invention may for example be a nucleotide sequence having about 70% or greater or preferably about 80% or greater or more preferably about 90% or greater or still more preferably about 95% or greater homology with all or part of a nucleotide sequence complementary to DNA used in the present invention (that is, the complement strand of DNA used in the present invention). Out of the total nucleotide sequence of the complement strand of DNA used in the present invention, desirable examples are (i) an antisense polynucleotide having about 70% or greater or preferably about 80% or greater or more preferably about 90% or greater or still more preferably about 95% or greater homology with the complement strand of a part of the nucleotide sequence (such as a nucleotide sequence near the initiation codon) that codes for the N-terminal part of the protein used in the present invention) when the antisense polynucleotide is designed for inhibiting translation and (ii) an antisense polynucleotide having about 70% or greater or preferably about 80% or greater or more preferably about 90% or greater or still more preferably about 95% or greater homology with the complement strand of the total nucleotide sequence of DNA used in the present invention including an intron when the antisense polynucleotide is designed for RNA decomposition with RNaseH.

Specific examples include an antisense polynucleotide having a nucleotide sequence or part of a nucleotide sequence complementary or substantially complementary to the nucleotide sequence of DNA containing a nucleotide sequence represented by SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18, and preferably for example an antisense polynucleotide having a nucleotide sequence or part of a nucleotide sequence complementary to the nucleotide sequence of DNA containing a nucleotide sequence represented by SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18 (more preferably an antisense polynucleotide having a nucleotide sequence complementary to the nucleotide sequence of DNA containing a nucleotide sequence represented by SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18).

An antisense polynucleotide is normally composed of about 10 to 40 or preferably about 15 to 30 nucleotides.

In order to prevent decomposition by nucleases and other hydrolyzing enzymes, the phosphoric acid residues (phosphates) of each of the nucleotides making up the antisense DNA can be replaced with chemically modified phosphoric acid residues such as phosphothioate, methylphosphonate, phosphorodithionate and the like. The sugar (deoxyribose) of each nucleotide may also be replaced with a 2'-O-methylated or other chemically modified sugar structure, or the bases thereof (pyrimidine, purine) may also be chemically modified, as long as it hybridizes with DNA having the nucleotide sequence represented by SEQ ID NO:2. These antisense polynucleotides can be manufactured using a known DNA synthesizer.

In the present invention, an antisense polynucleotide (nucleic acid) corresponding to a protein gene used in the present invention, and capable of blocking replication or expression of that gene, is designed and synthesized based on the nucleotide sequence data of DNA coding for the cloned or sequenced protein. This antisense polynucleotide can hybridize with RNA of the protein gene used in the present invention, and is capable of either interfering with the synthesis or functioning of that RNA, or of regulating and controlling expression of the protein gene used in the present invention through interaction with protein-associated RNA used in the present invention. A polynucleotide complementary to a selected sequence of protein-associated RNA used in the present invention, and a polynucleotide capable of hybridizing specifically with protein-associated RNA used in the present invention, are useful in vivo and ex vivo for regulating and controlling expression of a protein gene used in the present invention, and are also useful for treating and diagnosing disease. The term "corresponding" means homologous or complementary to a nucleotide, nucleotide sequence or specific nucleic acid sequence including a gene. "Correspondence" between a nucleotide, nucleotide sequence or nucleic acid and a protein normally indicates the amino acid sequence of a protein induced from (governed by) a nucleotide (nucleic acid) sequence or its complement. A 5'-terminal hairpin loop, 5'-terminal 6-base pair repeat, 5'-terminal untranslated region, polypeptide translation initiation codon, protein coding region, ORF translation termination codon, 3'-terminal untranslated region, 3'-terminal palindrome region or 3'-terminal hairpin loop or the like of a protein gene can be selected as a desirable object domain, but any domain within the protein gene can also be used.

In terms of the association between the target nucleic acids and a polynucleotide complementary to at least part of the object domain, when the target nucleic acids are capable of hybridizing with the object domain, the target nucleic acids can be called "antisense" with respect to the polynucleotide of the object domain. Examples of antisense polynucleotides include polynucleotides containing 2-deoxy-D-ribose, polynucleotides containing D-ribose, other types of polynucleotides that are the N-glycosides of purine or pyrimidine bases, other polymers having non-nucleotide frameworks (such as commercial protein nucleic acids and synthetic sequence-specific nucleic acid polymers) or other polymers containing special bonds (but which must contain nucleotides having arrangements that allow nucleotide hairpins or nucleotide attachments such as occur in DNA or RNA). These may be double-stranded DNA, single-stranded DNA, double-stranded RNA, single-stranded RNA or DNA:RNA hybrids, and may be unmodified polynucleotides (or unmodified oligonucleotides) or may have a known modification added thereto, such as for example by addition of a known label, capping or methylation, replacement of 1 or more natural nucleotides with analogs, intramolecular nucleotide modification such as with uncharged bonds (methylphosphonate, phosphotriester, phosphoramidate, carbamate, etc.) or with bonds having charge or sulfur-containing bonds (phosphorothioate, phosphorodithioate, etc.) for example, or with a protein (for example a nuclease, nuclease inhibitor, toxin, antibody, signal peptide, poly-L-lysine, etc.), sugar (monosaccharide or the like for example) or other side-chain group, or with an intercalator compound (acridine, psoralen or the like for example), or with a chelate compound (such as a metal, radioactive metal, boron, oxidizing metal or the like), or with an alkylating agent, or with a modified bond (for example, an α-anomeric nucleic acid or the like). The terms "nucleoside", "nucleotide" and "nucleic acid" mean not only those having purine and pyrimidine bases, but also include those having other modified heterocyclic bases. These may also include methylated purine and pyrimidine, acylated purine and pyrimidine or other heterocyclic rings. The sugar parts of modified nucleotides and modified nucleotides may also be modified, such as for example by substituting a halogen or aliphatic group for 1 or more hydroxyl groups, or by substituting an ether, amine or other functional group.

An antisense polynucleotide of the present invention is RNA, DNA or a modified nucleic acid (RNA, DNA). Specific examples of modified nucleic acids are degradation-resistant sulfurized and thiophosphate derivatives of nucleic acids, and polynucleoside amides and oligonucleoside amides. The antisense polynucleotide of the present invention can be designed for example as follows. That is, the antisense polynucleotide can be made more stable in cells, the cell permeability of the antisense polynucleotide can be enhanced, affinity for the target sense chain can be improved, or the toxicity of the antisense polynucleotide can be reduced if it is toxic. Many such modifications are reported in Pharm. Tech. Japan, Vol. 8, page 247 or page 395, 1992 and Antisense Research and Applications, CRC Press, 1993 and the like.

The antisense polynucleotide of the present invention may also contain altered or modified sugars, bases and bonds, or may be provided in a special form such as a liposome or microsphere, or may be applied by means of gene therapy, or may be provided in an adducted form. A polycation such as polylysine that serves to neutralize the charge of the phosphate structure, or a lipid that enhances interaction with the cell membrane and increases nucleic acid uptake (such as a phospholipid or cholesterol) or another hydrophobic molecule can be use in this adducted form. Suitable lipids for adduction include cholesterol and its derivatives (for example, cholesteryl chloroformate, cholic acid and the like). These can be attached to the 3'-terminal or 5'-terminal of the nucleic acid, or can be attached via a base, sugar or intramolecular nucleoside bond. Examples of other bases include capping bases arranged specifically at the 3'-terminal or 5'-terminal of the nucleic acid, which are used in block degradation by exonuclease, RNase and other nucleases. Examples of such capping bases include polyethylene glycol, tetraethylene glycol and other glycols as well as other hydroxyl protective groups known in the field, but are not limited to these.

The inhibitory activity of the antisense polynucleotide can be investigated using the transformant of the present invention, an in vivo or ex vivo gene expression system of the present invention, or an in vivo or ex vivo translation system of the protein for use in the present invention.

The preventative/therapeutic agent for cancer and other medicament of the present invention are explained in detail below.

A protein comprising substantially the same amino acid sequence represented by SEQ ID NO:1, SEQ ID NO:3, SEQ ID NO:5, SEQ ID NO:7, SEQ ID NO:9, SEQ ID NO:11, SEQ ID NO:13 or SEQ ID NO:15 is sometimes abbreviated as "protein A used in the present invention", while a protein comprising substantially the same amino acid sequence represented by SEQ ID NO:17 is sometimes abbreviated as "protein B used in the present invention".

An enzyme belonging to the acyl-CoA synthase family is also sometimes called "protein A used in the present invention", while fatty acid synthase is sometimes called "protein B used in the present invention".

Cancer cell apoptosis can be selectively and strongly induced and cancer cells can be selectively and effectively killed by simultaneously inhibiting the activity or expression of protein A used in the present invention and the activity or expression of protein B used in the present invention. Thus, a drug or the like obtained by combining (i) at least one selected from a substance that inhibits the activity of protein A used in the present invention (for example, acyl-CoA synthase activity) and a substance that inhibits expression of a gene for protein A used in the present invention with (ii) at least one selected from a substance that inhibits the activity of protein B used in the present invention (for example, fatty acid synthase activity) and a substance that inhibits expression of a gene for protein B used in the present invention can be used as a safe drug such as for example as a preventative/therapeutic agent for cancer (such as brain tumor, hypophyseal adenoma, glioma, acoustic neurilemmoma, retinal sarcoma, thyroid cancer, throat cancer, cancer of the larynx, tongue cancer, thymic cancer, mesothelial cancer, breast cancer, lung cancer, non-small-cell lung cancer, small-cell lung cancer, stomach cancer, esophageal cancer, duodenal cancer, large intestinal cancer, colon cancer, rectal cancer, liver cancer, hepatocarcinoma, pancreatic cancer, pancreatic endocrine tumor, bile duct cancer, gallbladder cancer, penis cancer, kidney cancer, renal pelvic cancer, ureter cancer, renal cell cancer, testicular cancer, prostate cancer, bladder cancer, vulvar cancer, uterine cancer, cervical cancer, cancer of the uterine body, uterine sarcoma, trophoblastic disease, vaginal cancer, ovarian cancer, ovarian germ cell tumor, skin cancer, malignant myeloma, mycosis fungoides, basal cell cancer, soft tissue sarcoma, malignant lymphoma, Hodgkin's disease, myelodysplastic syndrome, multiple myeloma, leukemia, acute myelocytic leukemia, chronic myelocytic leukemia, acute lymphocytic leukemia, chronic lymphocytic leukemia, adult T-cell leukemia, chronic myeloproliferative disease, pancreatic endocrine tumors, fibrous histiocytoma, leiomyosarcoma, rhabdomyosarcoma, cancers of unknown primary origin and the like), as a cancer cell apoptosis promoting agent, or as a cancer cell proliferation inhibiting agent.

The (i) at least one selected from a substance that inhibits the activity of protein A used in the present invention (for example, acyl-CoA synthase activity) and a substance that inhibits expression of a gene for protein A used in the present invention and the (ii) at least one selected from a substance that inhibits the activity of protein B used in the present invention (for example, fatty acid synthase activity) and a substance that inhibits expression of a gene for protein B used in the present invention may also be the same substance. Since such a substance, that is, a substance that inhibits (i') the activity of protein A used in the present invention or expression of a gene for protein A used in the present invention and (ii') the activity of protein B used in the present invention or expression of a gene for protein B used in the present invention, is also capable of simultaneously inhibiting the activity or expression of protein A used in the invention and the activity or expression of protein B used in the present invention, it can be used favorably as a drug such as a preventative/therapeutic agent for cancer, a cancer cell apoptosis promoting agent, or a cancer cell proliferation inhibiting agent.

Examples of substances that inhibit the activity of protein A used in the present invention include for example (a) compounds or salts thereof that inhibit the activity of protein A used in the present invention, (c) antibodies to protein A used in the present invention, and (f) mutants of protein A used in the present invention that have a dominant negative effect on protein A used in the present invention, or polynucleotides coding therefor and the like.

Since (a) a compound or salt thereof that inhibits the activity of protein A used in the present invention is capable of inhibiting the activity of protein A used in the present invention, it can be used favorably as a substance that inhibits the activity of protein A used in the present invention. A compound or salt thereof that inhibits the activity of protein A used in the present invention is not particularly limited as long as it is a compound or salt thereof capable of inhibiting an activity of protein A used in the present invention (such as acyl-CoA synthase activity), but examples include compounds and salts thereof that bind to protein A used in the present invention to thereby inhibit its activity. Such a compound or salt thereof may be a compound selected for example from the peptides, proteins, nonpeptide compounds, synthetic compounds, fermentation products, cell extracts, plant extracts, animal tissue extracts, plasma and the like for example. This compound may be a novel compound or a known compound. Examples of salts of this compound include physiologically acceptable metal salts, ammonium salts, salts with organic bases, salts with inorganic acids, salts with organic acids, salts with basic or acidic amino acids and the like. Desirable examples of metal salts include sodium salts, potassium salts and other alkali metal salts; calcium salts, magnesium salts, barium salts and other alkali earth metal salts; and aluminum salts and the like. Desirable examples of salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Desirable examples of salts with inorganic acids include salts with hydrochloric acid, hydrobromic acid, nitric acid, sulfuric acid, phosphoric acid and the like. Desirable examples of salts with organic acids include salts with formic acid, acetic acid, trifluoracetic acid, propionic acid, phthalic acid, fumaric acid, oxalic acid, tartaric acid, maleic acid, citric acid, succinic acid, malic acid, methanesulfonic acid, benzoic acid, benzenesulfonic acid, p-toluenesulfonic acid and the like. Desirable examples of salts with basic amino acids include salts with arginine, lysine, ornithine and the like, while desirable examples of salts with acidic amino acids include salts with aspartic acid, glutamic acid and the like.

Of these, a physiologically acceptable salt is preferred. Examples include alkali metal salts (for example, sodium salts, potassium salts and the like), alkali earth metal salts (for example, calcium salts, magnesium salts, barium salts and the like) and other inorganic metal salts and ammonium salts and the like when there is an acidic functional group in the compound and, and salts with hydrobromic acid, nitric acid, sulfuric acid, phosphoric acid and other inorganic acids and acetic acid, phthalic acid, fumaric acid, oxalic acid, tartaric acid, maleic acid, citric acid, succinic acid, methanesulfonic acid, p-toluenesulfonic acid and other organic acids when there is a basic functional group in the compound.

A low-molecular-weight compound is preferred as the compound or salt thereof that inhibits the activity of protein A used in the present invention.

Examples of the compound or salt thereof that inhibits the activity of protein A used in the present invention include Triacsin C, 2-bromopalmitic acid or salts of these, and Triacsin C or its salt is preferred.

This compound or its salt may be formulated and administered by ordinary methods.

Examples of compositions for oral administration include solid or liquid dosage forms, such as specifically tablets (including sugar-coated tablets and film-coated tablets), pills, granules, powders, capsules (including soft capsules), syrups, emulsions, suspensions and the like. These compositions can be manufactured by known methods, and may contain carriers, diluents or excipients commonly used in the pharmaceutical field. For example, lactose, starch, sucrose, magnesium stearate and the like can be used as carriers and excipients in tablets.

Examples of compositions for non-oral administration include injections, suppositories and the like, and injections include intravenous injections, subcutaneous injections, intracutaneous injections, muscular injections, drip injections, intraarticular injections and other formulations. These injections can be prepared by known methods, such as by dissolving, suspending or emulsifying the aforementioned substance in a sterile aqueous or oily liquid commonly used for injections. Physiological saline and isotonic fluid containing glucose or other adjuvants and the like can be used as aqueous liquids for injections, and a suitable solubilizer such as an alcohol (for example ethanol), polyalcohol (for example propylene glycol, polyethylene glycol) or non-ionic surfactant (for example polysorbate 80 or HCO-50 (polyoxyethylene (50 mol) adduct of hydrogenated castor oil)) may also be included. Sesame oil, soybean oil and the like can be used as oily liquids, and benzyl benzoate, benzyl alcohol or the like may be included as a solubilizer. The prepared injection liquid is normally packed in a suitable ampoule. A suppository for rectal administration is prepared by mixing the aforementioned substance with a suitable suppository base.

Since (c) an antibody to protein A used in the present invention and preferably an antibody having the effect of neutralizing (reducing or eliminating) the activity of protein A used in the present invention (neutralizing enzyme) is capable of inhibiting the activity of protein A used in the present invention, it can be used favorably as a substance that inhibits the activity of protein A used in the present invention.

This antibody can be administered by itself or as a suitable drug composition. The drug composition used for such administration contains the antibody or its salt with a physiologically acceptable carrier, diluent or excipient. This composition is provided in a dosage form suited to oral or non-oral (such as intravenous) administration. It is preferably provided as an inhalation.

The compositions described above may also contain other active components to the extent that these do not cause undesirable interactions when compounded with the antibody.

Since (f) a mutant of protein A used in the present invention that has a dominant negative effect on protein A used in the present invention, or a polynucleotide coding therefor, is capable of inhibiting the activity of protein A used in the present invention, it can be used favorably as a substance that inhibits the activity of protein A used in the present invention. In this Description, "a mutant of protein A used in the present invention that has a dominant negative effect on protein A used in the present invention" is a protein that, when expressed, has the effect of inhibiting (eliminating or reducing) the activity of protein A used in the present invention (see Taira, Kazunari Ed., Idenshi to Kino Sogai Jikkenho, Yodosha, p. 26-32, 2001, etc.).

Such a mutant of protein A used in the present invention or a polynucleotide coding therefor can be formulated and administered by ordinary methods.

A substance that inhibits expression of protein A used in the present invention is not limited as long as it inhibits expression of protein A used in the present invention, but may be (i) a substance that inhibits transcription of a gene (DNA) coding for protein A used in the present invention into mRNA coding for protein A used in the present invention or (ii) a substance that inhibits translation of mRNA coding for protein A used in the present invention into protein A used in the present invention. (i) A substance that inhibits transcription of a gene (DNA) coding for protein A used in the present invention into mRNA coding for protein A used in the present invention is not particularly limited as long as it inhibits transcription of a gene (DNA) coding for protein A used in the present invention into mRNA coding for protein A used in the present invention, but examples include substances that inhibit transcription of genes (DNA) coding for protein A used in the present invention into mRNA by binding to factors involved in transcription of genes (DNA) coding for protein A used in the present invention into mRNA. (ii) A substance that inhibits translation of mRNA coding for protein A used in the present invention into protein A used in the present invention is not particularly limited as long as it inhibits translation of mRNA coding for protein A used in the present invention into protein A used in the present invention, but examples include substances that inhibit translation of mRNA coding for protein A used in the present invention into protein A used in the present invention by binding to factors involved in translation of mRNA coding for protein A used in the present invention into protein A used in the present invention. Specific examples of such substances that inhibit expression of protein A used in the present invention include (b) a compound or salt thereof that inhibits expression of protein A used in the present invention, (d) an antisense polynucleotide containing a nucleotide sequence or part of a nucleotide sequence complementary or substantially complementary to the nucleotide sequence of a polynucleotide coding for protein A used in the present invention, (e) double-stranded RNA having an RNAi effect on a polynucleotide coding for protein A used in the present invention (such as siRNA or shRNA for a polynucleotide coding for protein A used in the present invention), and (g) a polynucleotide having ribozyme activity with respect to a polynucleotide coding for protein A used in the present invention.

Since (b) a compound or salt thereof that inhibits expression of protein A used in the present invention is capable of suppressing expression of protein A used in the present invention, it can be used favorably as a substance that inhibits expression of protein A used in the present invention. A compound or salt thereof that inhibits expression of protein A used in the present invention is not particularly limited as long as it inhibits expression of protein A used in the present invention, but may be (i) a compound that inhibits transcription of a gene (DNA) coding for protein A used in the present invention into mRNA coding for protein A used in the present invention or (ii) a compound that inhibits translation of mRNA coding for protein A used in the present invention into protein A used in the present invention. (i) A compound that inhibits transcription of a gene (DNA) coding for protein A used in the present invention into mRNA coding for protein A used in the present invention is not particularly limited as long as it inhibits transcription of a gene (DNA) coding for protein A used in the present invention into mRNA, but examples include compounds that inhibit transcription by binding to factors involved in transcription of genes (DNA) coding for protein A used in the present invention into mRNA. (ii) A compound that inhibits translation of mRNA coding for protein A used in the present invention into protein A used in the present invention is not particularly limited as long as it inhibits translation of mRNA coding for protein A used in the present invention into protein A used in the present invention, but examples include compounds that inhibit translation by binding to factors involved in translation of mRNA coding for protein A used in the present invention into protein A used in the present invention.

A compound or salt thereof that inhibits expression of protein A used in the present invention is preferably a low-molecular-weight compound.

Examples of compounds or salts thereof that inhibit expression of protein A used in the present invention include Triacsin C, 2-bromopalmitic acid or salts of these, and Triacsin C or its salt is preferred.

The aforementioned compound or salt thereof can be formulated and administered by ordinary methods in the same way as the aforementioned compound or salt thereof that inhibits the activity of protein A used in the present invention for example.

Since (d) an antisense polynucleotide containing a nucleotide sequence or part of a nucleotide sequence complementary or substantially complementary to the nucleotide sequence of a polynucleotide coding for protein A used in the present invention is of low toxicity, is capable of suppressing expression of a gene coding for protein A used in the present invention and is capable of suppressing expression of protein A used in the present invention, it can be used favorably as a substance that inhibits expression of protein A used in the present invention. Those described above and the like can be used as such antisense polynucleotides.

This antisense polynucleotide can be formulated and administered by known methods.

Moreover, this antisense polynucleotide can be administered orally or non-orally by ordinary methods to humans or mammals (such as rats, rabbits, sheep, pigs, cows, cats, dogs, monkeys and the like), either alone or after insertion into a suitable vector such as a retrovirus vector, adenovirus vector, adenovirus associated virus vector or the like. This antisense polynucleotide can be formulated as is or together with an adjuvant for promoting ingestion or other physiologically acceptable carriers, and can be administered via a gene gun or hydrogel catheter or other catheter. Alternatively, it can be made into an aerosol and administered locally to the trachea as an inhalant.

The antisense polynucleotide can also be formulated as an injection alone or together with a liposome or other carrier and administered intravenously, subcutaneously, into a joint cavity or into a cancer lesion site in order to improve the pharmacokinetics, extend the half-life and improve the efficiency of cellular uptake.

Since (e) double-stranded RNA having an RNAi effect on a polynucleotide coding for protein A used in the present invention (such as siRNA or shRNA for a polynucleotide coding for protein A used in the present invention) is of low toxicity, is capable of inhibiting translation of a gene coding for protein A used in the present invention and is capable of suppressing expression of protein A used in the present invention, it can be used favorably as a substance that inhibits expression of protein A used in the present invention. Examples of such double-stranded RNA having an RNAi effect on a polynucleotide coding for protein A used in the present invention include double-stranded RNA containing part of RNA coding for a protein used in the present invention (such as siRNA (small (short) interfering RNA) or shRNA (small (short) hairpin RNA for a polynucleotide coding for protein A used in the present invention).

Such double-stranded RNA can be designed and manufactured based on the polynucleotide sequence of the present invention in accordance with known methods (see for example Nature, Vol. 411, p. 494, 2001; Patent Publication No. 2002-516062; US Patent Application Disclosure No. 2002/086356, Description; Nature Genetics, Vol. 24, p. 180-183, 2000; Genesis, Vol. 26, p. 240-244, 2000; Nature, Vol. 407, p. 319-320, 2002; Genes & Deve., Vol. 16, p. 948-958, 2002; Proc. Natl. Acad. Sci. USA, Vol. 99, p. 5515-5520, 2002; Science, Vol. 296, p. 550-553, 2002; Proc. Natl. Acad. Sci. USA, Vol. 99, p. 6047-6052, 2002; Nature Biotechnology, Vol. 20, p. 497-500, 2002; Nature Biotechnology, Vol. 20, p. 500-505, 2002; Nucleic Acids Res., Vol. 30, e46, 2002).

The length of double-stranded RNA having an RNAi effect that is used in the present invention is normally 17 to 30 bases or preferably 19 to 27 bases of more preferably 20 to 22 bases.

This double-stranded RNA can be formulated and administered in the same way as the aforementioned antisense polynucleotide.

Since (g) a polynucleotide having ribozyme activity with respect to a polynucleotide coding for protein A used in the present invention is capable of suppressing expression of protein A used in the present invention, it can be used favorably as a substance that inhibits expression of protein A used in the present invention. Such a ribozyme can be designed and manufactured based on the polynucleotide sequence of the present invention in accordance with known methods (see for example Trends in Molecular Medicine, Vol. 7, p. 221, 2001; FEBS Lett., Vol. 228, p. 228, 1988; FEBS Lett., Vol. 239, p. 285, 1988; Nucl. Acids Res., Vol. 17, p. 7059, 1989; Nature, Vol. 323, p. 349, 1986; Nucl Acids Res., Vol. 19, p. 6751, 1991; Protein Eng., Vol. 3, p. 733, 1990; Nucl Acids Res., Vol. 19, p. 3875, 1991; Nucl. Acids Res., Vol. 19, p. 5125, 1991; Biochem. Biophys. Res. Commun., Vol. 186, p. 1271, 1992). For example, it can be manufactured by linking a known ribozyme to part of RNA coding for protein A used in the present invention. The part of RNA coding for protein A used in the present invention may be a part (RNA fragment) near a cleavage site on RNA of the present invention that can be cleaved by a known ribozyme. This ribozyme may be a large ribozyme such as group I intron ribozyme or M1 RNA (a subunit of RNaseP), or may be a hammerhead, hairpin or other small ribozyme (Protein, Nucleic Acid and Enzyme, Vol. 35, p. 2191, 1990). Regarding hammerhead ribozymes, see for example FEBS Lett., Vol. 228, p. 228, 1988; FEBS Lett., Vol. 239, p. 285, 1988; Protein, Nucleic Acid and Enzyme, Vol. 35, p. 2191, 1990; Nucl. Acids Res., Vol. 17, p. 7059, 1989 and the like. Regarding hairpin ribozymes, see for example Nature, Vol. 323, p. 349, 1986; Nucl. Acids Res., Vol. 19, p. 6751, 1991; Kagaku to Seibutsu, Vol. 30, p. 112, 1992 and the like.

This polynucleotide having ribozyme activity can be prepared and administered in the same way as the aforementioned antisense polynucleotide.

Examples of substances that inhibit the activity of protein B used in the present invention include for example (a) compounds and salts thereof that inhibit the activity of protein B used in the present invention, (c) antibodies to protein B used in the present invention and (f) mutants of protein B used in the present invention that have a dominant negative effect on protein B used in the present invention, or polynucleotides coding therefor and the like.

Since (a) a compound or salt thereof that inhibits the activity of protein B used in the present invention is capable of inhibiting the activity of protein B used in the present invention, it can be used favorably as a substance that inhibits the activity of protein B used in the present invention. A compound or salt thereof that inhibits the activity of protein B used in the present invention is not particularly limited as long as it is a compound or salt thereof capable of inhibiting an activity of protein B used in the present invention (such as fatty acid synthase activity), but examples include compounds and salts thereof that bind to protein B used in the present invention to thereby inhibit its activity. Such a compound or salt thereof may be a compound selected for example from the peptides, proteins, nonpeptide compounds, synthetic compounds, fermentation products, cell extracts, plant extracts, animal tissue extracts, plasma and the like for example. This compound may be a novel compound or a known compound. Examples of salts of this compound include those similar to salts of the compound that inhibits the activity of protein B used in the present invention as described above.

A low-molecular-weight compound is preferred as the compound or salt thereof that inhibits the activity of protein B used in the present invention.

Examples of the compound or salt thereof that inhibits the activity of protein B used in the present invention include Cerulenin, C75 or salts of these, and Cerulenin and C75 or salts of these are preferred.

This compound or its salt may be formulated and administered in the same way as the compound or salt thereof that inhibits the activity of protein A used in the present invention as described above.

Since (c) an antibody to protein B used in the present invention and preferably an antibody (neutralizing antibody) having the effect of neutralizing (reducing or eliminating) the activity of protein B used in the present invention is capable of inhibiting the activity of protein B used in the present invention, it can be used favorably as a substance that inhibits the activity of protein B used in the present invention.

This antibody can be formulated and administered in the same way as the aforementioned antibody to protein A used in the present invention.

Since (f) a mutant of protein B used in the present invention that has a dominant negative effect on protein B used in the present invention, or a polynucleotide coding therefor, is capable of inhibiting the activity of protein B used in the present invention, it can be used favorably as a substance that inhibits the activity of protein B used in the present invention. In this Description, "a mutant of protein B used in the present invention that has a dominant negative effect on protein B used in the present invention" is a protein that, when the polynucleotide coding therefor is expressed, has the effect of inhibiting (eliminating or reducing) the activity of protein B used in the present invention (see Taira, Kazunari Ed., Idenshi no Kino Sogai Jikkenho, Yodosha, p. 26-32, 2001, etc.).

Such a mutant of protein B used in the present invention or a polynucleotide coding therefor can be formulated and administered by ordinary methods.

A substance that inhibits expression of protein B used in the present invention is not limited as long as it inhibits expression of protein B used in the present invention, but may be (i) a substance that inhibits transcription of a gene (DNA) coding for protein B used in the present invention into mRNA coding for protein B used in the present invention or (ii) a substance that inhibits translation of mRNA coding for protein B used in the present invention into protein B used in the present invention. (i) A substance that inhibits transcription of a gene (DNA) coding for protein B used in the present invention into mRNA coding for protein B used in the present invention is not particularly limited as long as it inhibits transcription of a gene (DNA) coding for protein B used in the present invention into mRNA coding for protein B used in the present invention, but examples include substances that inhibit transcription of genes (DNA) coding for protein B used in the present invention into mRNA by binding to factors involved in transcription of genes (DNA) coding for protein B used in the present invention into mRNA. (ii) A substance that inhibits translation of mRNA coding for protein B used in the present invention into protein B used in the present invention is not particularly limited as long as it inhibits translation of mRNA coding for protein B used in the present invention into protein B used in the present invention, but examples include substances that inhibit translation of mRNA coding for protein B used in the present invention into protein B used in the present invention by binding to factors involved in translation of mRNA coding for protein B used in the present invention into protein B used in the present invention. Specific examples of such substances that inhibit expression of protein B used in the present invention include (b) a compound or salt thereof that inhibits expression of protein B used in the present invention, (d) an antisense polynucleotide containing a nucleotide sequence or part of a nucleotide sequence complementary or substantially complementary to the nucleotide sequence of a polynucleotide coding for protein B used in the present invention, (e) double-stranded RNA having an RNAi effect on a polynucleotide coding for protein B used in the present invention (such as siRNA or shRNA for a polynucleotide coding for protein B used in the present invention), and (g) a polynucleotide having ribozyme activity with respect to a polynucleotide coding for protein B used in the present invention.

Since (b) a compound or salt thereof that inhibits expression of protein B used in the present invention is capable of suppressing expression of protein B used in the present invention, it can be used favorably as a substance that inhibits expression of protein B used in the present invention. A compound or salt thereof that inhibits expression of protein B used in the present invention is not particularly limited as long as it inhibits expression of protein B used in the present invention, but may be (i) a compound that inhibits transcription of a gene (DNA) coding for protein B used in the present invention into mRNA coding for protein B used in the present invention or (ii) a compound that inhibits translation of mRNA coding for protein B used in the present invention into protein B used in the present invention. (i) A compound that inhibits transcription of a gene (DNA) coding for protein B used in the present invention into mRNA coding for protein B used in the present invention is not particularly limited as long as it inhibits transcription of a gene (DNA) coding for protein B used in the present invention into mRNA, but examples include compounds that inhibit transcription by binding to factors involved in transcription of genes (DNA) coding for protein B used in the present invention into mRNA. (ii) A compound that inhibits translation of mRNA coding for protein B used in the present invention into protein B used in the present invention is not particularly limited as long as it inhibits translation of mRNA coding for protein B used in the present invention into protein B used in the present invention, but examples include compounds that inhibit translation by binding to factors involved in translation of mRNA coding for protein B used in the present invention into protein B used in the present invention.

A compound or salt thereof that inhibits expression of protein B used in the present invention is preferably a low-molecular-weight compound.

Examples of compounds or salts thereof that inhibit expression of protein B used in the present invention include Cerulenin, C75 or salts of these, and Cerulenin and C75 or their salts are preferred.

This compound or salt thereof can be formulated and administered by ordinary methods in the same way as the aforementioned compound or salt thereof that inhibits the expression of protein A used in the present invention.

Since (d) an antisense polynucleotide containing a nucleotide sequence or part of a nucleotide sequence complementary or substantially complementary to the nucleotide sequence of a polynucleotide coding for protein B used in the present invention is of low toxicity, is capable of suppressing expression of a gene coding for protein B used in the present invention and is capable of suppressing expression of protein B used in the present invention, it can be used favorably as a substance that inhibits expression of protein B used in the present invention. Those described above and the like can be used as such antisense polynucleotides.

This antisense polynucleotide can be formulated and administered in the same way as the aforementioned an antisense polynucleotide containing a nucleotide sequence or part of a nucleotide sequence complementary or substantially complementary to the nucleotide sequence of a polynucleotide coding for protein A used in the present invention.

Since (e) double-stranded RNA having an RNAi effect on a polynucleotide coding for protein B used in the present invention (such as siRNA or shRNA for a polynucleotide coding for protein B used in the present invention) is of low toxicity, is capable of inhibiting translation of a gene coding for protein B used in the present invention and is capable of inhibiting expression of protein B used in the present invention, it can be used favorably as a substance that inhibits expression of protein B used in the present invention. Examples of such double-stranded RNA having an RNAi effect on a polynucleotide coding for protein B used in the present invention include double-stranded RNA containing part of RNA coding for a protein used in the present invention (such as siRNA (small (short) interfering RNA) or shRNA (small (short) hairpin RNA) for a polynucleotide coding for protein B used in the present invention).

Such double-stranded RNA can be designed and manufactured based on a polynucleotide sequence of the present invention using methods similar to those used for double-stranded RNA having an RNAi effect on a polynucleotide coding for protein A used in the present invention.

The length of double-stranded RNA having an RNAi effect that is used in the present invention is normally 17 to 30 bases or preferably 19 to 27 bases of more preferably 20 to 22 bases.

This double-stranded RNA can be formulated and administered in the same way as the aforementioned antisense polynucleotide.

Since (g) a polynucleotide having ribozyme activity with respect to a polynucleotide coding for protein B used in the present invention is capable of inhibiting expression of protein B used in the present invention, it can be used favorably as a substance that inhibits expression of protein B used in the present invention. Such a ribozyme can be designed and manufactured based on a polynucleotide sequence of the present invention in accordance with known methods (see for example Trends in Molecular Medicine, Vol. 7, p. 221, 2001; FEBS Lett., Vol. 228, p. 228, 1988; FEBS Lett., Vol. 239, p. 285, 1988; Nucl. Acids Res., Vol. 17, p. 7059, 1989; Nature, Vol. 323, p. 349, 1986; Nucl Acids Res., Vol. 19, p. 6751, 1991; Protein Eng., Vol. 3, p. 733, 1990; Nucl Acids Res., Vol. 19, p. 3875, 1991; Nucl. Acids Res., Vol. 19, p. 5125, 1991; Biochem. Biophys. Res. Commun., Vol. 186, p. 1271, 1992). For example, it can be manufactured by linking a known ribozyme to part of RNA coding for protein B used in the present invention. The part of RNA coding for protein B used in the present invention may be a part (RNA fragment) near a cleavage site on RNA of the present invention that can be cleaved by a known ribozyme. This ribozyme may be a large ribozyme such as group I intron ribozyme or M1 RNA (a subunit of RNaseP), or may be a hammerhead, hairpin or other small ribozyme (Protein, Nucleic Acid and Enzyme, Vol. 35, p. 2191, 1990). See the references described above with respect to hammerhead ribozymes and hairpin ribozymes.

This polynucleotide having ribozyme activity can be prepared and administered in the same way as the aforementioned antisense polynucleotide.

A preventative/therapeutic agent for cancer, cancer cell apoptosis promoting agent or cancer cell proliferation suppressing agent or the like prepared by combining a compound or salt thereof that inhibits the activity of protein A used in the present invention with a compound or salt thereof that inhibit the activity of protein B used in the present invention is preferred as the aforementioned preventative/therapeutic agent for cancer or other medicament of the present invention.

Moreover, since (i) a substance that inhibits the activities of an enzyme belonging to the acyl-CoA synthase family and fatty acid synthase and (ii) a substance that inhibits expression of a gene for an enzyme belonging to the acyl-CoA synthase family and expression of a fatty acid synthase gene and the like are capable of simultaneously inhibiting the activity or expression of protein A used in the present invention and inhibiting the activity or expression of protein B used in the present invention, they can be used favorably as the aforementioned preventative/therapeutic agent for cancer or other medicament of the present invention.

Moreover, a (i) a substance that inhibits the activities of an enzyme belonging to the acyl-CoA synthase family and fatty acid synthase and (ii) a substance that inhibits expression of a gene for an enzyme belonging to the acyl-CoA synthase family and expression of a fatty acid synthase gene may also be the same substance.

The preventative/therapeutic agent for cancer or other medicament of the present invention may be in a form capable of simultaneously inhibiting the activity or expression of protein A used in the present invention and the activity or expression of protein B used in the present invention. For example, (i) at least one selected from a substance that inhibits the activity of protein A used in the present invention and a substance that inhibits expression of a gene for protein A used in the present invention, and (ii) at least one selected from a substance that inhibits the activity of protein B used in the present invention and a substance that inhibits expression of a gene for protein B used in the present invention can be formulated as a combination preparation in a single drug composition (such as a tablet (including sugar-coated tablets and film-coated tablets), pill, granules, powder, capsule (including soft capsules), syrup, emulsion, suspension, injection, suppository or the like). Moreover, the preventative/therapeutic agent for cancer or other medicament of the present invention may also be a kit comprising (i) a medicament comprising at least one selected from a substance that inhibits the activity of protein A used in the present invention and a substance that inhibits expression of a gene for protein A used in the present invention and (ii) a medicament comprising at least one selected from a substance that inhibits the activity of protein B used in the present invention and a substance that inhibits expression of a gene for protein B used in the present invention. In this case, the (i) medicament comprising at least one selected from a substance that inhibits the activity of protein A used in the present invention and a substance that inhibits expression of a gene for protein A used in the present invention and the (ii) medicament comprising at least one selected from a substance that inhibits the activity of protein B used in the present invention and a substance that inhibits expression of a gene for protein B used in the present invention may be administered with a time interval in between as long as the activity or expression of protein A used in the present invention and the activity or expression of protein B used in the present invention can be simultaneously inhibited, but preferably they are administered simultaneously.

The dosage ratio (or compounding ratio) of the (i) at least one selected from a substance that inhibits the activity of protein A used in the present invention and a substance that inhibits expression of a gene for protein A used in the present invention and the (ii) at least one selected from a substance that inhibits the activity of protein B used in the present invention and a substance that inhibits expression of a gene for protein B used in the present invention in the preventative/therapeutic agent for cancer or other medicament of the present invention differs according to the types and/or combination of substance that inhibits the activity of protein A used in the present invention, substance that inhibits expression of a gene for protein A used in the present invention, substance that inhibits the activity of protein B used in the present invention and substance that inhibits expression of a gene for protein B used in the present invention, and according to the subject of administration, target illness, symptoms, administration route and the like, but for example the weight ratio is about 1:500 to 500:1 or preferably about 1:100 to 100:1 or more preferably 1:10 to 10:1 1 or still more preferably 1:5 to 5:1.

When using (i) at least one selected from a substance that inhibits the activity of protein A used in the present invention and a substance that inhibits expression of a gene for protein A used in the present invention, and (ii) at least one selected from a substance that inhibits the activity of protein B used in the present invention and a substance that inhibits expression of a gene for protein B used in the present invention as the aforementioned agent, they can be formulated by ordinary means in accordance with the methods described above for example.

For example, in the case of a composition for oral administration examples include solid and liquid dosage forms, such as specifically tablets (including sugar-coated tablets and film-coated tablets), pills, granules, powders, capsules (including soft capsules), syrups, emulsions, suspensions and the like. These compositions can be manufactured by known methods, and may contain carriers, diluents or excipients commonly used in the pharmaceutical field. For example, lactose, starch, sucrose, magnesium stearate and the like can be used as carriers and excipients in tablets.

Examples of compositions for non-oral administration include injections, suppositories and the like, and injections include intravenous injections, subcutaneous injections, intracutaneous injections, muscular injections, drip injections, intraarticular injections and other formulations. Injections can be prepared by known methods, such as by dissolving, suspending or emulsifying the aforementioned substance in a sterile aqueous or oily liquid commonly used for injections. Physiological saline and isotonic fluid containing glucose or other adjuvants and the like can be used as aqueous liquids for injections, and a suitable solubilizer such as an alcohol (for example ethanol), polyalcohol (for example propylene glycol, polyethylene glycol) or non-ionic surfactant (for example polysorbate 80 or HCO-50 (polyoxyethylene (50 mol) adduct of hydrogenated castor oil)) may also be included. Sesame oil, soybean oil and the like can be used as oily liquids, and benzyl benzoate, benzyl alcohol or the like may be included as a solubilizer. The prepared injection liquid is normally packed in a suitable ampoule. A suppository for rectal administration is prepared by mixing the aforementioned substance with a suitable suppository base.

It is convenient to prepare the aforementioned drug composition for oral or non-oral use in a unit dosage form for administration according to the dosages of the active ingredients. Examples of such unit dosage forms for administration include tablets, pills, capsules, injections (ampoules), suppositories and the like, and each dosage form for administration normally contains 5 to 500 mg or particularly 5 to 100 mg in the case of an injection or 10 to 250 mg in the case of other dosage forms of both (i) at least one selected from a substance that inhibits the activity of protein A used in the present invention and a substance that inhibits expression of a gene for protein A used in the present invention, and (ii) at least one selected from a substance that inhibits the activity of protein B used in the present invention and a substance that inhibits expression of a gene for protein B used in the present invention.

Each of the compositions described above may also contain other active components to the extent that these do not produce undesirable side-effects when compounded with the aforementioned substances.

Because a preparation obtained in this way is safe and of low toxicity, it can be administered orally or non-orally to humans or warm-blooded mammals (such as mice, rats, rabbits, sheep, pigs, cows, horses, birds, cats, dogs, monkeys, chimpanzees and the like).

The dosages of these compounds or salts thereof differ according to the effects, the target illness, the subject of administration and the symptoms, administration route and the like, but generally about 0.1 to 100 mg or preferably about 1.0 to 50 mg or more preferably about 1.0 to 20 mg per day of each substance is administered to an adult (body weight 60 kg) in the case of oral administration of (i) at least one selected from a substance that inhibits the activity of protein A used in the present invention and a substance that inhibits expression of a gene for protein A used in the present invention, and (ii) at least one selected from a substance that inhibits the activity of protein B used in the present invention and a substance that inhibits expression of a gene for protein B used in the present invention for the treatment of lung cancer. In the case of non-oral administration, the dosages differ depending on the target illness, the subject of administration, the symptoms, the administration route and the like, but in general it is convenient to administer about 0.01 to 30 mg or preferably about 0.1 to 20 mg or more preferably about 0.1 to 10 mg per day of each substance intravenously to an adult (body weight 60 kg) when administering (i) at least one selected from a substance that inhibits the activity of protein A used in the present invention and a substance that inhibits expression of a gene for protein A used in the present invention, and (ii) at least one selected from a substance that inhibits the activity of protein B used in the present invention and a substance that inhibits expression of a gene for protein B used in the present invention in injectable form for the treatment of lung cancer. In the case of other animals, an equivalent dosage per 60 kg of body weight can be administered.

This preventative/therapeutic agent for cancer or other medicament of the present invention can also be used in combination with hormone therapy agents, anticancer agents (such as a chemotherapy agents, immunotherapeutic agents and drugs that inhibit the activity of cell growth factors and cell growth factor receptors) or the like (hereunder called combined drugs). In this case the administration period is not limited, and these may be administered to the subject of administration either simultaneously or with a time interval in between. The dosages can be selected appropriately based on dosages in clinical use. The compounded ratio of the preventative/therapeutic agent for cancer or other medicament of the present invention and the combined drug can be selected appropriately according to the subject of administration, administration route, target illness, symptoms, combination and the like.

Examples of "hormone therapy agents" include fosfestrol, diethylstilbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, tadenan, mepartricin, raloxifene, ormeloxifene, levormeloxifene, anti-estrogens (e.g., tamoxifen citrate, toremifene citrate and the like), ER down-regulators (e.g., fulvestrant and the like), human chorionic gonadotropin, follicle-stimulating hormone, birth-control pills, mepitiostane, testrolactone, aminoglutethiimide, LH-RH agonists (e.g., goserelin acetate, buserelin, leuprorelin and the like), droloxifene, epitiostanol, ethinylestradiol sulfonate, aromatase inhibitors (e.g., fadrozole hydrochloride, anastrozole, retrozole, exemestane, vorozole, formestane and the like), anti-androgens (e.g., flutamide, bicartamide, nilutamide and the like), 5α-reductase inhibitors (e.g., finasteride, dutasteride, epristeride and the like), adrenocorticohormone drugs (e.g., dexamethasone, prednisolone, betamethasone, triamcinolone and the like), androgen synthesis inhibitors (e.g., abiraterone and the like), retinoids and drugs that retard retinoid metabolism (e.g., liarozole and the like), etc. LH-RH agonists (e.g., goserelin acetate, buserelin, leuprorelin and the like) are preferable.

Examples of "chemotherapy agents" include alkylating agents, antimetabolites, anticancer antibiotics, plant-derived anticancer agents and the like.

Examples of "alkylating agents" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucid, carboplatin, cisplatin, miboplatin, nedaplatin, oxaliplatin, altretamine, ambamustine, dibrospidiuim hydrochloride, fotemustine, prednimustine, pumitepa, ribomustin, temozolomide, treosulfan, trophosphamide, zinostatin stimalamer, adozelesin, cystemustine, bizelesin and the like.

Examples of "antimetabolites" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur and the like), aminopterine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine and the like.

Examples of "anticancer antibiotics" include actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride and the like.

Examples of "plant-derived anticancer agents" include etoposide, etoposide phosphate, vinblastine sulfate, vincristine sulfate, vindesine sulfate, teniposide, paclitaxel, docetaxel, vinorelbine and the like.

Examples of "immunotherapeutic agents (BRM)" include picibanil, krestin, sizofiran, lentinan, ubenimex, interferons, interleukins, macrophage colony-stimulating factor, granulocyte colony-stimulating- factor, erythropoietin, lymphotoxin, BCG vaccine, Corynebacterium parvum, levamisole, polysaccharide K, procodazole and the like.

The "cell growth factors" in the "drugs that inhibit the activity of cell growth factors and cell growth factor receptors" may be any substances that promote cell proliferation, and are normally peptides having a molecular weight of not more than 20,000 that are capable of exhibiting their activity at low concentrations by binding to a receptor, including (1) EGF (epidermal growth factor) or substances possessing substantially the same activity as EGF [e.g., EGF, heregulin (HER2 ligand) and the like], (2) insulin or substances possessing substantially the same activity as insulin [e.g., insulin, IGF (insulin-like growth factor)-1, IGF-2 and the like], (3) FGF (fibroblast growth factor) or substances possessing substantially the same activity as FGF [e.g., acidic FGF, basic FGF, KGF (keratinocyte growth factor), FGF-10 and the like], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), and the like], and the like.

"Cell growth factor receptors" may be any receptors capable of binding to the aforementioned cell growth factors, and specific examples include EGF receptor, heregulin receptor (HER2), insulin receptor, IGF receptor, FGF receptor-1 or FGF receptor-2 and the like.

Examples of "drugs that inhibit the activity of cell growth factors" include trastuzumab (trade mark: Herceptin (anti-HER2 antibody)), imatinib mesilate, ZD1839 or cetuximab, antibodies to VEGF (such as bevacizumab), antibodies to VEGF receptors, gefitinib, erlotinib and the like.

In addition to the aforementioned drugs, L-asparaginase, aceglatone, procarbazine hydrochloride, protoporphyrin-cobalt complex salt, mercuric hematoporphyrin-sodium, topoisomerase I inhibitors (e.g., irinotecan, topotecan and the like), topoisomerase II inhibitors (e.g., sobuzoxane and the like), differentiation inducers (e.g., retinoid, vitamin D and the like), angiogenesis inhibitors (e.g., thalidomide, SU11248 and the like), α-blockers (e.g., tamsulosin hydrochloride, naftopidil, urapidil, alfuzosin, terazosin, prazosin, silodosin and the like), serine/threonine kinase inhibitors, endothelin receptor antagonists (e.g., atrosentan and the like), proteosome inhibitors (e.g., bortezomib and the like), Hsp90 inhibitors (e.g., 17-AAG and the like), spirolactone, minoxidil, 1 1α-hydroxyprogesteron, bone absorption inhibitors and metastasis suppressors (e.g., zoledronic acid, alendronic acid, pamidronic acid, etidronic acid, ibandronic acid, clodronic acid) and the like can also be used.

When bases and amino acids are represented by abbreviations in this Description and in the sequence tables, these are based on the abbreviations given by the IUPAC-IUB Commission on Biochemical Nomenclature, or on abbreviations commonly used in the field. Examples are given below. When an amino acid may have an optical isomer, the L-form is indicated unless otherwise specified.
- DNA:: Deoxyribonucleic acid
- cDNA:: Complementary deoxyribonucleic acid
- A:: Adenine
- T:: Thymine
- G:: Guanine
- C:: Cytosine
- RNA:: Ribonucleic acid
- mRNA :: Messenger ribonucleic acid
- dATP:: Deoxyadenosine triphosphate
- dTTP:: Deoxythymidine triphosphate
- dGTP:: Deoxyguanosine triphosphate
- dCTP:: Deoxycytidine triphosphate
- ATP:: Adenosine triphosphate
- EDTA:: Ethylenediaminetetraacetic acid
- SDS:: Sodium dodecyl sulfate
- Gly:: Glycine
- Ala:: Alanine
- Val:: Valine
- Leu:: Leucine
- Ile:: Isoleucine
- Ser:: Serine
- Thr:: Threonine
- Cys:: Cysteine
- Met:: Methionine
- Glu:: Glutamic acid
- Asp:: Aspartic acid
- Lys:: Lysine
- Arg:: Arginine
- His:: Histidine
- Phe:: Phenylalanine
- Tyr:: Tyrosine
- Trp:: Tryptophan
- Pro:: Proline
- Asn:: Asparagine
- Gln:: Glutamine
- pGlu:: Pyroglutamic acid
- Sec:: Selenocysteine

Substituents, protective groups and reagents frequently mentioned in this description are abbreviated as follows.
- Me:: Methyl grouup
- Et:: Ethyl group
- Bu:: Butyl group
- Ph:: Phenyl group
- TC:: Thiazolidine-4(R)-carboxamide group
- Tos:: p-toluenesulfonyl
- CHO:: Formyl
- Bzl:: Benzyl
- Cl₂-Bzl:: 2,6-dichlorobenzyl
- Bom:: Benzyloxymethyl
- Z:: Benzyloxycarbonyl
- Cl-Z:: 2-chlorobenzyloxycarbonyl
- Br-Z:: 2-bromobenzyloxycarbonyl
- Boc:: t-butoxycarbonyl
- DNP:: Dinitrophenyl
- Trt:: Trityl
- Bum:: t-butoxymethyl
- Fmoc:: N-9-fluorenylmethoxycarbonyl
- HOBt:: 1-hydroxybenzotriazole
- HOOBt:: 3,4-dihydro-3-hydroxy-4-oxo-1,2,3-benzotriazine
- HONB:: 1-hydroxy-5-norbornene-2,3-dicarboxyimide
- DCC:: N,N'-dicyclohexylcarbodiimide

The sequence ID numbers in the sequence tables of this Description represent the following sequences.
[SEQ ID NO:1]
   Represents the amino acid sequence of human ACS1.
[SEQ ID NO:2]
   Represents the nucleotide sequence of cDNA coding for human ACS 1.
[SEQ ID NO:3]
   Represents the amino acid sequence of human ACS3
[SEQ ID NO:4]
   Represents the nucleotide sequence of cDNA coding for human ACS3.
[SEQ ID NO:5]
   Represents the amino acid sequence of isoform 1 of human ACS4.
[SEQ ID NO:6]
   Represents the nucleotide sequence of cDNA coding for isoform 1 of human ACS4.
[SEQ ID NO:7]
   Represents the amino acid sequence of isoform 2 of human ACS4.
[SEQ ID NO:8]
   Represents the nucleotide sequence of cDNA coding for isoform 2 of human ACS4.
[SEQ ID NO:9]
   Represents the amino acid sequence of isoform a of human ACS5.
[SEQ ID NO:10]
   Represents the nucleotide sequence of cDNA coding for isoform a of human ACS5.
[SEQ ID NO:11]
   Represents the amino acid sequence of isoform b of human ACS5.
[SEQ ID NO:12]
   Represents the nucleotide sequence of cDNA coding for isoform b of human ACS5.
[SEQ ID NO:13]
   Represents the amino acid sequence of isoform a of human ACS6.
[SEQ ID NO:14]
   Represents the nucleotide sequence of cDNA coding for isoform a of human ACS6.
[SEQ ID NO:15]
   Represents the amino acid sequence of isoform b of human ACS6.
[SEQ ID NO:16]
   Represents the nucleotide sequence of cDNA coding for isoform b of human ACS6.
[SEQ ID NO:17]
   Represents the amino acid sequence of human FAS (GeneBank NP_004095).
[SEQ ID NO:18]
   Represents the nucleotide sequence of cDNA coding for human FAS (GeneBank NM_004104).
[SEQ ID NO:19]
   Represents the nucleotide sequence of a primer used in Example 1.
[SEQ ID NO:20]
   Represents the nucleotide sequence of a primer used in Example 1.

### Examples

The present invention is explained in more detail below using examplies, but the present invention is not limited thereby.

### Example 1 Acquisition of FAS inhibitor resistance by ACS expression

### (1) Cloning of human ACS5 and construction of the expression vector

The human ACS5 gene was cloned by PCR from human large intestinal cancer HCT-15 cell cDNA. The following sequences were used as primers for cloning human ACS5.
5'-AAAGAATTCTATGCTTTTTATCTTTAACTTTTTGTTTTCCC-3' (SEQ ID NO:19)
5'-AAAGGATCCATAATCCTGGATGTGCTCATACAGGC-3' (SEQ ID NO:20)

(The primers were designed with TAT substituted for the stop codon TAG so that a flag tag could be attached to the 3'-terminal.) The reaction was performed in 35 cycles of 30 second at 94°C, 30 seconds at 65°C and 4 minutes at 72°C, using AmpliTaq DNA polymerase (Applied Biosystems). Both ends of the resulting cDNA fragment were cleaved with EcoRI and BamHI (Takara Bio) and cloned to pFLAG-CMV5 to obtain pFLAG-CMV-ACS5, and the DNA sequence was confirmed. The resulting cDNA sequence was identical with that of known human ACS5 (AB033899). The resulting ACS5 cDNA was excised from pFLAG-CMV-ACS5 with the FLAG tag attached to the 3' end, and incorporated into the retrovirus vector pHa-IRES-DHFR (Kage, K. et al., Int. J. Cancer, Vol. 97, p. 626 to 630, 2002) to construct pHa-ACS5-FLAG-IRES-DHFR.

### (2) Establishment of cells stably expressing ACS5

Cells stably expressing ACS5 were established as follows. The retrovirus vector pHa-ACS5-FLAG-IRES-DHFR constructed above and a blank pHa-IRES-DHFR vector were introduced into mouse PA317 fibroblast cells using a mammalian transfection kit (Stratagene), and virus liquid was obtained. Human glioma SF268 cells (ATCC) were then infected with the resulting virus suspension, and selected with 100 ng/ml of methotrexate as a selection agent to obtain the stable expression line SF268/ACS5 and SF268/mock as a control. The cell extracts of each cell line were analyzed by Western blotting using anti-FLAG-M2 antibody (Sigma) and anti-tubulin antibody (Sigma) for the control, and stable expression of the ACS5 protein was confirmed in SF268/ACS5.

### (3) Investigation of changes in FAS inhibitor sensitivity in cancer cells due to ACS expression

This study was performed using the SF268/ACS5 cell with stable high expression of ACS5 established by the methods above, together with SF268/mock cells as control cells and RPMI1640-10% FBS (fetal bovine serum)-20 mM HEPES (pH 6.5) as the cell culture liquid. The SF268/mock cells and SF268/ACS5 cells were seeded 100,000 cells/well on 6-well plates, and cultured at 37°C for 7 days with RPMI1640-10% FBS-20 mM HEPES (pH 6.5). Then, these were either left untreated or treated for 24 hours by addition of either 15 µg/ml of Cerulenin ([2R,3S,E,E)-2,3-epoxy-4-oxo-7,10-dodecadienamide] (Sigma)) or 15 µm/ml of C75 ([4-methylene-2-octyl-5-oxotetrahydrofuran-3-carboxylic acid] (Alexis Biochemicals)) as a FAS inhibitor to the medium, after which the number of surviving cells was measured by trypan blue exclusion. The number of surviving cells is shown as a percentage given 100% as the number of untreated SF268/mock cells.

The results are shown in Fig. 1.

Without treatment, the SF268/ACS5 cells with stable high ACS5 expression showed significantly higher cell survival rate than the control SF268/mock cells. Comparing sensitivity to FAS inhibitors, there was a clear growth suppression effect from Cerulenin and C75 in the control SF268/mock cells, but no growth suppression effect from Cerulenin or C75 in the SF268/ACS5 cells with stable high ACS5 expression. This shows that cancer cells overexpressing ACS acquire resistance to FAS inhibitors.

### Example 2 Investigation of combined effect of ACS inhibitor and FAS inhibitors

The anti-tumor effects of an ACS inhibitor (Triacsin C [2,4,7-undecatrienal nitrosohydrazone]) and FAS inhibitor (Cerulenin or C75) used in combination was investigated using the lung cancer cell strain NCI-H23 (ATCC). NCI-H23 cells were cultured in RPMI1640-10%FBS. The added drugs Triacsin C, Cerulenin and C75 were all purchased from Sigma. The NCI-H23 cells were seeded on 96-well plates at a concentration of 2000 cells/well, 1 day later (A) Triacsin C, (B) Cerulenin, (C) C75, (D) Triacsin C and Cerulenin and (E) Triacsin C and C75 were added and the cells were treated for 3 days, after which the cell survival rate was measured by the Sulforhodamine B method (Skehan, P. et al., J. Natl. Cancer Inst., Vol. 82, p. 1107 to 1112, 1990, and the results were compared to the results without treatment. The drugs were added at a concentration of 0.3 µM for Triacsin C, 2 µM for Cerulenin and 2 µM for C75. Each test was performed with n = 3. As a result, the cell survival rates under drug treatment conditions (A) through (E) above were (A) 56.3 ± 0.5%, (B) 70.2 ± 7.9%, (C) 61.2 ± 3.6%, (D) 13.4 ± 1.4% and (E) 15.3 ± 1.0% given 100% as the cell survival rate without drug treatment. These results show that anti-tumor effects are enhanced by combined use of an ACS inhibitor (Triacsin C) together with a FAS inhibitor (Cerulenin or C75).

### INDUSTRIAL APPLICABILITY

By simultaneously inhibiting the activity or expression of an enzyme belonging to the acyl-CoA synthase (ACS) family and the activity or expression of fatty acid synthase (FAS), it is possible to selectively and strongly induce apoptosis of cancer cells and selectively and effectively kill cancer cells. Thus, 1) a medicament prepared by combining (i) at least one selected from (a) a compound or salt thereof that inhibits the activity of an enzyme belonging to the acyl-CoA synthase family, (b) a compound or salt thereof that inhibits expression of a gene for an enzyme belonging to the acyl-CoA synthase family, (c) an antibody to an enzyme belonging to the acyl-CoA synthase family, (d) an antisense polynucleotide comprising a nucleotide sequence or part of a nucleotide sequence complementary or substantially complementary to the nucleotide sequence of a polynucleotide coding for an enzyme belonging to the acyl-CoA synthase family and (e) siRNA or shRNA for a polynucleotide coding for an enzyme belonging to the acyl-CoA synthase family, with (ii) at least one selected from (a) a compound or salt thereof that inhibits the activity of fatty acid synthase, (b) a compound or salt thereof that inhibit expression of a fatty acid synthase gene, (c) an antibody to fatty acid synthase, (d) an antisense polynucleotide comprising a nucleotide sequence or part of a nucleotide sequence complementary or substantially complementary to the nucleotide sequence of a polynucleotide coding for fatty acid synthase and (e) siRNA or shRNA for a polynucleotide coding for fatty acid synthase, and 2) a medicament containing (i) a substance that inhibits the activities of an enzyme in the acyl-CoA synthase family and of fatty acid synthase and/or (ii) a substance that inhibits expression of a gene belonging to the acyl-CoA synthase family and fatty acid synthase can be used as a safe drug such as for example as a preventative/therapeutic agent for cancer (such as brain tumor, hypophyseal adenoma, glioma, acoustic neurilemmoma, retinal sarcoma, thyroid cancer, throat cancer, cancer of the larynx, tongue cancer, thymic cancer, mesothelial cancer, breast cancer, lung cancer, non-small-cell lung cancer, small-cell lung cancer, stomach cancer, esophageal cancer, duodenal cancer, large intestinal cancer, colon cancer, rectal cancer, liver cancer, hepatocarcinoma, pancreatic cancer, pancreatic endocrine tumor, bile duct cancer, gallbladder cancer, penis cancer, kidney cancer, renal pelvic cancer, ureter cancer, renal cell cancer, testicular cancer, prostate cancer, bladder cancer, vulvar cancer, uterine cancer, cervical cancer, cancer of the uterine body, uterine sarcoma, trophoblastic disease, vaginal cancer, ovarian cancer, ovarian germ cell tumor, skin cancer, malignant myeloma, mycosis fungoides, basal cell cancer, soft tissue sarcoma, malignant lymphoma, Hodgkin's disease, myelodysplastic syndrome, multiple myeloma, leukemia, acute myelocytic leukemia, chronic myelocytic leukemia, acute lymphocytic leukemia, chronic lymphocytic leukemia, adult T-cell leukemia, chronic myeloproliferative disease, pancreatic endocrine tumors, fibrous histiocytoma, leiomyosarcoma, rhabdomyosarcoma, cancers of unknown primary origin and the like), as a cancer cell apoptosis promoting agent, or as a cancer cell proliferation inhibiting agent or the like.

The (i) at least one selected from (a) a compound or salt thereof that inhibits the activity of an enzyme belonging to the acyl-CoA synthase family, (b) a compound or salt thereof that inhibits expression of a gene for an enzyme belonging to the acyl-CoA synthase family, (c) an antibody to an enzyme belonging to the acyl-CoA synthase family, (d) an antisense polynucleotide comprising a nucleotide sequence or part of a nucleotide sequence complementary or substantially complementary to the nucleotide sequence of a polynucleotide coding for an enzyme belonging to the acyl-CoA synthase family and (e) siRNA or shRNA for a polynucleotide coding for an enzyme belonging to the acyl-CoA synthase family and (ii) at least one selected from (a) a compound or salt thereof that inhibits the activity of fatty acid synthase, (b) a compound or salt thereof that inhibit expression of a fatty acid synthase gene, (c) an antibody to fatty acid synthase, (d) an antisense polynucleotide comprising a nucleotide sequence or part of a nucleotide sequence complementary or substantially complementary to the nucleotide sequence of a polynucleotide coding for fatty acid synthase and (e) siRNA or shRNA for a polynucleotide coding for fatty acid synthase may also be the same substance.

The (i) substance that inhibits the activities of an enzyme in the acyl-CoA synthase family and fatty acid synthase and the (ii) substance that inhibits expression of a gene belonging to the acyl-CoA synthase family and fatty acid synthase may also be the same substance.

## Claims

1. A preventive/therapeutic agent for cancer prepared by combining (i) at least one selected from a substance that inhibits the activity of an enzyme belonging to the acyl-CoA synthase family and a substance that inhibits expression of a gene for an enzyme belonging to the acyl-CoA synthase family, with (ii) at least one selected from a substance that inhibits the activity of fatty acid synthase and a substance that inhibits expression of a fatty acid synthase gene.

2. The preventive/therapeutic agent according to Claim 1, wherein the enzyme belonging to the acyl-CoA synthase family is at least one selected from the proteins comprising the same or substantially the same amino acid sequences represented by SEQ ID NO:1, SEQ ID NO:3, SEQ ID NO:5, SEQ ID NO:7, SEQ ID NO:9, SEQ ID NO:11, SEQ ID NO:13 AND SEQ ID NO:15, or partial peptides or salts thereof.

3. The preventive/therapeutic agent according to Claim 1, wherein the enzyme belonging to the acyl-CoA synthase family is at least one selected from the proteins comprising the same or substantially the same amino acid sequences represented by SEQ ID NO:1, SEQ ID NO:3, SEQ ID NO:5, SEQ ID NO:7, SEQ ID NO:9, SEQ ID NO:11, or partial peptides or salts thereof.

4. The preventive/therapeutic agent according to Claim 1, wherein the fatty acid synthase is a protein comprising the same or substantially the same amino acid sequence represented by SEQ ID NO:17, or a partial peptide or salt thereof

5. The preventive/therapeutic agent according to Claim 1, prepared by combining (i) at least one selected from (a) a compound or salt thereof that inhibits the activity of an enzyme belonging to the acyl-CoA synthase family, (b) a compound or salt thereof that inhibits expression of a gene for an enzyme belonging to the acyl-CoA synthase family, (c) an antibody to an enzyme belonging to the acyl-CoA synthase family, (d) an antisense polynucleotide comprising a nucleotide sequence or part of a nucleotide sequence complementary or substantially complementary to the nucleotide sequence of a polynucleotide coding for an enzyme belonging to the acyl-CoA synthase family and (e) siRNA or shRNA for a polynucleotide coding for an enzyme belonging to the acyl-CoA synthase family, with (ii) at least one selected from (a) a compound or salt thereof that inhibits the activity of fatty acid synthase, (b) a compound or salt thereof that inhibits expression of a fatty acid synthase gene, (c) an antibody to fatty acid synthase, (d) an antisense polynucleotide comprising a nucleotide sequence or part of a nucleotide sequence complementary or substantially complementary to the nucleotide sequence of a polynucleotide coding for fatty acid synthase and (e) siRNA or shRNA for a polynucleotide coding for fatty acid synthase.

6. The preventive/therapeutic agent according to Claim 1, prepared by combining a compound or salt thereof that inhibits the activity of an enzyme belonging to the acyl-CoA synthase family with a compound or salt thereof that inhibits the activity of fatty acid synthase.

7. A preventive/therapeutic agent for cancer, comprising (i) a substance that inhibits the activities of an enzyme belonging to the acyl-CoA synthase family and fatty acid synthase, and/or (ii) a substance that inhibits expression of a gene for an enzyme belonging to the acyl-CoA synthase family and expression of a fatty acid synthase gene.

8. The preventive/therapeutic agent according to Claim 1, wherein the preventive/therapeutic agent for cancer is a combination preparation.

9. The preventive/therapeutic agent according to Claim 1, wherein the preventive/therapeutic agent is a kit comprising (i) a medicament comprising at least one selected from a substance that inhibits the activity of an enzyme belonging to the acyl-CoA synthase family and a substance that inhibits expression of a gene for an enzyme belonging to the acyl-CoA synthase family and (ii) a medicament comprising at least one selected from a substance that inhibits the activity of fatty acid synthase and a substance that inhibits expression of a fatty acid synthase gene.

10. A method for preventing/treating cancer, wherein (i) the activity of an enzyme belonging to the acyl-CoA synthase family and/or expression of a gene for the enzyme is inhibited and (ii) the activity of fatty acid synthase and/or expression of a gene for the enzyme is inhibited.

11. A method for preventing/treating cancer, wherein (i) an effective dose of at least one selected from a substance that inhibits the activity of an enzyme belonging to the acyl-CoA synthase family and a substance that inhibits expression of a gene for an enzyme belonging to the acyl-CoA synthase family, and (ii) an effective dose of at least one selected from a substance that inhibits the activity of fatty acid synthase and a substance that inhibits expression of a fatty acid synthase gene, are administered to a mammal.

12. A use of (i) at least one selected from a substance that inhibits the activity of an enzyme belonging to the acyl-CoA synthase family and a substance that inhibits expression of a gene for an enzyme belonging to the acyl-CoA synthase family, and (ii) at least one selected from a substance that inhibits the activity of fatty acid synthase and a substance that inhibits expression of a fatty acid synthase gene, to manufacture a preventive/therapeutic agent for cancer.

13. A cancer cell apoptosis promoting agent or cancer cell proliferation inhibiting agent prepared by combining (i) at least one selected from a substance that inhibits the activity of an enzyme belonging to the acyl-CoA synthase family and a substance that inhibits expression of a gene for an enzyme belonging to the acyl-CoA synthase family, with (ii) at least one selected from a substance that inhibits the activity of fatty acid synthase and a substance that inhibits expression of a fatty acid synthase gene.

14. The agent according to Claim 13, prepared by combining (i) at least one selected from (a) a compound or salt thereof that inhibits the activity of an enzyme belonging to the acyl-CoA synthase family, (b) a compound or salt thereof that inhibits expression of a gene for an enzyme belonging to the acyl-CoA synthase family, (c) an antibody to an enzyme belonging to the acyl-CoA synthase family, (d) an antisense polynucleotide comprising a nucleotide sequence or part of a nucleotide sequence complementary or substantially complementary to the nucleotide sequence of a polynucleotide coding for an enzyme belonging to the acyl-CoA synthase family and (e) siRNA or shRNA for a polynucleotide coding for an enzyme belonging to the acyl-CoA synthase family, with (ii) at least one selected from (a) a compound or salt thereof that inhibits the activity of fatty acid synthase, (b) a compound or salt thereof that inhibits expression of a fatty acid synthase gene, (c) an antibody to fatty acid synthase, (d) an antisense polynucleotide comprising a nucleotide sequence or part of a nucleotide sequence complementary or substantially complementary to the nucleotide sequence of a polynucleotide coding for fatty acid synthase and (e) siRNA or shRNA for a polynucleotide coding for fatty acid synthase.

15. The agent according to Claim 13, prepared by combining a compound or salt thereof that inhibits the activity of an enzyme belonging to the acyl-CoA synthase family with a compound or salt thereof that inhibits the activity of fatty acid synthase.

16. A cancer cell apoptosis promoting agent or cancer cell proliferation inhibiting agent, comprising (i) a substance that inhibits the activities of an enzyme belonging to the acyl-CoA synthase family and fatty acid synthase, and/or (ii) a substance that inhibits expression of a gene for an enzyme belonging to the acyl-CoA synthase family and expression of a fatty acid synthase gene.

17. A cancer cell apoptosis promotion method or cancer cell proliferation inhibition method, wherein (i) the activity of an enzyme belonging to the acyl-CoA synthase family and/or expression of a gene for the enzyme is inhibited and (ii) the activity of fatty acid synthase and/or expression of a gene for the enzyme is inhibited.
